# EUROPÄISCHE PATENTSCHRIFT

(11) **EP 2 477 588 B1**
(45) Veröffentlichungstag und Bekanntmachung des Hinweises auf die Patenterteilung: **27.11.2013**
(21) Anmeldenummer: 10752715.2
(22) Anmeldetag: 07.08.2010
(51) Int. Cl.: A61F 13/00, D04H 1/42, A61F 7/02, D04H 1/64, D04H 1/407, D04H 1/498

(54) **FLÄCHENGEBILDE**
TEXTILE FABRIC
STRUCTURE PLATE

(30) Priorität: 07.08.2009 DE 102009036588; 21.05.2010 DE 102010017065
(43) Veröffentlichungstag der Anmeldung: 25.07.2012
(73) Patentinhaber: pervormance international GmbH, 89075 Ulm (DE)
(72) Erfinder: RENNER, Gabriele, 89275 Elchingen (DE)
(86) Internationale Anmeldenummer: PCT/EP2010/004853
(87) Internationale Veröffentlichungsnummer: WO 2011/015377

(56) Entgegenhaltungen:
- EP-A2- 0 463 716
- EP-A2- 0 676 496
- EP-A2- 1 260 626
- WO-A1-01/47456
- WO-A1-03/012182
- WO-A2-2004/016425
- WO-A2-2004/105823
- DE-A1- 10 232 078
- DE-A1-102007 036 496
- US-A1- 2005 118 383

## Beschreibung

Die Erfindung betrifft ein Flächengebilde aus einem eine superabsorbierende Aktivierung aufweisenden Vlies, ein Verfahren zur Herstellung des Flächengebildes, eine Umhüllung, die das Flächengebilde umfasst, ein die Umhüllung verwendendes Kühlsystem sowie eine als Kühlartikel ausgebildete Umhüllung.

Flächengebilde, die eine absorbierende Aktivierung aufweisen, sind aus dem Hygienebereich bekannt. Hierbei ist jedoch vorgesehen, dass die Flächengebilde eine absorbierende Aktivierung in Form von Partikeln oder dergleichen aufweisen die dazu dienen, Flüssigkeiten aufzunehmen und dauerhaft zu binden. Die Eigenschaften der in den bekannten Flächengebilden eingesetzten sogenannten Superabsorbern dienen hierbei dazu, beispielsweise die Flüssigkeitsaufnahmekapazität von Windeln, Inkontinenzeinlagen und sonstigen saugfähigen Hygieneartikeln wesentlich zu erhöhen und die aufgenommene Flüssigkeit dauerhaft zu binden.

Im Stand der Technik sind hierzu Flächengebilde für den einmaligen Gebrauch bekannt, die eine Tragschicht, beispielsweise aus Zellulosefasern oder sonstigen vliesartigen Materialien aufweisen. Auf oder in diese Tragschichten sind die sogenannten Superabsorber in Form von Partikeln eingefügt. Aufgrund der nur mäßigen Beanspruchung und des Charakters als Wegwerfartikel findet keine aufwendige Verbindung zwischen den Superabsorberpartikeln und dem Material der Trägerschicht statt. Eine Fixierung der Superabsorberpartikeln erfolgt beispielsweise durch loses Einstreuen zwischen dem Fasermaterial oder den Schichten der entsprechenden Artikel. Auch sind die oben genannten Einmalartikel darauf ausgelegt, die anfallende Flüssigkeit aufzunehmen und zu binden. Eine Abgabe der aufgenommenen Flüssigkeit ist nicht vorgesehen beziehungsweise keinesfalls erwünscht. Sinn und Zweck der superabsorbierend aktivierten Artikel ist es vielmehr, eine stets trockene Oberfläche zur Verfügung zu stellen, und den Austritt von Flüssigkeit aus dem Artikel dauerhaft zu verhindern.

Da es sich bei den bekannten Artikeln um Wegwerfgegenstände handelt, die nicht zum mehrmaligen Gebrauch bestimmt sind, wird hier keinerlei Anstrengung darauf verwendet, die in den Gebilden vorgesehenen Superabsorber dauerhaft mit den Trägermaterialien zu verbinden. Bei entsprechender mechanischer Beanspruchung lösen sich die Partikel daher leicht aus dem Faserverbund und sammeln sich vorzugsweise in einem inneren, tiefliegenden Bereich des Artikels. Die absorbierenden beziehungsweise desorbierenden Eigenschaften werden dadurch signifikant verschlechtert.

Ein weiteres Einsatzgebiet von Partikeln die mit Superabsorbern ausgestattet sind, liegt in der Verwendung zur Kühlung. Dies macht sich den Umstand zu Nutze, dass bei der Abgabe von in den Superabsorbern aufgenommener Flüssigkeit beziehungsweise deren Verdunstung eine Kühlung aufgrund der benötigten Verdunstungswärme stattfindet. Die bekannten Produkte, die Superabsorber zur Kühlung einsetzen, weisen jedoch das Problem auf, dass diese entweder als Gelakkus, mit Gel gefüllte Kammern oder als Gel ausgebildet sind, das dadurch entsteht, dass Wasser oder eine sonstige Flüssigkeit in Taschen eingebracht wird, in denen der Superabsorber in Partikel-oder Kristallform vorgehalten ist. Die meisten dieser gelbasierten Produkte müssen jedoch bevor sie angewendet werden können, im Kühlschrank oder in einem Tiefkühlfach aufbewahrt werden, um richtig wirken zu können. Die Kühlwirkung wird dadurch erreicht, dass die Geltaschen beziehungsweise Gelakkus die Temperatur des Kühlschranks oder des Tiefkühlfaches aufnehmen und diese Temperatur teilweise länger halten sollen als heißes oder kaltes Wasser alleine. Der Nachteil dieser Verwendung des Superabsorbers ist das oftmals recht hohe Gewicht. Die für die Vorkühlung des Akkus beziehungsweise der Geltaschen oder -kammern notwendige Zeit und der meist langwierige und fehlerbehaftete Prozess, der dadurch bedingt ist, dass die Superabsorber, sofern diese in Textilien angeordnet sind, erst durch Wasserbehandlung zum Gel werden. Darüber hinaus weisen die bekannten Produkte eine nur geringe Kühlwirkungszeit auf.

Wird das superabsorbierende Material in Textilien angeordnet beziehungsweise in diese integriert, zeigt sich hier der Nachteil, dass die Textilien längere Zeit benötigen bis sie das Wasser vollständig aufnehmen, lange nass sind und tropfen. Teilweise durchdringt das in den Textilien gebildete Gel oder Wasser auch den Stoff und führt zu einem nassen oder schmierigen Effekt auf der Außenseite der Textilien, was die dauerhafte Verwendung der Textilien limitiert. Zudem dauert es einige Zeit, bis das Textilprodukt soweit mit Wasser beladen und getrocknet ist, dass es verwendet werden kann, ohne die gekühlten Gegenstände beziehungsweise Oberflächen oder Körperteile zu befeuchten.

Viele dieser Produkte sind nach einer oder wenigen Anwendungen nicht mehr gebrauchsfähig, verändern ihre Optik oder das Innere löst sich langsam auf. Vor allem sind die Produkte nicht waschbar oder oft nicht reinigbar, was die Anwendung auch begrenzt. Einige Produkte versuchen dies durch Textileigenschaften zu lösen, was allerdings in einem verschlechterten Verdunstungsprozess beziehungsweise schlechteren Kühleffekt resultiert.

Ausgehend von dem bekannten Stand der Technik ist es die Aufgabe der vorliegenden Erfindung, ein zur Kühlung geeignetes, superabsorbierend aktiviertes Flächengebilde und daraus hergestellte Gegenstände zur Verfügung zu stellen, die schnell, einfach, dauerhaft und mehrfach sowie über einen längeren Zeitraum einsetzbar sind.

Zur Lösung dieser Aufgabe schlägt die Erfindung mehrere Gegenstände vor.

Zunächst wird ein mehrfach verwendbares, mit Flüssigkeit beladbares und dadurch aktivierbares sowie bei Entladung, das heißt beispielsweise Verdunsten oder Verdampfen der geladenen Flüssigkeit beziehungsweise von Flüssigkeitskomponenten kühlendes Flächengebilde aus einem eine superabsorbierende Aktivierung aufweisenden Vlies vorgeschlagen, das sich für eine wiederholte Be- und Entladung mit einer Flüssigkeit eignet und wobei insbesondere der Entladevorgang zur Kühlung dient und wobei das Flächengebilde im trockenen, nicht mit Flüssigkeit oder Flüssigkeitskomponenten beladenen Zustand eine flächenbezogene Masse zwischen 50 und 1500 g/m², bevorzugt von 50 bis 700 g/m², aufweist. Unter einer superabsorbierenden Aktivierung ist hierbei zu verstehen, dass ein die superabsorbierende Aktivierung bildendes Material, in fester, gelöster, gelartiger oder flüssiger Form, das im, am oder auf dem Flächengebilde festgelegt ist, in der Lage ist ein Vielfaches seines Eigengewichtes an Flüssigkeiten aufzunehmen. Als Flüssigkeit in Frage kommen hierbei beispielsweise Wasser oder salzhaltige Lösungen sowie Gemische aus Wasser, Alkohol oder sonstigen Zuschlags-oder Zusatzstoffen. Die superabsorbierende Aktivierung des erfindungsgemäßen Flächengebildes kann dabei über die Einbringung sogenannter Superabsorberpolymere (SAP) durchgeführt werden, die entweder die Oberflächen des Flächengebildes beziehungsweise der Fasern oder Filamente beschichten oder in das Flächengebilde eingearbeitet, beispielsweise eingeklebt, eingewebt, eingesponnen oder in sonstiger geeigneter Art und Weise mit dem Flächengebilde verbunden sind. Die superabsorbierende Aktivierung weist dabei einen stabilen, dauerhaften Verbund mit dem Vlies beziehungsweise den das Vlies bildenden Fasern oder Filamenten auf, oder ist integraler Bestandteil des Vlieses, beziehungsweise der, das Vlies bildenden Fasern beziehungsweise Filamenten.

Die SAP-Polymere dienen dabei nicht allein zum mehrmaligen Aufnehmen und Abgeben von Flüssigkeit. Über die Art der Einbringung sowie die Form, Größe, Struktur und Zusammensetzung der Polymere beziehungsweise anderer Polymerkomponenten kann auch die Geschwindigkeit und gleichmäßige Verteilung der Flüssigkeitsaufnahme und Freisetzung sowie die Menge an aufgenommener Flüssigkeit gesteuert werden. Hierdurch können dann für den jeweiligen Verwendungszweck geeignete Kühlparameter eingestellt beziehungsweise definiert und die Kühlleistung sowie der Kühleffekt, der mit dem erfindungsgemäßen Flächengebilde erzielt werden kann, gesteuert werden.

Somit unterscheiden sich Zielsetzungs- und Anwendungsgebiet des erfindungsgemäßen Flächengebildes klar von den aus dem Stand der Technik bekannten Einwegartikeln, da letztere nicht für die mehrfache Verwendung sowie den Gebrauch zu Kühlzwecken geeignet und vorgesehen sind. Auch der zur Kühlung bisher verwendete Stand der Technik vereint nicht die angesprochenen Eigenschaften der vorliegenden Erfindung.

Die Erfindung erreicht einen zuverlässigen, dauerhaften Verbund der bevorzugt als Superabsorberpolymere (SAP) ausgeführten superabsorbierenden Aktivierung mit dem Vlies, wodurch unter anderem die Ablösung oder Ansammlung der Superabsorberpolymere an dem das Vlies begrenzenden Stoff- beziehungsweise Materialflächen vermieden wird, sodass ein entsprechend glitschiger oder schmieriger Effekt, der durch mangelhafte Polymerisierung der Superabsorberpolymere und dadurch vorhandene Monomere erzeugt wird, auf der Außenseite des BegrenzungsStoffs- beziehungsweise -materials dauerhaft vermieden wird. Dies wird im erfindungsgemäßen Flächengebilde dadurch unterbunden, dass eine nahezu vollständige Polymerisierung der Superabsorberpolymere angestrebt beziehungsweise realisiert wird. Noch vorhandene Monomere werden in einem nachgelagerten Separationsschritt aus dem Flächengebilde entfernt. Die Aktivierung ist dabei so stabil beschaffen, dass diese auch eine lange Einsatzdauer des Flächengebildes beziehungsweise des das Flächengebilde tragenden beziehungsweise umfassenden Gegenstandes (wie zum Beispiel eine Umhüllung) ermöglicht. Die Verbindung von Superabsorberpolymeren und Vlies- beziehungsweise Flächengebilde erfolgt hier in einem chemischen, mechanischen oder thermischen Prozess.

Es ergeben sich zusätzliche Anwendungsmöglichkeiten für das erfindungsgemäße Flächengebilde. Der Einsatz des erfindungsgemäßen Flächengebildes, z. B. in Kleidungsstücken ist damit problemlos möglich, da die Dauerhaftigkeit der superabsorbierenden Aktivierung im erfindungsgemäßen Flächengebilde beziehungsweise dem dieses bildenden oder in diesem vorgesehenen Vlies derart verlängert wird, dass eine verhältnismäßig lang-währende Benutzung des Kleidungsstückes mit (fast) gleichbleibender Kühlwirkung und -leistung möglich ist, ohne dass die Handhabbarkeit beeinträchtigende Effekte auftreten.

Aber auch andere Anwendungsbereiche, bei welchen insbesondere ein möglichst hygienischer Artikel günstig ist, werden durch den erfindungsgemäßen Vorschlag ermöglicht. Da auch im langwährenden Einsatz die superabsorbierende Aktivierung beziehungsweise das die superabsorbierende Aktivierung bildende Superabsorberpolymer in geeigneter Weise (wie beschrieben) in dem Vlies verbleibt, verkleben die sich bei herkömmlichen Produkten aus dem Vlies lösenden Polymerpartikel beziehungsweise noch vorhandene Monomere die Poren des das Vlies beziehungsweise das Flächengebildes umschließenden Textils nicht. Dies hat den Vorteil, dass, aufgrund der Tatsache, dass durch diese Grenzflächen gegebenenfalls Wasserdampf oder Wasser hindurchzutreten hat, um den erfindungsgemäßen Effekt zu ermöglichen, hier keine Sperrwirkung und damit Wirkbeeinträchtigung stattfindet. Zudem bleibt die Optik des Textils dauerhaft unverändert erhalten.

Eine superabsorbierende Aktivierung stellt beispielsweise auch die Einbringung von aus einem Superabsorber bestehenden Pad oder die Durchdringung des Flächengebildes mit strangartig ausgebildeten Superabsorbergebilden dar. Die Beladung des Flächengebildes mit einer Flüssigkeit erfolgt dabei beispielsweise durch das Eintauchen des Flächengebildes beziehungsweise eines das Flächengebilde umfassenden Gegenstandes in die Flüssigkeit, und hierbei insbesondere in Wasser, salzhaltige Lösungen und/oder Gemische aus Wasser, Alkohol und/oder Zuschlags- oder Zusatzstoffen. Neben dem Eintauchen in die ent-spechende Flüssigkeit kann erstmalig auch ein oberflächliches Benetzen, beispielsweise durch Besprühen oder Begießen mit der entsprechenden Flüssigkeit erfolgen, um das Flächengebilde zu beladen. Im Zuge der Benetzung beziehungsweise Durchdringung mit Flüssigkeit wird diese von dem Superabsorberpolymer beziehungsweise durch die superabsorbierende Aktivierung schnell, das heißt innerhalb weniger Sekunden, aufgesaugt und reversibel gespeichert.

Das erfindungsgemäße Flächengebilde ist dabei so ausgebildet, dass die superabsorbierende Aktivierung in der Lage ist, eine langandauernde Abgabe der Flüssigkeit durchzuführen. Im Verlauf dieser Abgabe von Flüssigkeit, kommt es zu deren Verdunstung und es tritt in der Folge ein Kühleffekt ein. Dieser liegt darin begründet, dass für die Verdunstung des Wassers beziehungsweise der sonstigen Flüssigkeit dem zu kühlenden Körper oder Gegenstand Wärme beziehungsweise thermische Energie entzogen werden muss und dieser sich dadurch abkühlt. Das erfindungsgemäße Flächengebilde ist dabei so ausgebildet, dass eine wiederholte Be- und Entladung mit einer Flüssigkeit stattfinden kann, ohne dass die superabsorbierende Aktivierung einen deutlichen Funktionsverlust zeigt. Dies wird durch eine dauerhafte Verbindung vom Flächengebilde und Superabsorber in einem chemischen, mechanischen oder thermischen Prozess erreicht.

Das erfindungsgemäße Flächengebilde, das besonders dauerhaft und stabil ausgeführt ist, weist, um für ein möglichst breites Einsatzgebiet zur Verfügung zu stehen, eine flächenbezogene Masse zwischen 50 g/m² und 1500 g/m² auf. Die angegebenen Werte beziehen sich dabei nur auf die Masse des unbehandelten, bevorzugt trockenen Flächengebildes, also ohne beladene Flüssigkeit und ohne die Masse eines das Flächengebilde möglicherweise umschließenden, einfassenden und/oder umhüllenden Materials einzuschließen. Während sich Flächengebilde mit einer ver-gleichweise geringen flächenbezogenen Masse besonders zur Ausrüstung von am Körper getragenen Gegenständen oder Kleidungsstücken, die den Träger nicht behindern sollen, eignen, ist ein Flächengebilde mit einer flächenbezogenen Masse von bis zu 700 g/m² und mehr besonders dazu geeignet eine hohe Menge an Flüssigkeit aufzunehmen und diese hohe aufgenommene Menge im Zuge der vorgesehenen Abgabe der Flüssigkeit auch wieder abzugeben. Die Flächengebilde mit hoher flächenbezogener Masse bilden beispielsweise Abdeckmaterialien, die zur Kühlung von größeren Gegenständen, beispielsweise bei Bränden, sehr hohen Temperaturen oder dergleichen eingesetzt werden. Die hohe flächenbezogene Masse geht einher mit einer besonders dauerhaften Ausbildung des Flächengebildes, sodass dies eine wesentlich verbesserte mechanische Stabilität aufweist und sich damit auch für stärker beanspruchende Einsatzgebiete wie beispielsweise im Sanitäts- oder Medizinbereich sowie für mobile Anwendungen, beispielsweise für die Abdeckung von Fahrzeugen oder Geräten wie auch von großen Maschinen eignet. Generell besteht jedoch die Möglichkeit für alle vorgesehenen Anwendungen des erfindungsgemäßen Flächengebildes mit im genannten Wertebereich liegenden flächenbezogenen Massen zu verwenden.

Neben den beiden großen Anwendungsbereichen, die sich insbesondere durch die flächenbezogene Masse einerseits zwischen 50 g/m² und 700 g/m² und andererseits eine flächenbezogene Masse von 500 g/m² bis 1500 g/m² auszeichnen, umfasst die Erfindung natürlich auch Vorschläge, die zwischen 50 g/m² und 100 g/m², 100 g/m² und 200 g/m², 200 g/m² und 300 g/m², 300 g/m² und 400 g/m², 400 g/m² und 500 g/m² sowie zwischen 500 g/m² und 600 g/m², 600 g/m² und 700 g/m², 700 g/m² und 800 g/m², 800 g/m² und 900 g/m² sowie von 900 g/m² bis 1500 g/m² oder mehr reichen. Die flächenbezogene Masse des Flächengebildes kann vom Verwender in Abhängigkeit vom Einsatzgebiet des Flächengebildes zudem frei aus den vorgenannten Wertebereiche ausgewählt beziehungsweise innerhalb oder außerhalb dieser Wertebereiche eingestellt werden.

Die oben genannte flächenbezogene Masse, die im erfindungsgemäßen Flächengebilde zwischen 50 g/m² und 1500 g/m² beträgt, bezieht sich auf das gesamte Flächengebilde, jedoch ohne umgebende, umhüllende oder umschließende Materialien.

Als vorteilhaft wird es in diesem Zusammenhang angesehen, wenn das Ausgangsbeziehungsweise Rohvlies eine flächenbezogene Masse zwischen 50 g/m² und 120 g/m² aufweist. Der Vliesanteil am Flächengebilde liegt damit beispielsweise zwischen 10% und etwa 25 %, je nachdem wie das letztendliche Flächengebilde massemäßig ausgestattet ist.

In diesem Zusammenhang ist zu bemerken, dass vorstehend und nachfolgend relative Angaben sich nicht auf das jeweils angegebene Beispiel begrenzt auslegen lassen, sondern natürlich die Erfindung auch in einer erfindungsgemäßen Variante einen Vliesanteil am Flächengebilde bei einer gesamten flächenbezogenen Masse von bis zu 1500 g/m² aufweisen kann.

Selbstverständlich besteht nach der Erfindung auch die Möglichkeit, dass das Flächengebilde mehrere Vliesschichten umfasst. Der Vliesanteil am Flächengebilde erhöht sich dann, beispielsweise verdoppelt oder verdreifacht er sich, je nachdem, wie viele Vlieslagen im Flächengebilde vorgesehen sind. Hierbei besteht dann auch die Möglichkeit die Vliesschichten durch zwischen- oder aufgelagerte Lagen aus einem nicht oder weniger absorbierenden Material oder dergleichen getrennt vorliegen.

Über die flächenbezogene Masse des Vlieses wird letztendlich auch die Eignung des Flächengebildes für die jeweiligen Einsatzgebiete festgelegt. So wird sich eine erhöhte flächenbezogene Masse auch auf die letztendliche Dichte des Vlieses beziehungsweise die Dicke des Vliesmaterials auswirken. Bei dem angegebenen Wertebereich handelt es sich um die flächenbezogene Masse des Vlieses, die sonstigen Bestandteile des erfindungsgemäßen Flächengebildes, wie beispielsweise die die superabsorbierende Aktivierung bildenen Materialien, eventuelle Decklagen oder Trägerlagen des Flächengebildes sowie zusätzliche Additive, wie beispielsweise Kleber oder sonstige funktionalisierende Aktivierungen oder Substanzen bleiben hierbei zunächst unberücksichtigt. Diese bilden in Zusammenwirken mit dem Vlies das Flächengebilde und definieren dessen flächenbezogene Masse. Die flächenbezogene Masse des Vlieses liegt, insbesondere bei einer Verwendung für Textilmaterialien, Medizinprodukte oder als Produkte für die Kühlung von Körpern oder Körperteilen, bevorzugt zwischen und 60 g/m² und 100 g/m², wobei diese Werte auch überbeziehungsweise unterschritten werden können. Als besonders bevorzugt erweist sich eine flächenbezogene Masse von 80 g/m².

Unabhängig von dem letztendlichen Flächengewicht beziehungsweise der flächenbezogenen Masse, ist die Dicke des in dem Flächengebilde verwendeten Vlieses, wobei der Begriff Dicke vorliegend gleichbedeutend mit der von der im Textilbereich verwendeten Bezeichnung Stärke zu sehen ist und die Höhe beziehungsweise eine vertikale Ausdehnung der Stoff- beziehungsweise Vliesbahn angibt. Diese liegt günstigerweise zwischen 0,1 mm und 30 mm, wobei eine Dicke/Stärke von 1 mm bis 10 mm bevorzugt ist und sich insbesondere eine Dicke/Stärke von 6 mm bis 9 mm, bevorzugt von 4 mm bis 7 mm als günstig erweist. Die genannten Dicken-/Stärkenbereiche des Vlieses in dem Flächengebilde werden vor allem durch die letztliche Verwendung des fertigen, das Vlies umfassenden Flächengebildes bestimmt. So werden beispielsweise Vliesstärken zwischen 0,1 mm und 10 mm in geeigneter Weise in Textilien oder in den vorher bereits genannten (medizinischen) Kühlprodukten eingesetzt, während mechanisch hochbelastete und unter Umständen reißgefährdete Textilien, wie Abdeckplanen oder dergleichen, eine höhere Dicke aufweisen, um hier die entsprechende Dauerhaftigkeit des Flächengebildes sicherzustellen. Ebenfalls über die Dicke/Stärke wird, im Zusammenspiel mit dem jeweils gewählten Superabsorber und dessen eingesetzter Menge beziehungsweise der entsprechenden Volumenanteile, die Aufnahmefähigkeit für die Flüssigkeit beziehungsweise die Menge an aufgenommener Flüssigkeit bestimmt. So besteht selbstverständlich in einem dickeren/stärkeren Vlies die Möglichkeit mehr Flüssigkeit zu speichern als in einem nur wenige Millimeter starken Vlies. Ebenfalls entscheidend ist die Dicke/Stärke des Vlieses für dessen Verwendung. Auch wird die Dicke/Stärke des Vlieses durch die Einbausituation in einer Umhüllung, beispielsweise in einem Kleidungsstück, einem Medizinartikel oder einer Abdeckplane bestimmt. Hierbei kann durch die Anordnung einer Umhüllung, die das Flächengebilde umgibt und begrenzt, die Dicke/Stärke des Vlieses weiter reduziert werden. So kann die Dicke/Stärke eines Ausgangsvlieses dadurch reduziert werden, dass das letztendliche, das Vlies aufweisende Flächengebilde eine oder auch mehrere Grenzlage/n oder ähnliches aufweist, die die Vlieseinlage komprimiert/komprimieren und gegebenenfalls partioniert/partionieren, um so die Dicke/Stärke zu reduzieren.

Als vorteilhaft wird angesehen, wenn das Vlies Verfestigungsfasern aufweist, die die Vliesstruktur insgesamt stabilisieren und verbessern. Die Verfestigungsfasern bestehen aus dem gleichen oder ähnlichem Material wie das übrige Vlies, sind jedoch aufgrund einer mechanischen Nachbearbeitung des Vlieses und hierbei insbesondere im Zuge einer beispielsweise mechanischen Vernadelung, im Vlies ausgerichtet. Es ist hierbei vorgesehen, dass Verfestigungsfasern die Dicke/Stärke des Vlieses im Wesentlichen durchdringen. Hierbei ist jedoch vorgesehen, dass die jeweiligen Verfestigungsfasern das Vlies nicht vollständig durchdringen, also eine Verbindung zwischen den äußeren Oberflächen des Vlieses bilden, sondern jeweils nur die Hälfte des Vlieses oder aber 2/3 oder 3/4 des Vlieses durchdringen. Selbstverständlich besteht auch die Möglichkeit einer Durchdringung des Vlieses durch die Verfestigungsfasern bis zur nahezu vollständigen Dicke/Stärke des Vlieses anzustreben. Hierbei werden dann Werte für die Durchdringung des Vlieses von bis zu 100 % erreicht. Eine sehr starke Vernadelung wird jedoch nicht beziehungsweise noch nicht angestrebt, um genügend Poren und Öffnungen im Vlies für Beschichtung und Ausrüstung zu belassen. Durch die Vernadelung reduziert sich gegebenenfalls auch die Dicke/Stärke des Vlieses. Bei der Vernadelung wird periodisch eine Struktur, beispielsweise ein Balken oder eine Schiene, die Nadeln, Haken oder Düsen aufweist, in das Vlies eingedrückt beziehungsweise auf dieses aufgedrückt, die die Fasern dadurch weiter befestigen. Im Verlauf des Aufdrückens dringen die Nadeln/Düsen teilweise, jedoch nur bis zu einer vorher definierten Tiefe in das Vlies ein und nehmen dabei einen Anteil der Vliesfasern mit und richten sie aus. Diese Fasern bilden anschließend die Verfestigungsfasern, die das Vlies von der Seite her, auf der die Nadeln/Düsen in das Vlies eingedrückt beziehungsweise auf dieses aufgedrückt werden, durchlaufen. Die Nadeln/Düsen können dabei gegebenenfalls eine Strukturierung in Form von Widerhaken oder dergleichen aufweisen, um hier den Vernadelungsprozess weiter zu verbessern. Der Anteil an Verfestigungsfasern, die im Vlies vorgesehen sind, ist abhängig von der Anzahl der eingesetzten Nadeln/Düsen der beaufschlagten Fläche, beziehungsweise von der Intensität und Dauer des Vernadelungsprozesses. Neben einer Vernadelung mittels Nadeln besteht auch die Möglichkeit, dass die Verfestigung des Vlieses in einem sogenannten Wasserstrahlverfestigungsverfahren erfolgt. Hierbei werden, ähnlich, wie bei der Beaufschlagung des Vlieses mit Nadeln, beispielsweise über Düsen feine Wasserstrahlen in das Vlies eingeleitet und bewirken auch eine Durchdringung des Vlieses durch einzelne, Verfestigungs-fasern bildende Fasern des Vliesmaterials.

Eine bevorzugte Weiterbildung des erfindungsgemäßen Flächengebildes sieht vor, dass eine mechanische, chemische oder thermische Vernetzung und/oder Vernadelung der einzelnen Fasern beziehungsweise von Filamenten des Vlieses untereinander erfolgt. Hierbei sei darauf hingewiesen, dass ein Vlies aus lose zusammenliegenden Fasern, welche miteinander verbunden sind oder werden, gebildet ist. Vliese oder Vliesstoffe zeichnen sich durch das Vorhandensein von Fasern aus, die wirr im Vliesstoff angeordnet sind. Zur letztendlichen Bildung des Vlieses beziehungsweise Vliesstoffes bedarf es dann der oben genannten Vernetzung und/oder Vernadelung der einzelnen Fasern beziehungsweise Filamente des Vlieses untereinander. Im Weiteren werden die Begriffe Fasern und Filamente stets gemeinsam und als Synonyme füreinander verwendet. Als Fasern und Filamente angesehen werden im Zusammenhang mit der vorliegenden Erfindung aus Polymeren gebildete dünne langgestreckte Gebilde mit definiertem Durchmesser. Fasern weisen im Gegensatz zu Filamenten, die generell unendlich lang ausgebildet sein können, eine begrenzte Länge auf.

Als Verfestigungsverfahren für die Vliese beziehungsweise Vliesstoffe eignet sich beispielsweise eine mechanische, thermische oder chemische Behandlung, beispielsweise eine Verfestigung durch Vernadeln oder durch Wasserstrahlverfestigung/-vernadelung. Daneben besteht im Zuge einer chemischen Verfestigung die Möglichkeit Bindemittel oder Kleber zum Rohvlies beziehungsweise zum Ausgangsvliesmaterial hinzuzufügen, die dann eine Verbindung beziehungsweise Verfestigung der Vliesfasern durchführen oder gewährleisten. Neben den genannten mechanischen oder chemischen Verfahren oder parallel zu diesen besteht auch die Möglichkeit einer Verfestigung des Vlieses durch thermische Beaufschlagung des Roh- oder Ausgangsmaterials beziehungsweise des Roh- oder Ausgangsvliesen. Hierbei ist vorgesehen, dass die Fasern mit erwärmter oder erhitzter Luft beziehungsweise in einem erhitzten Gasstrom kontaktiert werden. Dies führt zu einem oberflächlichen Aufschmelzen der Fasern beziehungsweise des Fasermantels und im Anschluss daran zu einer Verklebung des sich verflüssigenden Mantelmaterials mehrerer eng beieinanderliegender Fasern. Hierbei ist es entscheidend, dass der Gasstrom beziehungsweise die Dauer der Beaufschlagung so eingestellt wird, dass kein vollständiges Schmelzen der Fasern, was deren Reißen zur Folge hätte, stattfindet, sondern nur die Verflüssigung der äußeren Schichten der Fasern eintritt. Ein neben dem Beaufschlagen mit einem erhitzten Gasstrom durchführbares Verfahren sieht vor, dass das Rohvlies zwischen beheizten Walzen hindurchgeführt wird und es dann hier an den Oberflächen der Walzen zu einem zunächst oberflächlichen Aufweichen der Fasern und deren anschließendem Verkleben kommt. Hierbei kann gleichzeitig eine Reduzierung der Vliesstärke durchgeführt werden.

Je nach Ausgangsmaterial des Vlieses und den entsprechenden chemischen Eigenschaften der Fasern besteht neben der Verwendung eines erhitzten Gasstroms aus die Möglichkeit, die Temperaturbeaufschlagung der Fasern über einen Dampfstrom durchzuführen. Die Wirkung ist mit der vorher beschriebenen Wirkung vergleichbar. Über die chemischen Eigenschaften der Fasern beziehungsweise bereits im Herstellungsprozess kann die letztendliche Struktur der Faser mit einem Faserkern und einem Fasermantel festgelegt werden. Hierbei kann die Faser so eingestellt oder ausgeführt sein, dass der Faserkern im Vergleich zum Fasermantel eine unterschiedliche Schmelztemperatur aufweist. Diese Eigenschaft der Faser kann dann wiederum beim thermischen Verfestigen des Rohvlieses zum Tragen kommen, nämlich dann, wenn über eine entsprechende Temperaturbeaufschlagung lediglich das Anschmelzen des Fasermantels, jedoch nicht ein Schmelzen des Faserkerns durchgeführt werden soll.

Erfindungsgemäß ist vorgesehen, dass das Vlies als Nano- oder Mikrofaservlies ausgebildet ist. Das Vlies kann dabei aus Feinstfasern oder Feinstfilamenten gebildet werden. Die Einteilung der Vliese in die entsprechenden Materialkategorien erfolgt dabei in Annäherung beziehungsweise in Abhängigkeit von der Feinheit beziehungsweise dem Durchmesser der das Vlies bildenden Fasern. Nachfolgend wird, unabhängig davon, ob ein Nano- oder Mikrofaservliese oder sonstige Vliese zum Einsatz kommen, stets von einem Vlies gesprochen werden. Es soll hier festgehalten werden, dass der Begriff Vlies auch eine Ausgestaltung als Nano- oder Mikrofaservlies umfasst, ohne dies in jeweils gesondert festzuhalten. Der Begriff Vlies bildet somit einen Oberbegriff, unter den alle weiteren Vliesarten fallen, ohne jeweils explizit genannt zu werden. Gleiches gilt für die in der zuletzt beschriebenen bevorzugten Ausführungsform verwendeten Feinstfasern oder Feinstfilamente. Diese stellen nur durch die Feinheit beziehungsweise den Faser- oder Filamentdurchmesser zu definierende Bezeichnungen dar und fallen ebenso unter den Oberbegriff Faser beziehungsweise Filament. Wird in der vorliegenden Beschreibung somit der Begriff Fasern beziehungsweise Filamente verwendet, so sind hier gleichbedeutet Feinstfasern und Feinstfilamente zu verstehen, ohne dies explizit festhalten zu müssen. Auf die Bedeutung der Feinheit der Fasern wird nachfolgend gesondert eingegangen werden.

Vorzugsweise wird das Nanofaservlies aus Feinstfasern oder -filamenten mit einem Durchmesser von weniger als 10 µm, besonders bevorzugt von weniger als 1 µm, hergestellt, vorzugsweise durch elektrostatisches Spinnen. Die Fasern oder Filamente der Nanofaservliese bestehen vorzugsweise aus thermoplastischem und hydrophilem oder hydrophiliertem, schmelzspinnfähigem Polymerisat, besonders bevorzugt aus Polyurethan. Gerade die Verwendung von Nanofaservlies macht sich das hohe Wasser- beziehungsweise Fluidretentionsvermögen der Faser als solches günstig bemerkbar, die durch die Verwendung mit dem Superabsorber zu einer vorteilhaften Synergie gesteigert ist.

Das erfindungsgemäße Flächengebilde ist darauf ausgelegt, eine mehrfache beziehungsweise wiederholte Aufnahme und gesteuerte Abgabe von Flüssigkeit zuzulassen. Dies bedeutet, dass die superabsorbierende Aktivierung dauerhaft mit dem Flächengebilde verbunden werden muss. Um eine dauerhafte Verbindung zwischen dem Flächengebilde, beziehungsweise dem im Flächengebilde angeordneten Vlies sowie zusätzlich oder alternativ den Fasern oder Filamenten des Vlieses zu gewährleisten, muss die superabsorbierende Aktivierung abriebsfest und mechanisch stabil mit den entsprechenden Bestandteilen des Flächengebildes verbunden sein. Alternativ wird dies insbesondere dadurch erreicht, dass die Fasern oder Filamente des Vlieses ganz oder zumindest teilweise aus superabsorbierenden Polymeren (SAP) bestehen, das heißt, als einer SAP-Fasern/SAP-Filamente ausgebildet sind.

Ein weiterer Vorzug des -wie vorgenannt- auf dem Vlies befestigten beziehungsweise mit den Fasern oder Filamenten verbundenen Superabsorberpolymeren liegt darin, dass eine relativ homogene und feine Verteilung des superabsorbierene Polymeres auf den jeweiligen Oberflächen im Vlies erreicht und stablisiert wird. Durch die homogene Verteilung des superabsorbierenden Polymeres im Vlies beziehungsweise in oder auf den Fasern oder Filamenten verhindert auch eine gegenseitige Abdeckung der Polymere, die mit einer verminderten Wasseraufnahme einhergehen würde.

Eine bevorzugte Ausführungsform des erfindungsgemäßen Flächengebildes sieht vor, dass das Vlies und zusätzlich oder alternativ die das Vlies bildenden Fasern oder Filamente eine Beaufschlagung mit wenigstens einem superabsorbierenden Aktivierungsmittel aufweist beziehungsweise aufweisen, wobei der Anteil des beaufschlagten superabsorbierenden Aktivierungsmittels am Vlies beziehungsweise den Fasern oder Filamenten zwischen 0 und 800 Massenprozent bezogen auf die flächenbezogene Masse des Vlieses beträgt. Es hat sich hierbei gezeigt, dass das Vlies in der Lage ist das bis zu achtfache seiner Masse an Aktivierungs-mittel aufzunehmen. Über den Anteil an superabsorbierendem Aktivierungsmittel können die Eigenschaften des Vlieses und darüber die des Flächengebildes entsprechend eingestellt werden. Eine derartige Einstellung erfolgt in Abhängigkeit von dem letztendlich vorgesehenen Verwendungszweck des Flächengebildes, wobei stets im Auge behalten wird, dass beispielsweise für die Verwendung zur kurzzeitigen Kühlung unter anderem von Menschen oder Körperteilen eine geringere Beaufschlagung mit superabsorbierenden Aktivierungsmaterial ausreicht, während im Interesse einer lang andauernden Abgabe von Flüssigkeit und damit einer langen Kühlung eine höhere Beaufschlagung mit superabsorbierenden Aktivierungsmittel ratsam ist. Ausgehend von dem oben genannten Werten für die Flächengewichte des im erfindungsgemäßen Flächengebilde verwendeten Vlieses wird durch eine beispielsweise 800 %ige Beaufschlagung eines Vlieses mit einer flächenbezogener Masse von 120 g/m² ein letztendliches Vliesgewicht von 1.080 g/m² erreicht.

In einer als günstig angesehenen Ausführungsform des erfindungsgemäßen Flächengebildes ist vorgesehen, dass zur Verbindung des Vlieses beziehungsweise der Fasern oder Filamente mit dem superabsorbierenden Aktivierungsmittel die Beaufschlagung des Vlieses oder der Fasern oder Filamente mit einem Klebemittel, insbesondere auf Basis von Polyacrylat, vorgesehen ist. In diesem Zusammenhang ist vorgesehen, dass der Anteil des beaufschlagten Klebemittels am Vlies zwischen 0 und 85 Massenprozent, bezogen auf die flächenbezogene Masse des Vlieses beträgt. Die Beaufschlagung mit einem Klebemittel führt zu einer dauerhaften, abriebsresistenten und mechanisch stabilen Verbindung von superabsorbierendem Aktivierungsmittel und Flächengebilde, da hier eine unmittelbare Verbindung von Vlies beziehungsweise Fasern oder Filamenten des Vlieses und Superabsorber stattfindet. Die auf Basis von Polyacrylat gebildeten Klebemittel erweisen sich auch nach mehrmaligem Einsatz des Flächengebildes beziehungsweise nach einer mehr oder weniger intensiven Reinigung des Flächengebildes, beispielsweise nach dessen Verschmutzung im Einsatz, sowie bei häufigen, starken Temperaturwechseln, als besonders dauerhaft und gewährleisten somit eine lange Haltbarkeit des Flächengebildes beziehungsweise eine lang andauernde Befestigung der Superabsorber im Flächengebilde.

Durch einen hohen Anteil an Klebemittel kann auch die Formhaltigkeit des Vlieses beeinflusst werden. Mit einem erhöhten Klebemittelanteil kann hier aus dem Vlies ein entsprechend aktivierbares und formstabiles Formteil gebildet werden, das auch insbesondere nach mehreren Wasch- beziehungsweise Reinigungsvorgängen die Form stabil zu halten vermag.

Im Interesse einer besonders innigen Verbindung zwischen dem Vlies beziehungsweise den das Vlies bildenden Fasern oder Filamenten und dem superabsorbierenden Aktivierungsmittel erweist es sich als empfehlenswert, wenn das Vlies beziehungsweise zusätzlich die Oberfläche der Fasern oder Filamente eine Beschichtung mit dem superabsorbierenden Aktivierungsmittel beziehungsweise dem Klebemittel aufweist. Die Beaufschlagung des Vlieses, dessen Oberfläche beziehungsweise der Fasern oder Filamente mit dem superabsorbierenden Aktivierungsmittel beziehungsweise dem Klebemittel kann dabei neben dem Sprühverfahren mit entsprechendem Druck und Düsengrößen in einem sogenannten Foulardierverfahren erfolgen. Hierbei wird das Rohvlies beziehungsweise werden die das Vlies bildenden Fasern durch ein das Klebemittel beziehungsweise die superabsorbierenden Aktivierungsmittel oder eine Mischung aus den letztgenannten enthaltendes Bad gezogen und dabei allseitig mit der entsprechend zusammengesetzten Lösung benetzt. Die superabsorbierenden Aktivierungsmittel beziehungsweise das Klebemittel haften anschließend an der Oberfläche des Vlieses beziehungsweise an den Oberflächen der Fasern oder Filamente an. Der nicht am Vlies beziehungsweise den Fasern gebundene Teil der Lösung wird durch Kalandrieren des Vlieses beziehungsweise der Fasern oder Faserstränge aus diesem abgepresst. Im Anschluss an das Foulardierverfahren kann eine Beaufschlagung des vorbehandelten Vlieses beziehungsweise der vorbehandelten Fasern oder auch eines entsprechend ausgebildeten Faserstranges mit einem superabsorbierenden Aktivierungsmittel erfolgen, sofern dieses nicht in der Foulardierlösung enthalten war. Die noch klebrige Oberfläche des Vlieses beziehungsweise der Fasern wird dabei mit als Pulver oder in Partikelform vorliegendem Superabsorber kontaktiert, beispielsweise bestreut oder aber wird der Superabsorber auf die Vliesbahn beziehungsweise die Fasern oder Faserstränge aufgeblasen. Das Klebemittel härtet anschließend aus und verbindet die Fasern dauerhaft mit dem Superabsorbens. Neben der Verwendung eines Foulardierverfahrens besteht auch die Möglichkeit, eine Lösung, die entweder nur Klebstoffe oder aber Klebstoffe und superabsorbierende Aktivierungsmittel enthält, auf das Vlies aufzusprühen beziehungsweise über die Anordnung entsprechender Düsen in das Vlies einzupressen oder aufzublasen. Neben einer entsprechenden Behandlung des Vlieses ist selbstverständlich auch die Beschichtung von das Vlies bildenden Fasern oder Filamenten im Sprühverfahren möglich, wobei hierbei auch eine Sprühlösung verwendet werden kann, die sowohl Klebemittel als auch superabsorbierendes Aktivierungsmittel oder aber nur Klebemittel enthält. Hierbei besteht dann auch die Möglichkeit, dass die nachträgliche Anhaftung von Superabsorber an dem sprühbeschichteten Vlies beziehungsweise den sprühbeschichteten Fasern erfolgt. Zum Aushärten des Klebemittels und einer weiteren Verbesserung der Verbindung zwischen Superabsorbern und Vlies beziehungsweise Fasern kann eine Trocknung der beschichteten Trägermaterialien, eine Vernetzung des Klebemittels, beispielsweise mit UV-Licht, oder ein Sintern oder Backen, oder einer sonstigen thermischen Behandlung der Fasern oder des Vlieses durchgeführt werden. Vorteil des vorgenannten Vorgehens ist, dass neben einer besonders innigen und dauerhaften Verbindung von Vlies beziehungsweise Fasern oder der Filamenten des Vlieses mit den Superabsorbern beziehungsweise Superabsorberpolymeren auch eine homogene Verteilung des Superabsorbers im Flächengebilde erreicht wird, die wiederum zu einer verbesserten, insbesondere schnellen Aufnahme und gleichmäßigen Abgabe der Flüssigkeit führt. Durch die Einstellung entsprechend geeigneter Konzentrationen an Superabsorberpolymer für die Beschichtung kann letztendlich die Menge und Verteilung von Superabsorberpolymer auf den Fasern oder Filamenten beziehungsweise im Vlies gesteuert werden. Die beispielsweise als Kristalle vorliegenden Superabsorberpolymere können dabei optimal beabstandet an den Fasern/Filamenten beziehungsweise im Vlies angeordnet werden, sodass hier deren maximale und schnelle Quellfähigkeit gewährleistet werden kann. Ebenfalls erreicht wird hier eine dauerhafte Verbindung von Superabsorberpolymer und Faser/Filament beziehungsweise Vlies, da ein Anhaften der superabsorbierenden Polymere direkt an der Faser durchgeführt wird. Die Superabsorberpolymere sind somit fester Bestandteil des Vlieses und nicht lose angeordnet und beispielsweise unter zur Hilfenahme von Klebemitteln lediglich in ihrer Position fixiert. Es ergibt sich so ein flexibles Material, das aus einem Verbund von superabsorbierend aktivierten Fasern/Filamenten oder Superabsorberpolymerfasern gebildet ist und indem die Superabsorberpolymere dauerhaft und ortsfest fixiert sind.

Eine bevorzugte Weiterbildung des erfindungsgemäßen Flächengebildes sieht vor, dass die Fasern oder Filamente, die letztlich das Vlies bilden, eine Garnfeinheit von 0,1 bis 20 dtex [Gramm/10.000 Meter] aufweisen. Über die jeweilige Garnfeinheit wird im Anschluss auch die Vlieseigenschaft definiert. So bestehen beispielsweise Nanofaservliese aus Fasern oder Filamenten mit einer Garnfeinheit von weniger als 0,3 dtex, während Mikrofaservliese Garnfeinheiten von 0,5 bis 0,7 dtex aufweisen. Mit zunehmender Masse pro Länge sinkt die Garnfeinheit und steigt letztendlich der Durchmesser der Filamente beziehungsweise Fasern. Ebenfalls über die Garnfeinheit der das Vlies bildenden Fasern oder Filamente wird auch die Fähigkeit des Flächengebildes zur Aufnahme eines superabsorbierenden Aktivierungsmittels festgelegt. Aufgrund der relativ gesehen großen Oberfläche von Vliesen mit niedrigen Faserdurchmessern, das heißt, mit relativ niedrigen Massen pro Länge, steigt die Möglichkeit zur Anordnung superabsorbierender Aktivierungsmittel und damit die Fähigkeit, Flüssigkeit aufzunehmen. Mit abfallender Garnfeinheit, das heißt, mit sich zunehmend vergrößerndem Faserdurchmesser hingegen sinkt, bei zunehmender Robustheit des Vlieses, die Möglichkeit für die Anordnung zusätzlicher superabsorbierender Aktivierungsmittel auf der Oberfläche der Fasern. Es besteht somit die Möglichkeit aus einer Vielzahl von unterschiedlich gestalteten Vliesmaterialien, dasjenige auszuwählen, das für den beabsichtigten Einsatzzweck am besten geeignet ist, um anschließend daraus das erfindungsgemäße Flächengebilde zu erstellen.

Als günstig wird angesehen, wenn die Fasern, Fasergruppen oder Filamente, die als Oberbegriff für jegliche Fasern, Fasergruppen oder Filamente unterschiedlicher Garnfeinheit zu verstehen sind, aus natürlichen Polymeren, insbesondere auf Basis von Baumwolle, Bambus, Hanf, Zellulose, mineralischen Fasern, oder aber aus künstlichen Polymeren, insbesondere thermoplastischen Polymeren, bevorzugt aus Polyethylen, Polyamid, Polyester oder Polypropylen oder aus Polyurethan, Polyvinylchlorid, Kunstseideacrylfasern oder aber aus biotechnologisch erzeugten Polymeren und/oder Mischungen oder Abfallfasern der vorgenannten Polymere gebildet sind. In diesem Zusammenhang wird es auch als empfehlenswert angesehen, wenn das Vlies aus einer Mischung verschiedener Fasern, Fasergruppen oder Filamenten gebildet ist, um hier ein besonders anpassungsfähiges beziehungsweise vielseitig einsetzbares Flächengebilde zur Verfügung stellen zu können. Als günstig erweist es sich, wenn die Mischung zwischen 25% und 100% Polymerfasern, zwischen 0% und 20% Abfallfasern und/oder 0% bis 10% niedrigschmelzende Polymerfasern umfaßt. Die niedrigschmelzenden Polymerfasern bestehen hierbei insbesondere aus den thermoplastischen Polymeren Polyethylen, Polyamid, Polyester oder Polypropylen und vermitteln, aufgrund der niedrigschmelzenden Eigenschaft einen verbesserten Zusammenhalt des Vlieses nach dem Durchführen eines thermischen Verfestigungsverfahrens für das Vlies. Die oben genannte Verwendung von Fasern oder Filamenten aus natürlichen Polymeren und hierbei insbesondere von Polymeren auf Basis von Baumwolle, Bambus, Hanf oder Zellulose bietet den Vorteil, dass diese Fasern, je nach Behandlung des entsprechenden Polymers selbst eine absorbierende Eigenschaft aufweisen, die dann durch die Hinzunahme von superabsorbierenden Aktivierungsmitteln weiter verbessert wird, was in einer noch günstigeren AusgangsSituation für die Flüssigkeitsaufnahme resultiert. Die Verwendung von künstlichen Polymeren sowie von biotechnologisch erzeugten Polymeren bietet den Vorteil, dass hier die Möglichkeit besteht, die Eigenschaften der Fasern oder Filamente beziehungsweise des daraus gebildeten Vlieses besonders genau und anwendungsorientiert einzustellen. Durch die geeignete Auswahl von Faserpolymeren kann auch sichergestellt werden, dass nur ein geringer Teil der im Flächengebilde aufgenommenen Flüssigkeit an den Oberflächen der Fasern anhaftet und nicht von den Superabsorbern aufgenommen werden kann. Die geeignete Auswahl von Polymeren und die darüber einstellbare Strukturierung der letztendlich gebildeten Fasern kann auch dazu beitragen, dass die in das Flächengebilde eingefüllte Flüssigkeit durch die Fasern auf die an den Fasern anhaftenden beziehungsweise mit den Fasern verbundenen Superabsorbern beziehungsweise Superabsorberpartikel herangeführt und durch diese aufgesaugt werden kann.

Die verwendeten Fasern, Fasergruppen oder Filamente weisen bevorzugt keine einheitliche Garnfeinheit auf. Vielmehr sind die vorgenannten Fasermaterialien aus Gruppen mit variablen Anteilen an Fasern, Fasergruppen oder Filamenten mit wiederum jeweils unterschiedlichen Garnfeinheiten gebildet. Die Garnfeinheiten der einzelnen Fasern können dabei bevorzugt im Bereich von 0,1 dtex bis 20 dtex liegen. Die Ausführungen und Eigenschaft des Vlieses können über die geeignete Kombination verschiedener Fasermaterialien und/oder von Fasern mit unterschiedlichen Garnfeinheiten definiert werden.

Eine vorteilhafte Weiterbildung des erfindungsgemäßen Flächengebildes sieht vor, dass die Fasern oder Filamente in Form von Bikomponentenfasern vorliegen. Hierbei handelt es sich insbesondere um eine Mischung von zwei Polymeren unterschiedlicher Schmelztemperatur, die insbesondere ausgewählt sind aus der Gruppe bestehend aus Polyethylen, Polyamid, Polyester, Polyurethan und Polypropylen, wobei die Gruppe durch die Hinzunahme weiterer sich als geeignet erweisender Polymere mit entsprechendem Schmelzverhalten beliebig erweiterbar ist. Durch die Verwendung von Polymere mit unterschiedlicher Schmelztemperatur kann ebenfalls die Eigenschaft des aus den jeweiligen Fasern gebildeten Vlieses genau eingestellt werden. Zudem kann in einem thermischen Verfestigungsprozeß, der letztendliche Zusammenhalt beziehungsweise die Verfestigung des Vlieses nahezu punktgenau eingestellt werden. Über den Anteil von unterschiedlichen Polymeren kann auch ein auf das jeweilige Einsatzgebiet abgestimmtes Flächengebilde zur Verfügung gestellt werden. In der Regel umfasst die Erfindung auch Flächengebilde, bei welchen die Fasern oder Filamente nur aus einem (homogenen) Polymer besteht. Dieses Polymer stammt dabei aus der vorgenannten Gruppe. Natürlich ist es auch möglich, die Fasern beziehungsweise Filamente des Nano- beziehungsweise Mikrovlieses ebenfalls aus der vorgenannten Gruppe zu wählen.

Als im Sinne der Erfindung günstig wird es angesehen, wenn das Flächengebilde Fasern oder Filamente aufweist, die durch Trockenspinnen, Naßspinnen, Schmelzspinnen, Matrixspinnen oder Elektrospinnen gebildet sind. Im Zuge dessen ist vorgesehen, dass eine superabsorbierende Aktivierung des Flächengebildes oder des aus den Fasern oder Filamenten gebildeten Vlieses vor, während oder nach dem Spinnvorgang erfolgt. Beim Naßspinnen erfolgt die Herstellung der Fasern aus einer entsprechenden Polymerlösung, die eines oder mehrere der oben genannten Polymere enthalten kann. Diese Polymerlösung wird in ein Fällbad mit einem Lösungsmittel oder Lösungsmittelgemisch mittels Düsen eingesprüht, wonach das Polymer beziehungsweise die Polymermischung ausfällt und eine Faser bildet. Beim Trockenspinnen werden ebenfalls gelöste Polymere eingesetzt und diese aus einer Düse extrudiert. Die dabei gebildeten Fasern beziehungsweise Filamente koagulieren an der Luft oder in Stickstoff und das Lösungsmittel für die Polymere verdampft dabei. Das Trockenspinnverfahren eignet sich für Polymere, die in einem leicht flüssigen Lösungsmittel löslich sind. Beim Schmelzspinnen werden die Polymere aufgeschmolzen und durch eine oder mehrere Düsen gepreßt. Beim Austritt aus den Düsen bildet sich dann die Polymerfaser, die auf einer Spindel oder Spule aufgewickelt und für die nachträgliche Bildung von Vliesen zur Verfügung gestellt werden kann. Daneben besteht auch die Möglichkeit, die aus den Düsen austretenden Polymerfasern oder Filamente direkt auf einen Träger aufzubringen und hierbei das Vlies zu bilden. Je nach Ausgestaltung der Düse beziehungsweise bei Anordnung von mehreren Düsen besteht hier auch die Möglichkeit, verschiedene Polymere gleichzeitig aus den unterschiedlichen Düsen zu pressen und somit ein Vlies zu bilden, das aus mehreren Polymeren beziehungsweise aus Fasern oder Filamenten, die aus verschiedenen Polymeren bestehen, besteht. Hierüber können auch die Eigenschaften des Vlieses eingestellt werden. Als weitere Möglichkeit insbesondere zur Bildung von besonders dünnen Fasern aus Polymerlösungen steht das sogenannte Elektrospinnen zur Verfügung. Hierbei werden die Polymerlösungen in einem elektrischen Feld behandelt. Das Elektrospinnverfahren eignet sich für die Bildung von Nanofasern mit Durchmessern kleiner 1000 Nanometern. Derartige Fasern eignen sich dann zur Bildung von Nanofaservliesen. Je nach Auswahl des geeigneten Spinnverfahrens kann eine Einfügung der superabsorbierenden Aktivierung in das Flächengebilde beziehungsweise das Vlies vor, während oder nach dem Spinnvorgang erfolgen. Ist eine superabsorbierende Aktivierung beziehungsweise deren Einbringung vor dem Spinnvorgang vorgesehen, so erfolgt eine Beaufschlagung der jeweils verwendeten Polymerlösung mit einem superabsorbierenden Polymermaterial und die anschließende Verspinnung der so gebildeten Polymerlösung. Bei einer superabsorbierenden Aktivierung während des Spinnvorganges kann beispielsweise im Lösungsmittelbad, bei Verwendung von Naßspinnverfahren, ein superabsorbierendes Polymer oder entsprechend weitere superabsorbierende Aktivierungsmittel eingebracht werden. Diese werden dann beim Ausfallen der Polymerlösung im Fällbad mit den sich bildenden Fasern verbunden. Beim Schmelzspinnverfahren besteht neben der Einbringung von superabsorbierenden Aktivierungsmitteln in die auszudüsenden Polymerlösung auch die Möglichkeit, eine zusätzliche Düse vorzusehen, über die superabsorbierendes Polymer beziehungsweise ein sonstiges Aktivierungsmittel zusammen mit der Polymerlösung ausgedüst und auf eine entsprechende Trägerbahn aufgebracht wird. Dies bietet den Vorteil, dass die superabsorbierenden Aktivierungsmittel, und hierbei insbesondere superabsorbierende Polymere, unmittelbar und mechanisch in das sich bildende Vlies eingebracht werden können, und im Stapelprozeß mit den Fasern oder Filamenten des Vlieses verankert werden.

Als vorteilhaft wird angesehen, wenn die superabsorbierende Aktivierung durch wenigstens ein auf oder in die Fasern oder Filamente eingebrachtes beziehungsweise in das Vlies auf- oder eingebrachtes superabsorbierendes Polymer erfolgt. Daneben beziehungsweise zusätzlich besteht auch die Möglichkeit, dass die Fasern oder Filamente ganz oder teilweise aus wenigstens einem superabsorbierenden Polymer gebildet sind. Eine entsprechende Ausführungsform des Flächengebildes ergibt sich beispielsweise dann, wenn, wie oben ausgeführt, eine Koextrusion von superabsorbierendem Polymer und Faserpolymer durchgeführt wird. Gleiches gilt, wenn die superabsorbierenden Polymere in die Polymerlösungen, aus denen anschließend die Fasern beziehungweise Filamente gebildet werden, eingemischt sind. Durch gegebenenfalls entsprechende chemische Modifikation der superabsorbierenden Polymere besteht selbstverständlich auch die Möglichkeit, dass die Bildung von Fasern aus superabsorbierendem Polymer in den vorgenannten Spinnverfahren erfolgt.

Das superabsorbierende Polymer liegt vorteilhafterweise in Form von Partikeln vor, die günstigerweise Durchmesser zwischen 45 und 875 µm, insbesondere zwischen 326 und 674 µm, bevorzugt jedoch zwischen 400 und 500 µm aufweisen. Der Vorteil einer gleichmäßigen Durchmesserverteilung beziehungsweise einer klaren Fraktionierung der Partikel liegt zum Einen in der dadurch gegebenen Möglichkeit zur relativ homogenen Bestückung der Fasern beziehungsweise Vliese mit Superabsorberpolymer, zum Anderen darin, dass hierüber die durch die Partikel zur Verfügung gestellte absorptionswirksame Oberfläche definiert und auf den entsprechenden Verwendungszweck genau abgestimmt werden kann. Die verbesserte Fraktionierung verbessert die Flüssigkeitsaufnahmekapazität und -geschwindigkeit, da hierdurch eine gleichmäßige SAP-Verteilung realisiert wird und die Polymerpartikel oder -kristalle Flüssigkeit gleich schnell und in gleicher Menge aufnehmen ohne sich gegenseitig gegenüber der aufzunehmenden Flüssigkeit abzuschirmen beziehungsweise von der Flüssigkeitsversorgung zu trennen.

In einer anderen erfindungsgemäßen Variante ist vorgesehen, dass die Partikel insbesondere einen Durchmesser zwischen 50 µm und 150 µm aufweisen. Diese deutlich kleineren Partikel haben insbesondere im Zusammenwirken mit Mikro- oder Nanofasern, die zum Beispiel in einem Elektrospinnverfahren hergestellt worden sind, erhebliche Vorteile. Das Zusammenwirken des sehr viel feineren Fasermaterials der Mikro- oder Nanofaser mit den kleineren Superabsorberpolymerpartikeln in dem angegebenen Bereich, erleichtert erheblich die hydrophile Wirkung des Flächengebildes einerseits, aber auch die Ladebeziehungsweise Entladegeschwindigkeit andererseits. Dabei sind die Superabsorberpolymere klein genug, dass kein Gel-blocking-Effekt auftritt, also gewisse Bereiche des Superabsorberpolymeres an der Wasserspeicherung nicht teilnehmen. Gleichzeitig ist eine dermaßen mechanisch stabile Verbindung vorgesehen, dass die Superabsorberpolymerpartikel in hoher Güte an dem Fasermaterial verbleiben. Auch hier ist der kleinere Durchmesser der Partikel hilfreich. Die Verwendung von Partikeln mit kleinem Durchmesser ist dabei nicht auf Mikro- oder Nanofasern beschränkt.

Unter dem Durchmesser, den die Partikel bevorzugt aufweisen, ist somit ein Äquivalentdurchmesser zu verstehen, der sich beim Durchgang der Partikel durch ein Sieb anhand der Lochweite des Siebes feststellen läßt. Als günstig wird angesehen, wenn eine Mischung von Partikeln mit verschiedenen Durchmessern verwendet wird. Durch die Verwendung derartiger Mischungen können verschieden große Zwischenräume im Vlieskörper, die sich aufgrund einer ungeordneten Stapelung der Fasern beziehungsweise Filamente bilden, gleichmäßig durch die Partikel besetzt werden und somit ausschließend eine gleichförmige Verteilung der Flüssigkeit im Vlies beziehungsweise im Flächengebilde sichergestellt werden. Als vorteilhaft erweist es sich, wenn die Partikel kubisch, stäbchenförmig, polyedrisch, kugelig, abgerundet, winkelig, nadelartig, flocken- oder faserförmig oder dergleichen ausgebildet sind und hierbei insbesondere in Form von Pulvern, Kügelchen, Flocken oder ebenfalls als Fasern oder Molekül- beziehungsweise Partikelketten vorliegen. Die letztendliche Ausgestaltung der Partikel richtet sich nach der Wahl der verwendeten Fasern oder Filamente beziehungsweise nach dem letztendlichen Einsatzgebiet des Flächengebildes. Auch entscheidend für die Auswahl der jeweiligen Partikelform ist das Verfahren, mit dem eine Verbindung zwischen den superabsorbierenden Polymerpartikeln und den Fasern beziehungsweise Filamenten oder dem Flächengebilde beziehungsweise dem im Flächengebilde verwendeten Vlies vorgesehen ist. So wird beispielsweise bei einer rein mechanischen Verbindung von Partikeln und Fasern oder Filamenten beziehungsweise Vlies eine stäbchenförmige, nadelartige oder polyedrische Form der Partikel zu bevorzugen sein, während bei einer Verklebung beziehungsweise oberflächlichen Anhaftung der Partikel an den Fasern oder Filamenten und/oder dem Vlies eine kugelförmige, flocken- oder faserförmige oder nadelartige Ausführung der Partikel günstig erscheint, da hierbei eine vergrößerte Oberfläche, die mit dem Haftungsvermittler benetzt beziehungsweise kontaktiert wird, zur Verfügung steht. Generell können jedoch alle denkbaren Partikelformen mit allen Faser/Filament- beziehungsweise Vliesformen und Herstellungsverfahren verwendet werden.

In einer als günstig angesehenen Weiterbildung der Erfindung ist vorgesehen, dass das superabsorbierende Polymer ein chemisch vernetztes Copolymer aus Acrylsäure und einem ihrer Salze, insbesondere Natriumacrylat ist. Aus diesen Bestandteilen bilden sich in einer Polymerisationsreaktion lange Ketten, die über zusätzlich eingefügte Vernetzer zu einem lockeren, aber doch wasserunlöslichen Molekülknäuel vernetzt werden. Eine Vernetzung des Copolymers kann hierbei auch erst nach der Verbindung von superabsorbierendem Ausgangspolymer (Precusor) und dem Flächengebilde beziehungsweise den Fasern oder Filamenten erfolgen. Der Vernetzer wird dann erst nach einer entsprechenden Vermischung von superabsorbierendem Polymer und Fasern beziehungsweise Filamenten oder Flächengebilde beziehungsweise Vlies dem behandelten Material zugegeben. Im Zuge der nachträglichen Vernetzung beziehungsweise der nach Zufügung des Vernetzers einsetzende Polymerisationsreaktion der superabsorbieren-den Polymere erfolgt eine innige Verbindung zwischen dem superabsorbierenden Polymer und dem Flächengebilde beziehungsweise den Fasern oder Filamenten und/oder dem daraus gebildeten Vlies. Als günstig wird in diesem Zusammenhang angesehen, wenn das superabsorbierende Polymer beziehungsweise die superabsorbierenden Polymerpartikel die Fasern oder Filamente umlagert/umlagern beziehungsweise einschließt einschließen oder alternativ vollständig oder wenigstens teilweise, das heißt, über einen bestimmten Längenbereich der Fasern oder Filamente hin, beschichtet/beschichten. Diese Ausführungsform der Fasern oder Filamente erweist sich als vorteilhaft, da so bereits eine innige Verbindung von superabsorbierendem Polymer beziehungsweise den superabsorbierenden Polymerpartikeln und den Fasern oder Filamenten gebildet wird, die sich anschließend dazu eignet, das Flächengebilde beziehungsweise ein durch das Flächengebilde umfasstes Vlies superabsorbierend dauerhaft zu aktivieren.

Es besteht eine Vielzahl von Verfahren zur Bildung von Vliesen, und hierbei insbesondere zur Bildung von Nano- oder Mikrofaservliesen, die durch den Oberbegriff Vlies gleichfalls umfaßt sind. So wird es als vorteilhaft angesehen, wenn im Zusammenhang mit dem erfindungsgemäßen Flächengebilde das Vlies als Wirr-, Spinn- und/oder Stapelfaservlies ausgebildet ist. Die Anwendung eines entsprechenden Vliesbildungsverfahrens ist abhängig von der späteren geplanten Verwendung des Vlieses beziehungsweise der letztendlichen Ausgestaltung des das Vlies beinhaltenden Flächengebildes. Die Anwendung eines Wirrvliesverfahrens bietet sich hierbei insbesondere an, da das superabsorbierende Polymer hier mit besonders hoher Homogenität in das Vlies eingearbeitet werden kann. Im Herstellungsverfahrens für das Wirrvlies werden die für die Faserherstellung verwendeten Polymere, die verflüssigt vorliegen, bevorzugt aus einer Düse gepresst und in einem Luftstrom abgekühlt. Durch diesen Luftstrom wird das sich bildende Filament beziehungsweise die gebildete Faser auf ein als Sieb ausgebildetes Förderband befördert und mittels Absaugung an diesem sogenannten Siebband fixiert. Gleichzeitig mit dem Ausstoß des Polymers aus der Spinndüse kann eine Beaufschlagung des Luftstroms beziehungsweise des Förderbandes mit dem superabsorbierenden Polymer beziehungsweise mit entsprechenden Partikeln erfolgen. Diese werden durch die ebenfalls auf dem Siebband auftreffenden Filamenten in dem sich bildenden Vlies eingewoben oder eingesponnen und in ihrer Position fixiert. Um das letztendliche Eigenschaftsprofil des gebildeten Vlieses festzulegen, kann anschließend eine Vernadelung beziehungsweise eine wie vorhergehend bereits beschriebene Verfestigung des gebildeten Vlieses durchgeführt werden. Bei der Herstellung eines Vlieses als Stapelfaservlies werden die Fasern in einer sogenannten Krempel zu einem Vlies gelegt. Die Krempel fügt dabei lose einzelne Fasern zu einem Faserflor, dem Vlies, zusammen und richtet diese Fasern parallel zueinander aus. Auch hier kann das superabsorbierende Aktivierungsmittel in gleicher Weise in das Vlies eingebunden werden. Nach der Bildung des Rohvlieses kann eine Verfestigung des Vlieses in den bereits beschriebenen Verfahren erfolgen. Die Erfindung bleibt nicht auf die in den zuvor beschriebenen Verfahren gebildeten Vliese beschränkt, sondern bezieht sich in gleicher Weise auf sämtliche Vliese, die in geeignet erschein-den Herstellungsverfahren produziert werden und erstreckt sich auch auf aus den Fasern oder Filamenten gewebte, gestrickte oder in sonstiger Weise gewirkte Flächengebilde. Es besteht hierbei selbstverständlich auch die Möglichkeit, dass bereits aktivierte, das heißt mit superabsorbierendem Polymer beaufschlagte Fasern oder Filamente für die Bildung der Vliese zum Einsatz kommen und diese somit superabsorbierend aktivieren.

Bevorzugt kann eine mechanische Verbindung von superabsorbierendem Polymer beziehungsweise den aus dem superabsorbierenden Polymer gebildeten Partikeln und den Fasern oder Filamenten des Vlieses beziehungsweise des aus dem Vlies gebildeten Flächengebildes dadurch erreicht werden, dass die Partikel durch die kreuzweise übereinander gelegten Fasern oder Filamente im Flächengebilde gehalten beziehungsweise insbesondere in dieses eingesponnen werden. Auch diese Ausführungsform des erfindungsgemäßen Flächengebildes gewährleistet eine besonders dauerhafte und haltbare, insbesondere abriebs- und auch waschfeste Verbindung zwischen dem superabsorbierenden Polymer und dem Vlies beziehungsweise den Fasern oder den Filamenten des Vlieses und somit mit dem Flächengebilde. Diese Verbindung kann gegebenenfalls durch die zusätzliche, insbesondere gleichzeitige oder nachträgliche Beaufschlagung mit einem Klebemittel verbessert werden. Als besonders günstig wird in diesem Zusammenhang auch angesehen, wenn das Flächengebilde für eine mehrfache Benutzung vorgesehen ist. Hierbei bietet es sich auch an, dass das Flächengebilde waschbar und zwar insbesondere maschinenwaschbar oder chemisch reinigbar ausgebildet ist, und auch einer desinfizierenden oder sterilisierenden Behandlung standhält. Diese Eigenschaften des Flächengebildes werden durch die besonders dauerhafte Verbindung zwischen superabsorbierendem Polymer, das in Form einer Beschichtung, von eingesponnenen Partikeln, von mit den Fasern beziehungsweise dem Vlies oder der Vliesoberfläche verklebten superabsorbierenden Polymeren oder Partikeln daraus durchgeführt wird. Bei der Verwendung von Klebemitteln muss Wert darauf gelegt werden, dass diese mehrere Waschvorgänge beziehungsweise desinfizierende oder sterilisierende Behandlungen des Flächengebildes aushalten und durch die verwendeten Wasch-, Reinigungs- oder Desinfektionsmittel beziehungsweise Verfahren und/oder durch die Temperaturbeaufschlagung während des Wasch- oder Sterilisationsvorganges nicht aus dem Flächengebilde gelöst werden können.

Als besonders empfehlenswert wird im Interesse einer besonders innigen Verbindung von superabsorbierenden Polymerpartikeln und Fasern oder Filamenten eine Strukturierung oder Beschichtung der Oberfläche der Partikel beziehungsweise der Fasern oder Filamente angesehen. Diese Beschichtung beziehungsweise Strukturierung der Oberfläche kann dabei dergestalt ausgeführt sein, dass die Oberflächenstrukturierung oder -beschichtung eine Adhäsionswirkung entfaltet. Die Strukturierung kann in die jeweiligen Oberflächen durch chemische oder mechanische Behandlung eingebracht werden. Die Oberflächenstrukturierung ist dabei insbesondere in Form einer Nanostrukturierung oder - beschichtung ausgebildet. Die Ausführung der Partikel beziehungsweise Fasern oder Filamente mit der genannten Strukturierung oder Beschichtung der Oberfläche weist den Vorteil auf, dass die Verbindung zwischen Fasern und Filamenten und damit von dem Vlies und den superabsorbierenden Polymeren beziehungsweise den daraus gebildeten Partikeln ohne die zusätzliche Verwendung von Klebemitteln oder sonstigen haftungsvermittelnden Stoffen durchgeführt werden kann und auch eine Abnutzung der Strukturierung im Verlauf von Wasch- oder Desinfektions- beziehungsweise Sterilisationsvorgängen nicht oder nur in begrenztem Maße stattfindet, somit die Verbindung zwischen Fasern und Filamenten und superabsorbierender Aktivierung dauerhaft und besonders haltbar und abriebfest ausgebildet ist.

Eine als vorteilhaft angesehene Ausführungsform des erfindungsgemäßen Flächengebildes sieht vor, dass wenigstens eine Oberfläche des Flächengebildes aus von dem übrigen Flächengebilde abweichendem oder aus dem gleichen Material gebildet ist, wobei die Oberfläche dann Eigenschaften aufweist, die von den übrigen Eigenschaften des Flächengebildes abweichen. Als günstig erweist es sich, wenn die Oberfläche des Flächengebildes durch eine mechanische, thermische und/oder chemische Nachbehandlung und hierbei insbesondere durch eine mechanische Vernadelung beispielsweise durch eine Behandlung mit Wasserstrahlen oder Nadelleisten/-balken der entsprechenden Oberfläche des Vlieses gebildet ist. Durch die in der bevorzugten Ausführungsform beschriebene Oberflächenausbildung kann ein äußerer Abschluss des Flächengebildes erreicht werden, ohne dass hier zusätzliche Materiallagen oder Beschichtungen aufgebracht werden müssen. Das Flächengebilde besteht dann vollständig nur aus dem vorstehend bereits beschriebenen Vlies, das eine Oberflächenbehandlung erfahren hat. Als günstig wird in diesem Zusammenhang auch angesehen, wenn die Oberfläche durch ein nichtsuperabsorbierend aktiviertes Vlies, eine Membran, eine Folie, einen Stoff beziehungsweise eine Stoffbahn oder eine Beschichtung gebildet ist, wobei hierbei insbesondere die Oberfläche fest oder lösbar mit dem übrigen Material des Flächengebildes verbunden ist. So besteht hier auch die Möglichkeit, dass ein wie vorstehend beschriebenes Vlies, zusätzlich mit einem weiteren Vlies, das jedoch keine Aktivierung aufweist, belegt wird. Anstelle eines Vlieses kann hier auch eine Membran, ein Stoff oder eine Stoffbahn verwendet werden, wodurch gleichzeitig eine weitere Aktivierung des gesamten Flächengebildes dahingehend durchgeführt wird, dass die Membran, der Stoff oder die Stoffbahn eine Struktur aufweist, die beispielsweise das Entweichen von Wasserdampf aus dem Flächengebilde ermöglicht, nicht jedoch den Eintritt von Wasser. Die Anordnung einer derartigen Membran, eines Stoffes oder einer Stoffbahn auf beispielsweise einer Außenseite des Flächengebildes, bewirkt dessen zumindest einseitige Wasserdichtheit, ohne dabei die sonstigen Eigenschaften des Flächengebildes zu beeinträchtigen. Neben der Anordnung einer Membran, eines Stoffes oder einer Stoffbahn besteht erfindungsgemäß auch die Möglichkeit eine Folie als Oberfläche für das Flächengebilde zu verwenden. Hierdurch wird ein vollständiger Abschluss der Oberfläche gegen den Eintritt von Wasser und den Austritt von Wasserdampf gewährleistet, sodass beim Verdunsten der Flüssigkeit, die in den superabsorbierenden Polymeren gespeichert ist, eine gesteuerte beziehungsweise gerichtete Abführung des anfallenden Wasserdampfes durchgeführt werden kann.

Diese Ausführungsform des erfindungsgemäßen Flächengebildes ermöglicht dessen Einsatz in unter Kontaminationsgesichtspunkten kritischen Umgebungen. Die als Oberfläche verwendete Folie verhindert einen unmittelbaren Kontakt des Vlieses beziehungsweise des Inneren des Flächengebildes mit der belegten, zu kühlenden Fläche. Den gleichen Effekt bewirkt eine Beschichtung der Oberfläche beziehungsweise eine Ausbildung der Oberfläche als Beschichtung. Durch die geeignete Wahl des Beschichtungs-mittels kann zusätzlich eine weitergehende Aktivierung des Flächengebildes beziehungsweise dessen Oberfläche durchgeführt werden, auf die später detailliert eingegangen werden soll. Als weiteres Merkmal der bevorzugten Weiterbildung des erfindungs-gemäßen Flächengebildes wird angesehen, dass die Oberfläche fest oder lösbar mit den übrigen Materialien des Flächengebildes verbunden ist. Während eine lösbare Verbindung der Oberfläche beziehungsweise des oberflächenbildenden Materials mit dem übrigen Material des Flächengebildes eine erhöhte Flexibilität des Flächengebildes zulässt, bewirkt die unlösbare Verbindung von Oberfläche und beispielsweise Vliesmaterial einen wesentlich kompakteren Aufbau des gesamten Flächengebildes und verhindert beziehungsweise erschwert gleichzeitig eine Beschädigung des Flächengebildes beziehungsweise ein Reißen oder Beschädigen der Oberfläche. Eine weitere bevorzugte Ausführungsform der Erfindung sieht vor, dass die Oberfläche durch An- oder Verschmelzen der Fasern oder Filamente miteinander gebildet ist. Hierbei werden keine zusätzlichen Lagen am Flächengebilde angeordnet, sondern aufgrund einer Modifizierung oder Veredelung der Oberfläche der Abschluss des Flächengebildes hergestellt. Je nach Grad der Verschmutzung kann hier auch eine undurchlässige Oberfläche gebildet werden. Eine besonders günstige Ausgestaltung sieht dabei vor, dass das An- oder Verschmelzen der Fasern oder Filamente zur Bildung der Oberfläche in Verbindung mit einer Vernadelung durchgeführt wird. Hierdurch wird nicht nur ein Abschluss des Flächengebildes durchgeführt, sondern die Oberflächen- beziehungsweise Abschluss-Schicht zusätzlich durch die Vernadelung verfestigt. Im gleichen Sinne wie eine Vernadelung wirkt hierbei auch eine Wasserstrahlbehandlung der Oberfläche. Auch besteht die Möglichkeit, dass zunächst die oberflächenahen Schichten des Vlieses vernadelt und anschließend einer thermischen Behandlung unterzogen werden, wobei die Fasern beziehungsweise die Faseroberflächen angeschmolzen und im verflüssigten Zustand beziehungsweise nach teilweiser Verflüssigung oder Aufweichung der-Oberflächen der Fasern verbunden werden.

Eine weitere Ausführungsform der Erfindung sieht vor, dass die Oberfläche permeabel ausgebildet ist und Poren für den Durchtritt von Flüssigkeiten und/oder Gasen, insbesondere von Wasser, Alkohol, Alkohol-/Wassergemischen und/oder Wasser-, Alkohol- oder Wasser/Alkoholgemischdampf aufweist. Durch diese Ausgestaltung der Oberfläche kann der Kühleffekt des Flächengebildes weiter verbessert werden, da der bei der Verdunstung der in den superabsorbierenden Polymeren gespeicherten Flüssigkeit entstehende Dampf aus dem Flächengebilde heraus abgegeben werden kann. Die Permeabilität der Oberfläche für Flüssigkeit ermöglicht gleichzeitig auch das problemlose Beladen des Flächengebildes durch Eintauchen oder Besprühen mit der Flüssigkeit. Aufgrund dessen, dass die Porengröße kleiner ausgebildet ist als der Minimaldurchmesser der superabsorbierenden Partikel, die im Flächengebilde gegebenenfalls verwendet sind, wird verhindert, dass Partikel, die sich trotz der vorgesehenen dauerhaften Verbindung mit den Fasern oder Filamenten beziehungsweise dem Vlies lösen, aus dem Flächengebilde herausfallen können. Hierdurch wird eine dauerhafte und gleichbleibende Kühlleistung des Flächengebildes gewährleistet. Die Oberfläche, die, wie bereits vorher angemerkt, als Membran oder Folie ausgebildet werden kann, verhindert gleichzeitig das bei eine durch die abfallenden Partikel bedingte Gelbildung, die entsteht, wenn sich mehrere Partikel, die sich zu einem Konglomerat zusammengefügt haben, mit Flüssigkeit in Kontakt kommen, an der Außenseite, das heißt, an der dem Nutzer des Flächengebildes zugewandten Oberfläche spürbar wird und gegebenenfalls als störend empfunden wird.

Als empfehlenswert wird angesehen, wenn das Flächengebilde eine Kompartimentierung aufweist. Diese Kompartimentierung kann durch Einsteppen, Abnähen, Abkleben oder Schweißen der Oberfläche des Flächengebildes erfolgen. Durch eine derartige Kompartimentierung des Flächengebildes wird eine verbesserte Anpassbarkeit des Flächengebilde an die jeweils zu kühlende Oberfläche erreicht. Zudem kann verhindert werden, dass sich Partikel, die sich von dem Flächengebilde beziehungsweise aus dem Vlies oder den das Vlies bildenden Fasern oder Filamenten lösen, im gesamten Flächengebilde verteilt werden und sich hier Punkte oder Bereiche bilden, an denen sich die Partikel sammeln und anhäufen. Es kann durch die Kompartimentierung des Flächengebildes somit auch bei sich ablösenden Partikeln eine gleichbleibende und vor allem über das gesamte Flächengebilde verteilte Kühlung erreicht werden. Das Einsteppen, Abnähen oder Abkleben des Flächengebildes zur Bildung der Kompartimentierung kann dabei nachträglich, das heißt, nach der Fertigstellung des Flächengebildes, beispielsweise auch nach dem Auflegen oder Anordnen von zusätzlichen (Deck-) lagen auf oder im Flächengebilde erfolgen. Gleichzeitig besteht auch die Möglichkeit, dass die Kompartimentierung im Verlauf der Vliesbildung beziehungsweise der Modifizierung oder Veredelung einer oder mehrerer der Oberfläche des Flächengebildes oder Vlieses mit eingebracht wird. Insbesondere das Verschweißen der Oberfläche zur Bildung der Kompartimentierung beziehungsweise um zu einer weniger durchlässigen Oberfläche zu gelangen kann im Zuge einer thermischen Behandlung der Vliesoberfläche beziehungsweise der Oberfläche des Flächengebildes in einem Arbeitsgang durchgeführt werden. Bei der Kompartimentierung des Flächengebildes kann auch eine Festlegung der im Übrigen lösbar mit dem Flächengebilde verbundenen Oberfläche, die beispielsweise aus einer Membran, einer Stoffbahn eines Netzstoffes, einer Folie oder dergleichen, aber auch aus einem zusätzlich aufgebrachten oder verbundenen, nicht superabsorbierend aktivierten Vlies oder sonstigen geeignet erscheinenden Materialien gebildet ist, an den Nähten beziehungsweise Schweißstellen mit dem Flächengebilde erfolgen.

Um eine Möglichkeit zu schaffen, das Flächengebilde in einer Vielzahl von Umweltbedingungen anzuwenden und gleichzeitig das Flächengebilde für eine Vielzahl von Verwendungszwecken zur Verfügung stellen zu können, wird es als günstig angesehen, wenn wenigstens eine Oberfläche des Flächengebildes eine zusätzliche Aktivierung aufweist. Eine derartige Aktivierung kann neben den aus dem Stand der Technik bekannten chemischen oder physikalischen Ausrüstungsmöglichkeiten im Textilbereich auch über eine wie nachfolgend beschriebene Ausrüstung der für die Bildung der Oberfläche verwendeten Materialien, insbesondere von Vliesen, Membranen, Stoffen oder Stoffbahnen sowie Folien erreicht werden. Unter dem Begriff Ausrüstung ist im Zusammenhang mit der vorliegenden Erfindung eine Veredelungsmaßnahme an den Ausgangsmaterialien des Vlieses, seiner Fasern oder Filamente und/oder der die Oberfläche bildenden Stoffe, Garne, Folien oder Fasern zu verstehen, um den Gebrauchswert und die Materialeigenschaften des fertigen Materials beziehungsweise der Umhüllung an den jeweils vorgesehenen Einsatzzweck anzupassen beziehungsweise zu optimieren.

Darüber hinaus eignet sich das Flächengebilde auch zur Verwendung in der Feuerbekämpfung beziehungsweise im Feuer- oder Hitzeschutz, wobei sich hierbei eine flammfeste beziehungsweise feuerfeste, funkenflugresistente und/oder gegenüber Metall/Glutspritzern resistente Aktivierung/Ausrüstung der Oberfläche beziehungsweise der Materialien anbietet, die ein Verbrennen beziehungsweise eine Beschädigung der Oberfläche auch dann verhindert oder verzögert, wenn keine Restflüssigkeit mehr in den superabsorbierenden Polymeren beziehungsweise dem Flächengebilde vorhanden ist. Die entsprechend aktivierten Flächengebilde eignen sich dann auch für den Einsatz in Hitzeschutzabdeckungen und Schutzbekleidung für Berufe in denen mit glühenden oder hocherhitzten Flüssigkeiten hantiert werden muss, wie beispielsweise in Schmelzenbeziehungsweise Eisen-oder Glashütten. Wie auch bei der Brandbekämpfung im Feuerschutz und auch zur Verhinderung von Flächenbränden.

Die Oberfläche kann daneben eine hydrophilisierende Aktivierung/Ausrüstung aufweisen, um hierüber die Beladung der Umhüllung beziehungsweise des Flächengebildes zu verbessern und/oder zu beschleunigen. Somit können Stoffe, Gewebe oder Materialien, die herstellungs- oder einsatzbedingt nicht auf die Aufnahme von Flüssigkeit beziehungsweise Wasser ausgelegt sind, hierfür aktiviert beziehungsweise ausgerüstet werden. Die derartige Ausrüstung erlaubt die Nutzung der ursprünglich mechanischen Eigenschaften der Materialien bei gleichzeitiger Verwendung des Flächengebildes und/oder der Umhüllung im Sinne der vorliegenden Erfindung. Die Hydrophilisierung kann beispielsweise bei in der Feuerbekämpfung zwischen als Feuer- oder Hitzeschutz eingesetzten Geweben oder Stoffen durchgeführt werden, da die hier in der Regel verwendeten Materialien normalerweise keine bis nur geringe Wasseraufnahmefähigkeit aufweisen, nach entsprechender Ausrüstung jedoch eine Flüssigkeit aufnehmen, speichern und an die superabsorbierenden Aktivierungen weiterleiten können.

Um eine besonders einfache Wiederverwertung beziehungsweise Wiederverwendung des Flächengebildes durchführen zu können, weist wenigstens eine Oberfläche des Flächengebildes eine selbstreinigende Aktivierung/Ausrüstung auf. Diese selbstreinigende Aktivierung/Ausrüstung kann beispielsweise durch eine Beschichtung mit Nanopartikeln beziehungsweise unter Beigabe von Nanopartikeln zum Ausgangsmaterial durchgeführt werden. Daneben besteht auch die Möglichkeit, dass die Oberfläche schmutzabweisend aktiviert/ausgerüstet ist, wobei hier alle Techniken zum Einsatz kommen können, die bereits aus dem Textilbereich bekannt sind, um Oberflächen einer schmutzabweisende Aktivierung, beziehungsweise Stoffen, Garnen oder Fasern eine entsprechende Ausrüstung zu verleihen. Besonders beim Einsatz des Flächengebildes für die medizinische Kühlung oder für sonstige mikrobiell kritische Anwendungen, beispielsweise auch im Lebensmittelbereich, empfiehlt sich wenigstens eine Oberfläche des Flächengebildes biozid, antiviral oder antimikrobiell zu beschichten beziehungsweise auszurüsten. Eine derartige Beschichtung/Ausrüstung kann dabei zusätzlich auf der Oberfläche des Flächengebildes angeordnet beziehungsweise in dieses integriert werden. Es besteht jedoch auch die Möglichkeit, dass die Bestandteile des Flächengebildes und hierbei insbesondere das Vlies antimikrobiell aktiviert/ausgerüstet werden. In jedem Fall kann die Aktivierung durch die zusätzliche Beaufschlagung des Flächengebildes, des Vlieses oder der Fasern oder Filamente, die das Vlies bilden, mit entsprechenden Aktivierungs-/Ausrüstungsstoffen erfolgen. In Frage kommt hierbei beispielsweise die zusätzliche Anordnung/Beigabe von Nanopartikeln sowie die Einwebung oder Einspinnung von Silberfäden, die dann die anti-mikrobielle Wirkung im Zusammenspiel mit der im Flächengebilde eingebrachten Flüssigkeit entwickeln. Im Zusammenhang mit einer bioziden Beschichtung besteht auch die Möglichkeit, das Flächengebilde algizid, fungizid oder herbizid zu aktivieren, um dieses dann beispielsweise als Abdeckung zu verwenden. Daneben besteht auch die Möglichkeit zur repellenten, UV-blocken-den und/oder antistatischen Beschichtung beziehungsweise Ausrüstung der Oberfläche beziehungsweise der die Oberfläche bildenden Materialien, Stoffen, Folien oder Membrane.

Neben den vorgenannten Aktivierungen beziehungsweise Ausrüstungen der Oberfläche, der die Oberfläche bildenden Materialien und/oder des Flächengebildes selbst kann hier auch eine zusätzliche Ausrüstung beziehungsweise Ausstattung mit Substanzen durchgeführt werden, die insbesondere bei der Verwendung für kosmetische oder medizinische Anwendungen vorteilhaft sind. Derartige Substanzen können beispielsweise hautpflegende Substanzen, wie Produkte auf Basis von Aloe Vera oder sonstigen dermatologisch als vorteilhaft angesehenen Substanzen, sein. Neben diesen Substanzen besteht die Möglichkeit einer zusätzlichen Ausstattung, Ausrüstung oder Beschichtung beziehungsweise Benetzung der Oberfläche des Flächengebildes beziehungsweise der die Umhüllung bildenden Materialien mit die Wundheilung fördernden Substanzen, sodass das Flächengebilde, das erfindungsgemäß auch als Wundabdeckung verwendet werden kann, hier zusätzlich einen die Wundheilung fördernden Effekt zeigt.

Wird die Ausrüstung der Oberfläche beziehungsweise der die Umhüllung und/oder das Flächengebilde bildenden Materialien auf chemischem Wege, durch Beigabe, Auftragen oder Einarbeiten entsprechender, auf die jeweilige Ausrüstung abgestimmter Substanzen durchgeführt, so kann diese Ausrüstung im Verlauf der Nutzung und/oder nach mehreren ReinigungsVorgängen verlorengehen beziehungsweise Wirkungseinbußen aufweisen. Um hier eine Erneuerung beziehungsweise Auffrischung der Ausrüstung vorzunehmen, ist vorgesehen, der für die Aktivierung des Flächengebildes verwendeten Flüssigkeit entsprechende Auffrischungs-beziehungsweise Erneuerungsmittel oder Substanzen beizufügen, um die Wiederherstellung der Ausrüstung auf diesem Wege zu erreichen beziehungsweise auch um eine entsprechende Ausrüstung zu erzeugen.

Die Erfindung umfasst gleichermaßen auch ein Verfahren zur Herstellung eines Flächengebildes, das die oben ausgeführten Ausformungen aufweist. Das erfindungsgemäße Verfahren umfasst dabei die Schritte des zur Verfügungstellens von Fasern oder Filamenten. Dieses zur Verfügungstellen kann in einem Spinnverfahren erfolgen, wobei hier bevorzugt die Fasern oder Filamente durch Trockenspinnen, Nassspinnen, Schmelzspinnen, Matrixspinnen oder Elektrospinnen gebildet sind. Im Spinnprozess wird aus den gewonnenen Fasern oder Filamenten ein Vlies hergestellt, das anschließend oder im Verlauf der Herstellung dazu dient superabsorbierendes Polymer aufzunehmen. Hierzu umfasst das Verfahren den weiteren Schritt des Auf- oder Einbringens von superabsorbierendem Polymer. Dies erfolgt erfindungsgemäß in an oder auf die Fasern oder Filamente und findet vor, während oder nach dem zur Verfügungstellen der Fasern oder Filamente statt. Je nachdem, wie das superabsorbierende Polymer an, in oder auf den Fasern oder Filamenten angeordnet wird, erfolgt in einem weiteren oder parallelen Schritt des Verfahrens das Verbinden der Fasern oder Filamente mit dem superabsorbierenden Polymers. Hierbei kann zum Einen vorgesehen werden, dass die Partikel lose zwischen den zum Vlies gestapelten Fasern verteilt und durch die Fasern beziehungsweise Filamente letztendlich im Vlies fixiert werden. Zum Anderen besteht auch die Möglichkeit, dass, wenn es sich bei dem superabsorbierenden Polymer um ein vernetzbares Copolymer handelt, zunächst der Precursor des Polymers mit den Fasern oder Filamenten vermengt wird und anschließend eine Vernetzung des Precursor zum fertigen, superabsorbierenden Polymer erfolgt. Im Verlauf des Vernetzens, bei dem mit den Precursorn des Polymers Molekülketten gebildet werden, erfolgt dann eine Festlegung des Polymers an den Fasern oder Filamenten und letztendlich die Aktivierung des Flächengebildes. Das erfindungsgemäße Verfahren umfasst als weiteren Schritt die Bildung eines Flächengebildes aus den Fasern oder Filamenten beziehungsweise aus dem Vlies, das aus den Fasern oder Filamenten entstanden ist.

In einer vorteilhaften Weiterbildung des Verfahrens ist vorgesehen, dass das Verbinden der Fasern oder Filamente zum Vlies beziehungsweise zum Flächengebilde durch eine mechanische, chemische oder thermische Verbindung erfolgt. Bei einer mechanischen Verbindung ist das superabsorbierende Polymer allein durch die Adhäsion an den Fasern oder Filamenten beziehungsweise durch die entsprechend dichte Packung der Fasern oder Filamente im Vlies gegeben. Bei einer thermischen Verbindung von Fasern beziehungsweise Filamenten und superabsorbierendem Polymer erfolgt das oberflächliche Anschmelzen der Fasern oder Filamente, sodass hier eine Aufweichung oder Verflüssigung des Fasermantels erfolgt und die superabsorbierenden Polymere letztlich an die Fasern oder Filamente angeschmolzen werden. Nach dem Wiedererhärten der Fasern bleiben die superabsorbierenden Polymere dann mit der Faser verbunden. Im Zuge einer chemischen Verbindung zwischen den Fasern oder Filamenten und dem superabsorbierenden Polymer erfolgt die zusätzliche Beaufschlagung der Fasern oder Filamente beziehungsweise des daraus gebildeten Vlieses oder Flächengebildes mit einem Klebe- oder Vernetzungsmittel. Erfolgt die Beaufschlagung mit einem Klebemittel, so wird eine zusätzliche Substanz in das Flächengebilde eingefügt, die dann die Haftung zwischen den Fasern oder Filamenten beziehungsweise dem Vlies oder Flächengebilde und den superabsorbierenden Polymeren vermittelt. Wird ein Vernetzungs-mittel als chemisches Verbindungsmedium genutzt, so wirkt dieses lediglich auf die superabsorbierenden Polymere und bewirkt deren Vernetzung und im Zuge dessen die Verbindung mit den Fasern oder Filamenten des Vlieses beziehungsweise Flächengebildes. Daneben kann durch chemische Beaufschlagung der Fasern oder Filamente beziehungsweise des Vlieses oder Flächengebildes auch ein oberflächliches Anschmelzen der Fasern oder Filamente erreicht werden, die dann in der Lage sind, eine dauerhafte Verbindung mit den beispielsweise partikelförmig vorliegenden superabsorbierenden Polymeren einzugehen. Diese dauerhafte Verbindung erfolgt nach Trocknen beziehungsweise Neutralisieren der die Fasern aufweichenden beziehungsweise "schmelzenden" chemischen Substanz. Zusätzlich zur Verbindung oder Vernetzung, von superabsorbierendem Polymer und Fasern oder Filamenten beziehungsweise Vlies oder Flächengebilde kann auch in einem Verfahrensschritt die Verbindung der Fasern oder Filamente untereinander erfolgen. Auch hier besteht die Möglichkeit, die entsprechende Verbindung und damit einhergehend die Verfestigung beziehungsweise Verdichtung des Vlieses durch chemische, mechanische oder thermische Einwirkung zu bewirken. Hierbei kann die Verbindung der Fasern oder Filamente durch Vernadeln erfolgen. Gleichermaßen wirksam zur Verbindung der Fasern oder Filamente ist eine Wasserstrahlbehandlung des Rohvlieses, bei dem ähnlich wie beim Vernadeln auch eine Verfestigung und weitergehende Verbindung der Fasern oder Filamente durchgeführt wird. Beim chemischen Verbinden erfolgt eine Beaufschlagung des Rohvlieses mit einem Klebemittel beziehungsweise mit einer Substanz, die den Fasermantel angreift und aufweicht, sodass die Fasern oder Filamente an ihren jeweiligen Kreuzungspunkte verbunden werden und aneinander haften. Das thermische Verbindungsverfahren wurde bereits bei der Bildung der Oberfläche für das fertige Vlies diskutiert. Hierbei erfolgt die Beaufschlagung der Fasern oder Filamente mit einem erwärmten oder erhitzten Gasstrom oder mit Wasserdampf, was zu einer Aufschmelzung oder Aufweichung zumindest des Fasermantels führt. Die solchermaßen aufgeweichten oder angeschmolzenen Fasern werden nach anschließender Druckbeaufschlagung des Rohvlieses miteinander verklebt und im Zuge der Abkühlung des Vlieses erfolgt dann eine Aushärtung der verbundenen Faserstruktur und die dauerhafte Verbindung der Fasern darin. Eine weitere bevorzugte Ausführungsform des Verfahrens sieht vor, dass die Fasern oder Filamente durch Trockenspinnen, Nassspinnen, Schmelzspinnen, Matrixspinnen oder Elektrospinnen gebildet sind und eine Verbindung von Fasern oder Filamenten beziehungsweise einem Ausgangsmaterial für die Fasern oder Filamente und dem superabsorbierenden Polymer vor, während oder nach dem Spinnvorgang erfolgt. Hierbei sind die Fasern oder Filamente aus einem Polymer oder Polymergemisch gebildet, das vor dem Spinnen in verflüssigtem Zustand vorliegt. Die Polymerlösung kann dann mit dem superabsorbierenden Polymer, das beispielsweise in Partikelform oder aber auch als Lösung vorliegt, vermengt und im nachfolgenden Spinnprozess gemeinsam mit dem Faser- oder Filamentpolymer ausgesponnen werden. Das superabsorbierende Polymer ist dann fest in die Struktur der Fasern oder Filamente integriert und aktiviert diese damit superabsorbierend. Bei dieser Vorgehens-weise bedarf es keiner zusätzlichen Beaufschlagung des aus den Fasern oder Filamenten gebildeten Vlieses beziehungsweise des daraus bestehenden Flächengebildes mit einem superabsorbierenden Aktivierungsmittel. Dieses kann selbstverständlich zusätzlich eingefügt werden, um das letztendlich gebildete Flächengebilde weitergehend zu aktivieren. Neben der Zugabe des superabsorbierenden Polymers als Zuschlagsstoff zum Ausgangsmaterial der Fasern oder Filamente besteht selbstverständlich auch die Möglichkeit, dass die Fasern oder Filamente vollständig aus dem superabsorbierenden Polymer gebildet sind. Dieses wird dann ebenfalls in einem der oben genannten Spinnverfahren eingesetzt wobei dabei Fasern oder Filamente gebildet werden. Hierbei besteht natürlich auch die Möglichkeit, dass das superabsorbierende Polymer zusammen mit einer weiteren Trägersubstanz verwendet wird, beispielsweise dann, wenn ein superabsorbierendes Polymer verwendet wird, das nicht im Spinnverfahren verarbeitet werden kann. Neben der gleichzeitigen oder zeitlich nahen Beaufschlagung der Fasern oder Filamente mit dem superabsorbierenden Polymer beziehungsweise einem sonstigen superabsorbierenden Aktivierungsmittel besteht auch die Möglichkeit, dass ein Verfahrensschritt vorgesehen ist, bei dem das superabsorbierende Polymer nach der Bildung des Flächengebildes auf dieses aufgetragen wird. Hierbei besteht auch die bereits vorgenannte Möglichkeit einer chemischen Vernetzung des superabsorbierenden Polymers und der damit verbundenen Festlegung im Flächengebilde. Daneben kann selbstverständlich auch eine mechanische Verbindung des superabsorbierenden Polymers mit dem Flächengebilde erfolgen, beispielsweise, wenn die superabsorbierenden Polymere, die partikelförmig vorliegen, zwischen zwei Vliesen angeordnet werden, die keine superabsorbierende Aktivierung aufweisen. Auch hier kann zunächst eine Precursorsubstanz des superabsorbierenden Polymers auf dem Flächengebilde aufgetragen werden und anschließend, unter Hinzunahme eines chemischen Vernetzungsmittels das letztendliche superabsorbierende Polymer nach der Einbringung in das Flächengebilde durch Vernetzung gebildet wird. Als günstig wird im Verfahren angesehen, wenn das superabsorbierende Polymer in Form von Partikeln vorliegt. Die partikelförmige Beaufschlagung des Flächengebildes erlaubt eine besonders genaue Einstellbarkeit der letztendlich im Flächengebilde vorhandenen beziehungsweise gebundenen Polymermenge.

Ein weiterer Aspekt der Erfindung umfasst eine Umhüllung, die zwei oder mehr Lagen aufweist und dabei wenigstens eine aus einem Flächengebilde, wie oben beschrieben, gebildete textilen Lage umfasst.

Diese Umhüllung kann als Lagen z. B. eine Außen- oder Innenhaut beispielsweise aus einem flächigen Material gebildet, aufweisen, zwischen denen sich mindestens ein Flächengebilde befindet. Die Lagen der Umhüllung sind somit zusätzlich zu den eigentlichen Oberflächen des Flächengebildes vorhanden und umschließen das Flächengebilde. Selbstverständlich besteht auch die Möglichkeit, dass die Außen- oder Innenhaut der Umhüllung beziehungsweise die diese Außen- oder Innenhaut bildenden Lagen flächig auch auf dem Flächengebilde aufliegen und mit den Oberflächen des Flächengebildes in direktem Kontakt stehen, wie dies beispielsweise in einer bevorzugten Weiterbildung der erfindungsgemäßen Umhüllung vorgesehen ist.

Als günstig erweist es sich, auch mit Blick auf die Beladung des Flächengebildes mit einer Flüssigkeit, wenn die Lage beziehungsweise die Lagen aus einem Stoffgewebe oder einem Textil gebildet ist/sind. Darüber hinaus besteht die Möglichkeit, die Lage (n) als Membran(e) oder Folie (n) auszubilden. Hierdurch kann dann eine weitere Funktionalisierung der Umhüllung erreicht werden, wenn beispielsweise über die das Flächengebilde umschließenden oder aufnehmenden Lagen eine weitere Funktionalisierung der Umhüllung erreicht wird. Ist beispielsweise die Lage als Klimamembran ausgeführt, so kann hier auch bei Nichtverwendung des Flächengebildes zur Kühlung dennoch ein Austritt von Wasserdampf erfolgen, ohne dass hier ein Eintritt von Flüssigkeit von der Außenseite her in die Umhüllung stattfindet, da die Membran entsprechende Funktionalitäten übernimmt. Die Ausführung wenigstens einer Lage aus einer beispielsweise flüssigkeitsundurchlässigen Folie ermöglicht die einseitige Abschirmung der Umhüllung gegenüber der zur Aktivierung des Flächengebildes beispielsweise in die Umhüllung eingebrachten Flüssigkeit.

Daneben besteht auch die Möglichkeit die Lage aus einer Kombination oder in einer Sandwichbauweise aus einem oder mehreren Stoffgeweben und einer oder mehreren Membranen oder Folien zu bilden. Auch kann die Lage aus einem Mikrofaserstoffgewebe bestehen, wodurch der Vorteil erzielt wird, dass hier eine schnelle oberflächliche Trocknung der Umhüllung gegeben ist, da hier die aufgebrachte Flüssigkeit zügig zu den in der Umhüllung vorgesehenen Flächengebilden geleitet wird. Den vorgenannten Materialien für die Ausbildung der Lage beziehungsweise der Lagen steht auch noch ein Einsatz von Stoffgeweben oder Textilien zur Verfügung, die Metallfäden, Funktionsfasern wie beispielsweise Kokosfasern, Carbon- oder mineralische Fasern, natürlichen oder künstlichen Ursprungs aufweisen. Die Verwendung von beispielsweise Kokosfasern bietet sich insbesondere an, weil diese Fasern sehr dehnbar, fest und langlebig sind. Neben der hohen Abrasionsbeständigkeit sind diese Fasern auch unempfindlich gegen Pilze und bakteriellen Befall und können eine lange Feuchtebeaufschlagung überdauern ohne sich zu zersetzen. Ein weiterer Vorteil der Verwendung von Kokosfasern liegt in deren isolierenden, schallschluckenden, antistatischen und schwer entzündlichen Eigenschaften begründet, sodass sich aus entsprechenden Fasern gebildete Umhüllungen beziehungsweise Lagen von Umhüllungen für den Einsatz in einem feuchten Umfeld eignen und zudem auf natürliche Art und Weise eine weitere Funktionalisierung der Umhüllung gewährleisten können. Ähnliches gilt für Bambusfasern sowie weitere innovative Funktions-fasern.

Vorteil der Verwendung von Carbonfasern ist deren hohe Dauerhaftigkeit bei geringem Gewicht und deren hohe Steifigkeit. Durch die Verwendung mineralischer Fasern unterschiedlichster Ausprägung können weitere Funktionalisierungen der Umhüllung erzielt werden, die somit die Einsatzmöglichkeiten für die erfindungsgemäße Umhüllung im Zusammenwirken mit dem erfindungsgemäßen Flächengebilde weiter erhöhen.

Werden Metallfäden in der Umhüllung verwendet, so ergibt sich der Vorteil, dass aufgrund der entsprechend anstellbaren Wärmeleitfähigkeit bei der Auswahl des Metalls der Wärmetransport in der Umhüllung eingestellt und somit die Kühlleistung der entsprechenden Umhüllung definiert oder subjektiv verstärkt werden kann.

Die erfindungsgemäße Umhüllung eignet sich auch als Abdeckung für Fahrzeuge, Schiffe, Flugzeuge, Geräte oder Maschinen. Hier kann neben einem kühlenden Effekt beim Betrieb der entsprechenden Fahrzeuge, Geräte oder Maschinen die Infrarotsichtbarkeit der im Betrieb erwärmten Geräte, Maschinen, Fahrzeuge, Flugzeuge oder Schiffe reduziert oder sogar eliminiert werden. Hierdurch wird die Wärmeabstrahlung entsprechender Fahrzeuge, Flugzeuge, Schiffe, Maschinen oder Geräte oder aber auch von Gebäuden unterbunden, sodass diese nicht mehr mit entsprechenden Detektionsgeräten (beispielsweise Wärmebildkameras oder dergleichen) erfasst werden können. Bei der mobilen Anwendung entsprechender Umhüllungen, beispielsweise auf Fahrzeugen an Schiffen, Flugzeugen oder Zügen, werden diese für entsprechende Erfassungsgeräte nahezu unsichtbar beziehungsweise wird deren Form entsprechen verschleiert.

Eine bevorzugte Ausführungsform der Erfindung sieht vor, dass entweder die Oberfläche des Flächengebildes oder aber wenigstens eine Lage der Umhüllung eine Ausstattung aufweist, die den damit bedeckten oder umhüllten Gegenstand gegenüber elektromagnetischer Strahlung abschirmt oder aber diese Strahlung absorbiert. Hierdurch wird zum Einen ein verbesserter Schutz der entsprechend umhüllten Gegenstände, Fahrzeuge, Geräte, Maschinen, aber auch von Körperteilen oder dergleichen gegenüber elektromagnetischer Strahlenbeaufschlagung erreicht, zum Anderen wird der bedeckte Gegenstand, das umhüllte Fahrzeug, die entsprechend abgedeckte Maschine oder ein entsprechend umhülltes Gebäude für ein weiteres Segment des elektro-magnetischen Spektrums unsichtbar und ist somit nicht mit entsprechenden Detektionsgeräten erfassbar oder ortbar.

Die entsprechende Ausstattung der Umhüllung wird beispielsweise auf der Außenseite der Lage angebracht oder in diese eingearbeitet. Die Ausgestaltung der entsprechenden Ausstattung richtet sich dann nach dem jeweiligen Verwendungszweck, das heißt, ob hier eine ledigliche Abschirmung gegenüber elektromagnetischer Strahlung erreicht werden soll, oder aber, ob die elektromagnetische Strahlung vollständig absorbiert werden muss.

In einem weiteren Aspekt der Erfindung weist das in der Umhüllung vorgesehene Flächengebilde eine oder mehrere Membrane auf, die entweder das Flächengebilde umhüllen und somit die Oberfläche definieren und gegebenenfalls entsprechend funktionalisieren oder aber innerhalb des Flächengebildes zusätzlich zu dem dort vorgesehenen Vlies oder aber auch im Vlies selbst angeordnet sind. Hierdurch wird eine Kompartimentierung des Vlieses erreicht und gleichzeitig eine verbesserte Steuerung der Abgabe von Flüssigkeit beziehungsweise des Austrittes von Wasserdampf, aber auch des Eindringens von Flüssigkeit beziehungsweise deren Verteilung im Flächengebilde verbessert und steuerbar.

Die Umhüllung ist vorteilhafterweise als Textilmaterial, Funktionstextil, kühlendes Funktionselement oder Pad, als Bekleidungsstück, Kleidungsaccessoire, als Hosenträger, als Schutzkleidung, als fest oder lösbar in einem Kleidungsstück wie insbesondere einer Jacke, einem Anzug, einer Hose, einer Schürze, einem Overall oder dergleichen angeordnetes beziehungsweise anordenbares Futter, als Plane, als Decke, als Decken- oder Dachkonstruktion, als Medizinprodukt, als OP-Decke, Pulskühler, Halsband, Kühlband, Kühltasche, als Bandage, Fuß- oder Gelenkbandage, Orthese, Gesichts- oder Augenmaske, Strumpf, Strumpfhose, als Kopfbedeckung, Helm, als Schuh oder Stiefel, als Abdeckung, insbesondere für Gebäude, Fahrzeuge, Flugzeuge, Schiffe, Maschinen, Geräte, Oberflächen oder Bodenflächen, als Abdeckplane beziehungsweise Abdeckschutzplane, als Markise, Zeltstoff, Sonnendach oder als Ausrüstungsgegenstand zum Schutz vor hohen Temperaturen, als Tasche, Beutel, als Blut- oder Bluttransportbeutel, als Beutel für den Organtransport, als Flaschen- oder Ampullenkühler zum Schutz von biologischen und/oder humanen Komponenten und Stoffen sowie pharmazeutischen Wirkstoffen und Produkten gegen zu starke Erwärmungen und gegenüber hohen Temperaturen, als Rucksack oder als sonstiges einen Kühleffekt vermittelndes Sport- oder Outdoorprodukt , beispielsweise als Sporttrikot, Sportbekleidung, Unterwäsche, ausgebildet. Daneben besteht auch die Möglichkeit einer Ausbildung als Aufbewahrungs- , Transportschutz oder Tierkühlungsprodukt, insbesondere zur Kühlung von landwirtschaftlichen Nutztieren, wie beispielsweise Kühen oder auch als Kühlweste für Haustiere wie z. B. Hunde oder Katzen sowie Kühldecke und/oder -bandage für Pferde, Hunde oder Katzen sowie alle weiteren als in diesem Zusammenhang geeignet erscheinenden Ausführungsformen. Wird die Umhüllung als kühlendes Funktionselement ausgebildet, so ist hierbei auch umfasst, dass das Funktionselement zum Einstecken in die Tasche oder mehrere Bereiche beziehungsweise Abschnitte oder Aufnahmen eines Bekleidungsstückes geeignet ist, um hier gegebenenfalls nur Bedarfsweise zur Kühlung herangezogen zu werden.

Die Umhüllung, die mit dem textilen Flächengebilde ausgestattet ist, kann besonders vorteilhaft für die medizinische Kühlung eingesetzt werden. Diese Kühlung wird im Normalfall mit Eis-Akkus, eisgekühlten Gelkissen, etc. durchgeführt. Spezialisierte Ärzte und Physiotherapeuten warnen hier vor der Unterkühlung der Bereiche durch Eis aufgrund der Verengung der Gefäße, besonders bei Temperaturen unter 10°C - 15°C. Zudem ist die Kühlung oft nur kurz wirksam, es entsteht Nässe durch Kondenswasser, die Materialien sind hart und schwer. Daher ist es sinnvoll, Alternativen zu den gebräuchlichen Eisanwendungen zu schaffen, um betroffene Menschen und Tiere (wie z.B. Pferde) schneller wieder beschwerdefrei zu bekommen und unerwünschte Ereignisse, die durch Eisanwendung entstehen zu verhindern.

Die Flächengebilde, die in der Umhüllung vorgesehen sind, können dabei fest in der Umhüllung beziehungsweise am oder im eigentlichen Produkt angeordnet oder als kühlendes Funktionselement zum Beispiel vollständig getrennt von der Umhüllung ausgeführt werden. Daneben besteht auch die Möglichkeit, dass kühlende Funktionselemente in einem Bekleidungsstück eingesteckt oder als lösbar oder fest angeordnetes Futter vorgesehen werden. Das Kleidungsstück weist hierfür dann geeignete Taschen oder Befestigungspunkte auf. Das beladene und aktivierte Funktionselement oder das Futter wird hierbei bei Bedarf in diese Tasche eingesteckt beziehungsweise am Kleidungsstück beziehungsweise am Gegenstand angeordnet und kühlt dort in bewährter Weise entweder punktuell oder großflächig den das Produkt tragenden Körper oder Gegenstand. Selbstverständlich besteht auch die Möglichkeit hier körperteilangepasste Bandagen und ganz oder teilweise mit Kühlelementen versehenen Orthesen oder zum Beispiel Handschuhe, Füsslinge oder dergleichen zur Verfügung zu stellen, die mit einem kühlenden Funktionselement, das aus der erfindungsgemäßen Umhüllung gebildet ist, ausgestattet sind. Ein entsprechend aktiviertes Kleidungsstück beziehungsweise eine sonstige Auflage eignet sich dann zum Beispiel auch zur Kühlung bei Herz- /Kreislaufbeschwerden.

Aufgrund von Hitzeeinwirkungen vor allem bei älteren Personen mit Herz-/Kreislaufschwäche oder Haus- und/oder Nutztieren, wie z.B. älteren Hunden, Pferden, Katzen, Kühen etc. mit Herz-/Kreislaufschwäche ist eine schnelle und einfache effiziente Kühlung bei hohen Temperaturen besonders wichtig. Hitzetote Menschen und Tiere in den Sommermonaten und in heißen Ländern sollten nicht zur Regel werden. Gerade in diesem Bereich ist eine Klimaregulierung des Körpers sinnvoll und kann in Form von kühlenden Kleidungsstücken, Kopfbedeckungen und Accessoires durchgeführt werden. Aufgrund des sowieso schon schwächeren Herz-/Kreislaufsystems eignet sich hierfür eine entsprechend ausgeführte Umhüllung gemäß der vorliegenden Erfindung, da diese je nach Ausführungsform ein geringes Gewicht und keine Eistemperaturen, sondern physiologisch kühle Temperaturen, das heißt über 10°C - 15°C, aufweist. Bei der Anwendung am Körper, zum Beispiel als Kleidungstück, bilden sich hierbei keine Wassertropfen oder Gelreste auf der Kleidung, die dann an die Köperoberfläche der Träger gelangen können. Dabei umfasst die Erfindung auch textile Umhüllungen, insbesondere sonstige Textilprodukte wie auch Bekleidungsstücke, aber auch Unterwäsche, die direkt auf der Haut getragen werden und daher eine unmittelbare Kühlwirkung entfaltet, sowie Sportbekleidung und dergleichen. Gerade im Sportbereich wie auch im Arbeitsschutzbereich spart der Körper Energie, da das Kühlprodukt die Thermoregulation des Körpers unterstützt und diesen entlastet. Diese erhöht die Leistungsfähigkeit beim Arbeiten aber auch im Sport. In Studien wurden hierbei 5% - 10% Leistungssteigerung festgestellt.

Im Bereich Arbeitsschutz kommt die Entlastung des Herzkreislauf- und Stoffwechselsystems des Betroffenen hinzu, was zu sinkendem Ausfall-, Unfall- und Krankheitstagen und insgesamt längerer Lebensarbeitszeit führt.

Im medizinischen Bereich ist vorgesehen, dass die erfindungsgemäße Umhüllung zum Beispiel als Fuß- oder Gelenkbandage (z. B. Knie-, Hüft-, Schulter-, Band-, Armgelenk) Verwendung findet oder aber auch als Strumpf beziehungsweise Strumpfhose einsetz-bar ist. Auch im Operationsbereich ist die erfindungsgemäße Umhüllung in verschiedenster Weise einsetzbar. Neben einer OP-Decke ist es auch möglich, entsprechende Gesichtsmasken oder Augenmasken vorzuhalten und zum Beispiel bei entsprechenden Operationen, wie zum Beispiel Schönheitsoperation oder wiederherstellende Operationen im Gesichtsbereich, eine schnelle, aber nicht zu kalte und dauerhaft funktionierende Kühlung zur Verfügung zu stellen.

Des Weiteren umfasst die Erfindung Pulskühler, Halsbänder, Kühlbänder, Kühltaschen oder andere Gegenstände, bei denen eine entsprechend kühlende Wirkung vorteilhaft ist.

Ein weiterer Anwendungsbereich der Erfindung liegt insbesondere auch in dem Einsatz der erfindungsgemäßen Lösung mit einer Umhüllung aus einem textilen Gebilde in einer Verwendung als Sonnendach, Markise beziehungsweise Zeltstoff.

Ebenso ist der erfindungsgemäße Einsatz auch bei Kopfbedeckungen wie Helmen und so weiter günstig, wobei sowohl Arbeitsschutzhelme (Feuerwehrhelme), aber auch Helme, die im Freizeitbereich eingesetzt werden, wie zum Beispiel Motorrad- oder Fahrradhelme, oder auch Fechthelme im Sportbereich, durch den Einsatz der Erfindung eine erhebliche Verbesserung erfahren.

Darüber hinaus kann über die erfindungsgemäße Umhüllung beziehungsweise deren entsprechende Ausführung auch eine Kühlung bei Multipler Sklerose und anderen neurologischen Erkrankungen und Verletzungen durchgeführt werden. Die durch das Uhthoff-Phänomen gekennzeichnete Hitzeempfindlichkeit von ca. 80 % der Betroffenen bei Multipler Sklerose (MS) führt zu stärkeren Symptomen und KrankheitsSchüben sowie Erschöpfungszuständen bei höheren Temperaturen. Auch querschnittsgelähmte Personen, Parkinsonpatienten und andere neurologische Krankheitsbilder haben Hitzeempfindlichkeit und ErschöpfungsSyndrome zur Folge. Diese Erscheinungen können durch Kühlung des Körpers wieder rückgängig gemacht werden, treten durch Körperkühlung erst gar nicht auf. Da diese Betroffenen in der Regel in ihrer Bewegungsfreiheit bereits eingeschränkt sind aber dennoch am öffentlichen Leben teilnehmen, empfiehlt sich hier die Verwendung der erfindungsgemäßen Umhüllung als kühlende Kleidungssysteme, die leicht ausgeführt sind und dennoch eine andauernde Kühlung zur Verfügung stellen.

Bei der Verwendung der Umhüllung als Kleidungsstück, als Decke oder Abdeckung ist auch die dauerhafte Anordnung der superabsorbierenden Polymere im Flächengebilde beziehungsweise die Verbindung der Polymere mit den Fasern oder Filamenten des Vlieses von besonderer Bedeutung. Durch die erfindungsgemäße Ausgestaltung des Flächengebildes beziehungsweise der Umhüllung treten bei der Anwendung keine sich lösenden Partikel oder sonstigen superabsorbierenden Aktivierungsmittel aus dem Kleidungsstück, der Decke oder Abdeckung oder ähnlichen aus, die unter Umständen an der dem Körper zugewandten Oberfläche Feuchtigkeit entstehen lassen würden. Als weiterer Vorteil der erfindungsgemäßen Umhüllung ist hierbei zu sehen, dass diese schnell einsetzbar ist und sich besonders im unbeladenen Zustand leicht transportieren lässt. Die Umhüllung beziehungsweise die darin angeordneten Flächengebilde kann/können dann erst an Ort und Stelle beziehungsweise im Bedarfsfall beladen werden und seine Kühlfunktion entwickeln.

Besonders bei der Verwendung als Medizinprodukt ist die antimikrobielle, antivirale oder biozide Beaufschlagung zumindest der Oberflächen des Flächengebildes beziehungsweise der Umhüllung besonders wichtig, um hier die Gefahr von Kontaminationen oder Ansteckungen weitgehend zu reduzieren. Daneben empfiehlt sich auch eine waschbare beziehungsweise desinfizier- oder sterilisierbare Ausführung der Umhüllung oder zumindest der oberen beziehungsweise äußeren Oberflächen.

In diesem Zusammenhang ist natürlich zu bemerken, dass die antimikrobielle, antiviriale oder biozide Beaufschlagung beziehungsweise Wirkung des Flächengebildes beziehungsweise einzelner Bestandteile des Flächengebildes (Vlies, SAP-Aktivierung, etc.) nicht nur bei Medizinprodukten einsetzbar ist, sondern bei sämtlichen Produkten, die im Rahmen dieser Anmeldung beschrieben sind. Insbesondere umfasst dies auch Anwendungen, die außerhalb des klassischen medizinischen Bereiches, wie z. B. im Sport-, Arbeits- und dergleichen Bereich liegen.

Die antimikrobielle, antivirale oder biozide Beaufschlagung beziehungsweise Aktivierung und daraus auch resultierende sterile oder desinfizierende Wirkung erfolgt dabei zum Beispiel unmittelbar auf der Faser, bevor diese zu einem Vlies verarbeitet wird. Alternativ ist es möglich, diese Beaufschlagung beziehungsweise Aktivierung auf dem flächigen Vlies anzuordnen, und zwar sowohl vor, wie auch nach der Anordnung des Superabsorbers in dem Vlies. Insofern ist es auch möglich, dass das gesamte fertige Flächengebilde mit einer antimikrobiellen, antiviralen oder bioziden Beaufschlagung behandelt wird.

Schlussendlich ist es auch möglich, dass der Oberstoff, beziehungsweise die die Außen- oder Innenhaut der Umhüllung bildenden (Deck-) Lagen zwischen dem/denen das Flächengebilde eingebaut ist, eine antimikrobielle, antivirale oder biozide Eigenschaft beziehungsweise Beaufschlagung aufweist.

Die Umhüllung beziehungsweise ihre entsprechenden Ausführungsformen als Decke, Abdeckung, Kleidungsstück, etc., eignet sich selbstverständlich auch zur Kühlung bei Herzinfarkt oder Schlaganfall. In zahlreichen Studien und Publikationen wurde festgestellt, dass die Überlebensrate von Patienten mit Herzinfarkt oder Schlaganfall v. a. bei Herzstillstand deutlich höher ist und Nachfolgeerscheinungen aufgrund des Infarkts oder Anfalls geringer ausfallen, wenn der Körper des Betroffenen unter die normale Körpertemperatur gekühlt wird. Hier ist auch der Ansatzpunkt stationärer Apparate. Die erfindungsgemäße Umhüllung stellt hier eine Lösung dar, die bereits auf der Fahrt ins Krankenhaus angewendet werden kann bzw. beim Transport im Krankenhaus die Kühlkette nicht abreißen lässt. Vermieden werden hier aufgrund der erfindungsgemäßen Ausführung der Umhüllung Nachteile wie Feuchtigkeit, ungeregelte Eistemperatur, hohes Gewicht, etc.

Die erfindungsgemäße Umhüllung ist auch in anderen medizinischen Bereichen für Kühlzwecke optimal geeignet. Die gute Eignung definiert sich dabei über die zur Verfügung stehende Kühltemperatur, die letztendlich von der Temperatur der beladenen Flüssigkeit abhängt sowie auf die unter Umständen sehr lang wirkende Kühleigenschaft. Es ist dabei gefunden worden, dass ein zu starkes Kühlen, also das Kühlen mit einer zu niedrigen Temperatur, zu unangenehmen Ergebnissen führt. So ist eine entsprechend desinfizierte beziehungsweise sterile Umhüllung auch bei stumpfen Verletzungen, bei Operationen, aber auch bei Schwellung, Blutergüssen oder bei durch Spritzen verursachte Verletzungen einsetzbar. Der Anwendungsbereich umfasst dabei sowohl die Chirurgie, wie auch Orthopädie oder auch Schönheitschirurgie. Gerade im letztgenannten chirurgischen Bereich ist eine schonende, aber lang anhaltende Kühlwirkung von Vorteil.

Ein weiteres Einsatzgebiet für die erfindungsgemäße Umhüllung stellt die Kühlung im Sport, in der Freizeit und in der Arbeit dar. Aufgrund des hohen Energiebedarfs bei der Wärmeregulation des Körpers sind Mensch und Tier und hierbei insbesondere Arbeiter und Sportler bereits ab 18°C deutlich in ihrer Leistung reduziert, was durch noch höhere Außentemperaturen zusätzlich verstärkt wird. Durch den Einsatz von Kühlung, vor und nach dem Sport, im Training und in Ruhepausen kann Energie eingespart werden, was zu einer deutlichen Leistungssteigerung führt. Kühlung an Hitzearbeitsplätzen erhöht Konzentration und Leistung und verhindert gesundheitliche Schäden. Erschöpfungssyndrome können verhindert werden. Auch hier ist ein einfaches, sauberes, trockenes und strapazierfähiges Kühlprodukt in Form der erfindungsgemäßen Umhüllung sinnvoll, das sowohl mit als auch ohne Vorkühlung über lange Zeit funktioniert. Positiv ist hierbei der Effekt, dass die zusätzliche Verdunstungskälte bei Vorkühlung einen Synergieeffekt ergibt, der in einer reduzierten Temperatur von bis zu -2°C an zusätzlichem Kühleffekt reicht. Außerdem wird die Kühldauer deutlich verlängert, was den Anwendungsbereich deutlich vergrößert, vor allem bei hoher Luftfeuchtigkeit.

Ein weiteres Einsatzgebiet für die erfindungsgemäße Umhüllung beziehungsweise eine ihrer zahlreichen Ausführungsformen stellt die Anwendung zur Kühlung von Gegenständen und der Umwelt dar. An heißen Arbeitsplätzen (z.B. in der Nähe von Maschinen, Geräten, Öfen, Schmelzen, etc.), in der Nähe von Feuer (z. B. beim Brandschutz in Häusern oder bei Waldbränden) oder bei Hitze in heißen Ländern oder im Sommer (z.B. unter Markisen, in Zelten oder unter Dächern) wie auch zum Erreichen beziehungsweise Halten einer bestimmten Temperatur von Produkten (z.B. von Nahrungsmitteln, Blutkonserven, Medikamenten, Ampullen, Flaschen, etc.) und beim Transport und in Logistikketten ist eine Kühlung wichtig und teilweise unabdingbar. Hier darf zudem kein Material oder Wasser austreten. Da ein Anschluss an sonstige Kühlkreisläufe nicht immer möglich ist und zudem teilweise zu niedrige Temperaturen dabei auftreten und der entsprechende Anschluss auch mit hohen Kosten verbunden ist, ist ein energieunabhängiges Kühlsystem, wie es die erfindungsgemäße Umhüllung zur Verfügung stellt, von besonderem Vorteil.

Selbstverständlich gibt es noch weitere Kühlanwendungen, wie zum Beispiel im Zusammenhang mit Frühgeburten, bei Chemo- und Strahlentherapie, bei ZNS- und StoffWechselerkrankungen, bei Hyperthemie, Brandverletzungen, bei Hitzearbeitern und in anderen Situationen, die hier nicht ausführlich diskutiert werden können, jedoch gleichermaßen umfasst sind. Die erfindungsgemäße Umhüllung in allen ihren Ausführungsformen eignet sich jedoch in allen Fällen für die Kühlung, die darauf abzielt, den Einfluss der Außentemperatur auf einen Körper oder Gegenstand soweit zu reduzieren, dass hier keine dauerhaften Schäden zu befürchten sind. Die erfindungsgemäße Umhüllung beziehungsweise das damit zur Verfügung stellbare Kühlsystem bietet hierbei zahlreiche Vorteile, da es sich um ein einfaches, schnell einsetzbares und vor allem oberflächlich trockenes sowie geräteunabhängiges Kühlsystem handelt, das zudem eine lang anhaltende Kühlleistung aufweist und gegebenenfalls mit weiteren Kühlsystemen kombinierbar ist.

Um eine möglichst vielseitige Anwendung der erfindungsgemäßen Umhüllung gewährleisten zu können, wird es als vorteilhaft angesehen, wenn die Umhüllung waschmaschinenwaschbar ist. Auch eine chemische Reinigung, Sterilisierung oder Desinfizierung der Umhüllung ist günstigerweise durchführbar. Neben dem oben bereits ausgeführten Kühleffekt stellt die Umhüllung günstigerweise auch einen Klimaeffekt zur Verfügung. Unter einem Klimaeffekt ist zu verstehen, dass die erfindungsgemäße Umhüllung nicht unkontrolliert kühlt, sondern vielmehr dazu geeignet ist, den Temperaturzustand eines mit der Umhüllung bedeckten Menschen oder Gegenstandes konstant zu halten. Zusätzlich zu diesem klimatisierenden Effekt weist die Umhüllung einen zusätzlichen positiven Effekt auf das Klima auf, da eine Kühlung bei der Verwendung der erfindungsgemäßen Umhüllung CO₂-neutral und somit klimafreundlich erfolgen kann. Als günstig erweist es sich, wenn die Umhüllung erst nach oder durch Beladung mit Flüssigkeit, insbesondere Wasser oder einem Gemische mit Wasseranteil gekühlt wird. Eine weitere Verbesserung beziehungsweise Verlängerung der Kühlleistung wird erreicht, wenn die Umhüllung beziehungsweise das Flächengebilde mit vorgekühlter Flüssigkeit und hierbei beispielsweise mit vorgekühlter Salzlösung beladen wird. Dies bedeutet, dass die Umhüllung im entladenen beziehungsweise nicht mit Feuchtigkeit oder Flüssigkeit beladenen Zustand ein nur geringes Gewicht aufweist. Je nach Ausführung des die Umhüllung bildenden Flächengebildes kann hier zusätzlich zunächst auch ein gewisser Isolations- und damit Wärmeeffekt durch die Umhüllung gegeben sein. Die erfindungsgemäße Umhüllung weist somit einen Doppelnutzen auf. Dieser erweist sich insbesondere bei medizinischen Anwendungen als vorteilhaft, da mit der erfindungsgemäßen Umhüllung sowohl eine Isolierdecke wie auch eine Kühlumhüllung bedarfsweise zur Verfügung gestellt werden kann. Durch geeignete Beschichtung der Oberfläche der Umhüllung, beispielsweise mit einer Reflexionsschicht, kann die Isolierleistung verbessert werden. Gleichzeitig verlängert bei Kühlanwendung die Reflexionsschicht die Kühldauer, da die Aufheizung durch Wärmestrahlung verringert wird. Erst nach der Beladung mit Feuchtigkeit beginnt die Umhüllung, im Zuge der Verdunstung der beladenen Flüssigkeit und/oder aufgrund der Temperatur der Flüssigkeit einen Kühleffekt zu entwickeln und damit den bedeckten Körper oder Gegenstand abzukühlen beziehungsweise dessen Temperatur bis zu einem gewissen Niveau konstant zu halten. Eine weitere Ausführungsform der erfindungsgemäßen Umhüllung sieht vor, dass diese nicht allein dazu verwendet wird, nach Beladung mit Feuchtigkeit zu kühlen, sondern dass es vielmehr vorgesehen ist, die Umhüllung zusätzlich zur verdunstungsbedingten Kühlung im beladenen Zustand vorzukühlen. Hierbei kann der bereits vorstehend diskutierte zusätzliche Kühleffekt zusätzlich zur Verdunstungkühlung des vorgekühlt in die Umhüllung eingefüllten Mediums oder durch Vorkühlen des Produktes nach Beladen mit Flüssigkeit erreicht werden.

Daneben ist erfindungsgemäß vorgesehen, dass die Umhüllung mit einer Flüssigkeit, die aus einem Gemisch aus Alkohol und Wasser gebildet ist, beladen wird. Diese Beladung mit einem Gemisch aus Alkohol und Wasser bewirkt, dass bereits unmittelbar nach dem Beladen ein Verdunsten des Alkohols und die damit verbundene Herabsetzung der Temperatur der Umhüllung aufgrund der im Verlauf der Verdunstung entzogenen Wärme erfolgt. Nach dem Verdunsten des Alkohols beziehungsweise zeitgleich damit, setzt des Weiteren eine Verdunstung des im Gemisch enthaltenen Wassers ein, wodurch ein langanhaltender Kühleffekt in der Umhüllung erzeugt wird. Für eine optimale Kühlung wird es als besonders günstig angesehen, wenn das Gemisch zwischen einem 1 und 70 Vol.-%, bevorzugt zwischen 5 und 50 Vol.-%, insbesondere jedoch zwischen 10 und 30 Vol.% Alkohol und zwischen 30 und 99 % Wasser enthält. Je nach geforderter Geschwindigkeit der Abkühlung der Umhüllung, kann der Alkoholanteil im Gemisch entsprechend der oben angegebenen Werte beziehungsweise im Bereich dieser Werte variiert werden. Es besteht so die Möglichkeit, eine Kühlung angepasst auf die jeweiligen Umweltbedingungen beziehungsweise Außentemperaturen, die beim Einsatz der Umhüllung herrschen, durchzuführen beziehungsweise die entsprechende Kühlleistung an diese Bedingungen oder die Vorgaben durch den Benutzer anzupassen.

Der in dem Gemisch verwendete Alkohol ist günstigerweise ausgewählt aus der Gruppe, bestehend aus Ethanol, Isopropanol oder Mischungen aus diesen Alkoholen. Als gesundheitlich unkritisch und somit zur Verwendung beim Einsatz an Menschen oder Tieren besonders geeignet erweist sich die Verwendung von Ethanol und Isopropanol .

Um die Kühlleistung weiter zu verbessern oder aber um entsprechende Zusatzwirkungen des verwendeten Gemisches zu erreichen, wird es als vorteilhaft angesehen, wenn das Alkohol-Wasser-Gemisch die Beimischung eines Zusatzstoffes aufweist. Als Zusatzstoffe eignen sich hierbei insbesondere ätherische Öle oder Duftstoffe (natürlich oder künstlich), die zum Einen eine weitergehende Kühlung, beispielsweise bei Auftrag auf Hautpartien bewirken, oder aber die beim Verdunsten des in die Umhüllung beziehungsweise ein in der Umhüllung angeordnetes Flächengebilde eingefüllten Gemisches eine insbesondere als angenehm empfundene Duftwirkung entwickeln oder als Repellens fungieren. Bei den verwendbaren ätherischen Ölen handelt es sich bevorzugt um Menthol oder Campher, da hierbei die entsprechenden Kühlwirkungen beim Auftragen dieser Substanzen auf menschliche Haut bekannt sind und als zusätzlicher Kühleffekt ausgenutzt werden können. Neben der Duft- oder KühlWirkung können auch solche Zusatzstoffe beigefügt werden, die eine pulsive Wirkung beispielsweise auf Insekten oder Zecken aufweisen und den Verwender der Umhüllung somit zusätzlich vor Insektenstichen oder Zeckenbissen schützen. Neben der Verwendung von Menthol und Campher kommen somit weitere natürliche ätherische Öle beispielsweise aus Gewürznelken oder aromatischen Süßgräsern in Frage. Auch eingesetzt werden können künstliche Repellentien wie zum Beispiel Icaridin oder Aromastoffe.

Um zu verhindern, dass das Alkohol-Wasser-Gemisch missbraucht wird, empfiehlt es sich, dass ein Vergällungsmittel als Zusatzstoff beigemischt wird. Unabhängig vom verwendeten Zusatzstoff ist erfindungsgemäß vorgesehen, dass dessen Anteil im Bereich von 0,1 bis 5 Vol.% am Gesamtgemisch liegt.

Insbesondere mit Blick auf die Verwendung der Umhüllung im medizinischen Bereich, spielt Sterilität eine besonders große Rolle. Es wird daher als vorteilhaft und günstig angesehen, wenn das im Gemisch verwendete Wasser ausgewählt ist aus der Gruppe bestehend aus destilliertem Wasser, demineralisiertem Wasser oder Reinstwasser. Je nach Hygiene- und Sterilitätsanforderungen erfolgt dann durch den Fachmann eine entsprechende Auswahl der jeweils dem Gemisch beizugebenden Wasserkomponente. Werden hier keine erhöhten Anforderungen gestellt, kann auch Leitungswasser oder mineralisiertes Wasser zum Einsatz kommen. Im Bereich der verwendeten Alkohole besteht selbstverständlich auch die Möglichkeit entsprechende Grade der Reinheit (technisch, zur Synthese, rein, reinst, zur Analyse) zu verwenden, die sich wiederum ebenfalls von der beabsichtigten Verwendungsweise und dem vorgesehenen Verwendungsumfeld ergeben.

In einer als empfehlenswert angesehenen Ausführungsform der erfindungsgemäßen Umhüllung ist vorgesehen, dass das Gemisch aus Alkohol und Wasser beziehungsweise das Gemisch aus Alkohol und Zusatzstoff als Konzentrat vorgehalten und erst vor der Beladung der Umhüllung durch Zugabe von weiterem Wasser auf eine Endkonzentration verdünnt wird. So kann beispielsweise das Konzentrat in Form von Ampullen oder besonders platzsparenden Flaschen der Umhüllung zur späteren bedarfsweisen Verwendung beigegeben werden. Es wird dem Verwender somit die Möglichkeit eröffnet, dass die Umhüllung nur mit Wasser oder alternativ mit einem Gemisch aus Alkohol und Wasser und/oder einem Zusatzstoff zu beladen, wobei die letztendliche Beladung beziehungsweise das entsprechende Beladungsmittel abhängig ist vom jeweiligen Einsatzzweck beziehungsweise den Anforderungen an die Kühlleistung der Umhüllung.

Neben der Beigabe von ätherischen Ölen, Duftstoffen (natürlich oder künstlich) oder Vergällungsmitteln als Zusatzstoff, kann das Gemisch alternativ oder zusätzlich eine Beimischung von Bioziden, Fungiziden, Viruziden, antimikrobiellen oder antibiotischen Substanzen aufweisen. Auch hierbei ist erfindungsgemäß vorgesehen, dass die Beimischung bei einem Anteil von 0,1 bis 5 Vol.% liegt. Durch die vorgenannten Substanzen wird zum Einen eine konservierende Wirkung erzielt und zudem verhindert, dass es zu einer Besiedelung der Umhüllung mit Organismen, beispielsweise Pilzen oder Bakterien, kommt. Somit eignet sich die Umhüllung für Zwecke mit erhöhten Anforderungen an die Produkthygiene und es kann darüber hinaus erreicht werden, dass während der Verdunstung und dem Kühleinsatz der Umhüllung gleichzeitig eine Abgabe der jeweiligen Substanzen erfolgt, was beispielsweise mit einem kurativen Effekt verbunden sein kann. So empfiehlt sich beispielsweise die Beigabe einer antibiotischen Substanz bei der Verwendung der Umhüllung als Wundabdeckung. Die antimikrobielle Aktivierung beziehungsweise Ausrüstung der Umhüllung macht vor allem dann Sinn, wenn die Umhüllung zur Kühlung beispielsweise im Lebensmittelbereich verwendet wird. Verwendung finden können hierbei beispielsweise auch gesundheitlich wenig bedenkliche Silberlösungen, insbesondere auch Mikro- oder Nanosilber. Auch möglich ist die Beigabe von hautpflegenden Produkten beispielsweise auf der Basis von Aloe Vera oder von sonstigen dermatologisch als empfehlenswert angesehenen Substanzen. Auch Mittel zur Unterstützung der Wundheilung eignen sich als Zusatzstoff.

Die Beladung der Umhüllung mit einem Gemisch bleibt nicht auf ein Gemisch aus Alkohol und Wasser beziehungsweise den in diesem Zusammenhang beschriebenen sonstigen Ergänzungsmittel beschränkt. Vielmehr kann hier auch ein Gemisch aus Wasser und einem die Ausrüstung der Umhüllung beziehungsweise des Flächengebildes wieder herstellende, auffrischende oder generell erzeugenden Substanz vorgesehen werden, da sich, nach mehreren Wasch- beziehungsweise Reinigungsvorgängen eine entsprechende Ausrüstung der Oberfläche beziehungsweise der Umhüllung abschwächt. Die Verwendung eines Gemisches aus Wasser und dem die Ausrüstung erneuernden beziehungsweise auffrischenden Mittel beziehungsweise der entsprechenden Substanz, kann somit dazu verwendet werden, während des Beladens gleichzeitig die Ausrüstung zu erneuern oder aufzufrischen. Sofern chemisch kompatibel, kann das entsprechende Auffrischungsmittel für die Ausrüstung auch der Mischung aus Alkohol und Wasser beigegeben werden. So besteht beispielsweise die Möglichkeit, dass eine Vielzahl von verschieden zusammengestellten Gemischen angeboten wird und der Verwender für den jeweiligen Einsatzbereich ausgewählte Gemische verwendet, um eine Aktivierung der Kühleigen-schaft und gleichzeitig eine entsprechende Aktivierung oder Spezialisierung der Umhüllung auf einen bestimmten Anwendungsbereich durchzuführen. Denkbar ist beispielsweise, dass die für die Aktivierung verwendete Flüssigkeit einen Anteil an Wasser aufweist, über den die Kühlleistung sichergestellt beziehungsweise aktiviert wird und zusätzlich einen Teil Alkohol aufweist, der eine beschleunigte Kühlung mit dem Flächengebilde beziehungsweise der Umhüllung bewirkt und zusätzlich zu der Mischung noch weitere Substanzen, beispielsweise biozide Substanzen vorgesehen sind. Zusätzlich oder alternativ hierzu kann auch ein die Ausrüstung der Umhüllung beziehungsweise des Flächengebildes erneuernde oder auffrischende Substanz beigegeben werden, die dann zusammen mit der Aktivierungsflüssigkeit für die superabsorbierenden Anteile auf das Material beziehungsweise Gewebe aufgetragen beziehungsweise in dieses eingebracht wird und hier die Ausrüstung entsprechend erneuert oder auffrischt. Eine derartige Flüssigkeit zur Erneuerung oder Auffrischung der Ausrüstung kann beispielsweise die Funkenflugbeständigkeit, die mit Resistenz gegenüber Metall- beziehungsweise Glutspritzern, beziehungsweise die Feuerbeständigkeit oder auch die Hydrophilisierung des Gewebes, der Oberfläche beziehungsweise des Flächengebildes erneuern, auffrischen oder wiederherstellen.

Eine vorteilhafte Weiterbildung der Erfindung sieht vor, dass die Umhüllung zur oder nach der jeweiligen Vorkühlung in fest installierten oder tragbaren Kühlbehältern aufbewahrt oder transportiert wird. In dieser Ausführungsform der Erfindung eignet sich diese besonders zur medizinischen Kühlung in den vorher bereits genannten Fällen und in der Notfallmedizin. Somit kann z. B. eine mit Wasser bereits beladene und sterilisierte Kühldecke in Plastikfolie vakuumverschweißt ready-to-use im Notarztwagen oder Rettungsfahrzeug vorgehalten werden.

Daneben besteht auch die Möglichkeit, beispielsweise bei Bränden, die Umhüllung als Abdeckplane für Gegenstände oder ganze Gebäude auszuführen und in entsprechenden Kästen oder sonstigen Behältern an den Brandort zu transportieren. Die Umhüllung bewirkt dann einen Doppeleffekt, so wird zunächst durch die vorgekühlte Abdeckung eine Temperaturreduzierung in der Nähe des Brandherdes beziehungsweise am abgedeckten Gegenstand erreicht. Ist dieser Effekt durch die fortgesetzte Hitzebeaufschlagung aufgebraucht, erfolgt eine Kühlung durch die Verdunstung der in der Umhüllung vorhandenen Flüssigkeit. Die Verwendungsdauer der Umhüllung wird dadurch signifikant erhöht und der Kühleffekt zusätzlich verbessert. Im Falle von großen Planen kann der Brand eingedämmt werden und greift nicht auf weitere Flächen, Gegenstände, Gebäude über. Weiter verbessert wird die Umhüllung durch Beaufschlagung oder Imprägnierung mit einer flammhemmenden Substanz. Hierdurch wird die Brandwiderstands-dauer erhöht und die Zeit für Löschungsmaßnahmen verlängert.

Eine weitere vorteilhafte Ausführungsform der Erfindung sieht vor, dass die Umhüllung in Kombination mit Kaltluft oder in einer Kühlkammer verwendet wird. Durch diese Maßnahme beziehungsweise in dieser Verwendung kann die Kühlleistung der Umhüllung weiter verbessert und der Kühleffekt weiter verlängert werden. Auch eignet sich die Umhüllung dann besonders bei der Behandlung von entzündlichen Erkrankungen im Medizinbereich. Zusätzlich sieht diese Ausführungsform der Erfindung vor, dass die Umhüllung im Zusammenhang mit kühlungsbedürftigen Lebensmitteln oder sonstigen Stoffen, wie beispielsweise Medikamenten oder temperatursensiblen Chemikalien verwendet wird.

Die erfindungsgemäße Umhüllung weist, wie oben bereits ausgeführt, zwei oder mehr Lagen auf. Als vorteilhaft erweist es sich in diesem Zusammenhang, wenn wenigstens ein Teil der Umhüllung beziehungsweise wenigstens eine Lage aus feuer- oder flammenfestem, flüssiges Metall abweisenden beziehungsweise gegen die Beaufschlagung mit flüssigem Metall resistenten, wasserdichtem, selbstreinigendem, schmutzabweisendem, antimikrobiellem und/oder biozidem Material gebildet ist oder aus einem Material besteht, das eine gummiartige Konsistenz auf-weist. Darüber hinaus besteht auch die Möglichkeit, dass wenigstens eine Lage oder ein Teil der Umhüllung eine feuer- oder flammenfeste, gegen flüssiges Metall resistente oder dieses abweisende, wasserdichte, selbstreinigende, schmutzabweisende, biozide, antivirale, antimikrobielle, hydrophilisierende oder gummiartige Beschichtung, Aktivierung oder Ausrüstung aufweist. Die Beschichtung kann nach Fertigstellung der Umhüllung beziehungsweise nach dem Anordnen der entsprechenden Lagen am Flächengebilde gebildet werden. Darüber hinaus besteht auch die Möglichkeit, dass die Lage beziehungsweise das Lagenmaterial oder ein das Flächengebilde umgebendes Material mit der entsprechenden Aktivierung, Beschichtung oder Ausrüstung versehen ist und dieses Material erst nachträglich mit dem superabsorbierend aktivierten Bestandteil beziehungsweise dem Flächengebilde der Umhüllung verbunden wird. Die entsprechende Aktivierung, Beschichtung oder Ausrüstung kann durch Streichen, Sprühen, Aufspritzen, oder Tauchen aufgetragen beziehungsweise an der Umhüllung und/oder der wenigstens einen Lage angebracht werden. Handelt es sich bei der Beschichtung um eine flamm-oder feuerfeste, biozide, antimikrobielle oder antivirale Aktivierung, so können entsprechende Stoffe oder besonders beaufschlagte Fasern bereits in das Flächengebilde, das Vlies beziehungsweise in die übrigen, die Lage oder die Umhüllung bildenden Materialien bei deren Herstellung oder nachträglich eingebracht werden. Eine Aktivierung erfolgt dann gegebenenfalls auch erst im Zusammenhang mit der Beaufschlagung mit Feuchtigkeit. Zur selbstreinigenden oder schmutzabweisenden Aktivierung/Ausrüstung empfiehlt sich eine unmittelbare Behandlung der Oberfläche der Umhüllung beziehungsweise der wenigstens einen Lage, da regelmäßig nur diese mit Schmutz oder sonstigen Verunreinigungen in Kontakt treten. Die entsprechend aktivierten oder beschichteten Lagen oder Teile der Umhüllung schützen dann aufgrund ihrer Aktivierung den Rest der Umhüllung und hierbei insbesondere das in der Umhüllung vorgesehene superabsorbierend aktivierte Flächengebilde vor Schmutz und Verunreinigungen.

Neben den vorgenannten Lage, kann durch eine entsprechende Behandlung der Umhüllung, eine Hydrophilisierung bestimmter Gewebe, die normalerweise eine Wasser- oder Flüssigkeitsaufnahme nicht zulassen, durchgeführt werden. Nach einer entsprechenden hydrophilisierenden Ausrüstung entsprechender Materialien oder Gewebe kann dann auch über diese Gewebe eine Aufnahme beziehungsweise ein Eintritt von Flüssigkeit in die Umhüllung erfolgen, sodass hier dann eine Aktivierung des superabsorbierend aktivierten Flächengebildes stattfinden kann, während die mechanischen oder sonstigen speziellen chemischen Eigenschaften des ursprünglich nicht hydrophilen Gewebes oder Materials und eine Eignung für die entsprechenden Verwendungszwecke, beispielsweise dem Schutz gegen Feuer, Funkenflug und starke Hitzebeaufschlagung erhalten bleiben.

Eine vorteilhafte Weiterbildung der Umhüllung sieht vor, dass in einer oder mehreren der Lagen Kanäle zur Belüftung oder zum Verteilen von Flüssigkeit vorgesehen sind. Eine entsprechende Ausführung ist besonders dann empfehlenswert, wenn die Lage selbst beziehungsweise die Umhüllung eine wasserdichte oder gummiartige Beschichtung aufweist beziehungsweise aus einem wasserdichten oder gummiartigen Material gebildet ist, das den Eintritt von Wasser beziehungsweise Flüssigkeit generell unmöglich macht. Hierbei können die Kanäle in den Lagen nach Art eines Kapilareffektes die Flüssigkeit in das Flächengebilde hineinleiten und dort die gleichmäßige Verteilung der Flüssigkeit sicherstellen, sodass ein gleichmäßiger Kühleffekt gegeben ist. Gleichermaßen dienen die Kanäle nach der Beladung des Flächengebildes beziehungsweise nach Aktivierung der Umhüllung durch Beladen mit Flüssigkeit dazu, den bei Verdunstung entstehenden Wasserdampf aus der Umhüllung beziehungsweise dem Flächengebilde abzutransportieren, um hierbei den Kühleffekt aufrechtzuerhalten. Als empfehlenswert wird angesehen, wenn die Kanäle manuell verschließbar ausgebildet sind, um hierdurch eine gewisse Steuerungswirkung auf den in das Flächengebilde eingeleiteten Flüssigkeitsstrom ausüben zu können. Daneben besteht selbstverständlich auch die Möglichkeit, dass die Kanäle automatisch schließend ausgeführt werden, um so eine bedarfsweise selbstregulierende Beaufschlagung beziehungsweise Einleitung von Flüssigkeit in das Flächengebilde erfolgt. Der automatische Verschluss kann beispielsweise dann erfolgen, wenn ein bestimmter Temperatur- oder Feuchtigkeitsbereich unter-oder überschritten wird.

Als günstig wird in diesem Zusammenhang auch angesehen, wenn die Aufnahme oder Abgabe von Flüssigkeit über Mikropumpen erfolgt beziehungsweise wenn eine osmotische Aufnahme beziehungsweise Abgabe von Flüssigkeit erfolgt. Die Verwendung von Mikropumpen gewährleistet eine gleichmäßige Verteilung der Flüssigkeit beziehungsweise deren gleichmäßige Abgabe an die Umhüllung beziehungsweise das Flächengebilde. Auch kann, was als empfehlenswert angesehen wird, ein Flüssigkeitsreservoir in der Umhüllung vorgesehen werden, das insbesondere als Wanne, Kanister, oder Flasche oder dergleichen ausgebildet ist und zur Aufnahme oder Abgabe von Flüssigkeit dient. Dieses Flüssigkeitsreservoir kann mit den Mikropumpen in Verbindung stehen, sodass diese dann die Flüssigkeit aus dem Flüssigkeitsreservoir entnehmen und in der Umhüllung beziehungsweise dem darin angeordneten Flächengebilde verteilen. Gleichermaßen kann auch ein Entzug von Flüssigkeit aus der Umhüllung beziehungsweise dem Flächengebilde über die Mikropumpen erfolgen. Zusätzlich oder alternativ hierzu kann auch ein Schlauchsystem vorgesehen werden, das dem Flächengebilde oder der Umhüllung zugeordnet ist und mit dem Flüssigkeitsreservoir in Verbindung steht. Über das Schlauchsystem kann im Zusammenwirken mit den Mikropumpen oder aber aufgrund eines sich ausbildenden osmotischen Gefälles die Flüssigkeit in der Umhüllung beziehungsweise in Flächengebilde verteilt werden. Auch hier stellt sich dann eine gleichmäßige Flüssigkeitsverteilung ein, die einen gleichmäßigen Kühleffekt bewirkt.

Durch osmotische Wirkung oder auch Mikropumpen ist aber auch eine schnelle Entleerung der Umhüllung, zum Beispiel wenn diese als Abdeckung von Maschinen oder Häusern verwendet wird, möglich.

Somit dient die Mikropumpe nicht nur zur Aufnahme sondern auch zur Abgabe von Flüssigkeit, wie auch ebenfalls der osmotische Effekt zur Aufnahme wie auch zur Abgabe der Flüssigkeit einsetzbar ist.

Das Flächengebilde ist vorteilhafterweise an oder in der Umhüllung fest angeordnet. Eine Verbindung zwischen Flächengebilde und Umhüllung kann dabei durch Verbindungsmittel wie Verkleben, Verschweißen oder Vernähen erfolgen. Neben der festen Anordnung des Flächengebildes an oder in der Umhüllung besteht auch die Möglichkeit, dass das Flächengebilde lösbar in der Umhüllung angeordnet ist. Hierbei empfiehlt es sich dann, das Flächengebilde durch Einstecken in Taschen, die in der Umhüllung vorgesehen sind, mit dieser zu verbinden. Neben der Anordnung entsprechender Taschen oder sonstiger Aufnahmen, besteht auch die Möglichkeit, ein dahingehend ausgebildetes Flächengebilde über Knöpfe, durch Verbindung mit einem Reißverschluss, einem Klettverschluss oder sonstigen lösbaren Verbindungsmittel mit der Umhüllung zu verbinden.

Zu den sonstigen Verbindungsmitteln zählen insbesondere Druckknöpfe, Schnallen, Steckverschlüsse, Clipsverschlüsse, Verbindungsmittel mit Gurten oder Gürteln, Klemmverschlüsse und ähnliches. Diese lösbare Verbindung zwischen Flächengebilde und Umhüllung bietet den Vorteil, dass eine flexible Anordnung der Flächengebilde und damit die flexible Ausgestaltung der Kühlung und deren Durchführung erreicht werden kann. So besteht beispielsweise die Möglichkeit durch das Einstecken oder Anordnen des Flächengebildes in Sportbekleidungen nur bestimmte Regionen des Sportlerkörpers zu kühlen. Gleiches gilt selbstverständlich auch im Zusammenhang mit der Kühlung von Gegenständen oder von Tieren beziehungsweise bei der Kühlung von Patienten. Die bedarfsweise Anordnung von lösbaren Kühlelementen beziehungsweise einen Kühleffekt bewirkenden Flächengebilden wird somit möglich.

Die Erfindung stellt weiterhin ein Kühlsystem zur Verfügung, das sich insbesondere für den Einsatz im Rahmen einer medizinisch notwendigen, kontrollierten und überwachbaren Kühlung von Menschen und Tieren eignet und eine Umhüllung, wie vorher beschrieben, umfasst. Die Überwachung kann nicht nur im medizinischen Bereich, sondern auch bei Sport und im Arbeitsschutz sinnvoll sein, um z. B. die Leistungsfähigkeit der entsprechenden Personen zu überwachen. Genauso gilt dies für empfindliche Maschinen, Gegenstände, Fahrzeuge, Flugzeuge, Schiffe oder andere Objekte deren Temperaturüberwachung sinnvoll und notwendig ist. Die medizinische Kühlung wird hierbei durch physikalische Verdunstung des in der Umhüllung aufgenommenen Wassers beziehungsweise einer sonstigen Flüssigkeit durchgeführt. Das Kühlsystem hat den Vorteil, dass es im unbeladenen Zustand, das heißt, im flüssigkeitsfreien Zustand aus einem besonders leichtem Material besteht, das leicht transportiert, aufbewahrt und angewendet werden kann und im unbeladenen Zustand zudem keine Belastung, insbesondere für Patienten, darstellt. Da die Umhüllung aus einem besonders flexiblen Material, nämlich aus einem Textilmaterial gebildet ist, erlaubt den flexiblen Einsatz des Kühlsystems für eine Fülle von Anwendungen und gewährleistet gleichzeitig die Erzielung einer optimalen Kühlwirkung durch den besonders körperbeziehungsweise gegenstandsnah möglichen Einsatz. Das flexible Material kann dabei an die zu kühlenden Körperregionen von Mensch und Tier optimal angepasst werden und erst nach der entsprechenden Anpassung mit einer definierten Menge an Flüssigkeit befüllt, begossen oder besprüht werden. Die Flüssigkeitsaufnahme erfolgt dabei besonders schnell. Aufgrund der besonderen Eigenschaften des in der Umhüllung verwendeten und damit auch im Kühlsystem eingesetzten superabsorbierenden Aktivierungsmittels kann eine Kühlung binnen Sekunden stattfinden. Die Flüssigkeitsaufnahme kann bis bis zu einem Mehrfachen des Gewichts des Ausgangsmaterials des Kühlsystems. Aufgrund der besonderen Ausgestaltung beziehungsweise der Eigenschaften des superabsorbierenden Aktivierungsmittels wird die aufgegebene Flüssigkeit vollständig aufgenommen, unter mechanischem Druck jedoch nicht wieder abgegeben. Dadurch entsteht beim Einsatz des Kühlsystems keine Nässe, die die gekühlten Körper oder Gegenstände beziehungsweise deren Teile feucht werden lassen würde. Hierdurch wird auch verhindert, dass Messungen, beispielsweise notwendige medizinische Messungen, durch die Befeuchtung verfälscht oder verhindert werden.

Das erfindungsgemäße Kühlsystem umfasst vorteilhafterweise einen Aufbewahrungsbehälter, welcher die mit Flüssigkeit beladene Umhüllung aufnimmt. Im medizinischen sowie im Sportbereich kann somit ein Produkt zur Verfügung gestellt werden, das ready-to-use ist. Das heißt, in dem im erfindungsgemäßen Kühlsystem vorgesehenen Aufbewahrungsbehälter wird die Umhüllung, die bereits mit Flüssigkeit beladen ist, vorgehalten und ist, nach Öffnen des Aufbewahrungsbehälters gleich einsetzbar, da unmittelbar nach der Entnahme der Umhüllung aus dem Aufbewahrungsbehälter das Verdunsten beziehungsweise Verdampfen der Flüssigkeit einsetzt und damit die Kühlleistung zur Verfügung steht.

Eine vorteilhafte Weiterbildung des erfindungsgemäßen Kühlsystems sieht daher vor, dass der Aufbewahrungsbehälter als insbesondere evakuierbare, verschweißbare KunststoffVerpackung vorgesehen ist. Hierin wird die Umhüllung im nicht evakuierten Zustand des Behälters eingelegt, und nach Evakuieren und Verschließen des Behälters in diesem vorgehalten. Aufgrund der Evakuierung und luftdichten Verschweißung der Kunststoffverpackung ergibt sich eine dauerhafte Lagerfähigkeit der entsprechend voraktivierten Umhüllung, die dann bedarfsweise entnommen werden kann, um beispielsweise nach Verletzungen bei sportlicher Betätigung, oder im Medizinbereich zur Kühlung herangezogen werden.

Ein weiterer Vorteil einer Kunststoffverpackung ist darin zu sehen, dass die Umhüllung ebenso wie die für die Beladung verwendeten Flüssigkeiten unter sterilen Bedingungen in den Behälter eingebracht oder nach Einbringung sterilisiert werden können, sodass auch in hygienisch kritischen Umgebungen, beispielsweise im Medizin- oder OP-bereich, aber auch in den anderen vorgenannten Anwendungsbereichen eine Verwendung der erfindungsgemäßen Umhüllung ermöglicht wird. Neben der verschweißbaren Einwegkunststoffverpackung besteht selbstverständlich auch die Möglichkeit, dass der Aufbewahrungsbehälter als wiederverschließbarer beziehungsweise wiederverwendbarer Behälter aus Kunststoff oder Metall ausgebildet ist. Auch hier ist dann die Möglichkeit gegeben den Behälter entsprechend zu sterilisieren oder zu dekontaminieren, um auch hier die Verwendbarkeit in kritischen Umgebungen ermöglichen zu können. Bei den wiederverwendbaren und wiederverschließbaren Behältern erweist es sich weiterhin als günstig, wenn der Aufbewahrungsbehälter weitere Flüssigkeit für das wiederholte Beladen und Aktivieren der Umhüllung aufnimmt, bereitstellt oder z. B. in Art eines Durchlaufkühlers weitergibt, sodass eine Unabhängigkeit von einer externen Flüssigkeitsquelle erreicht werden kann. Die Umhüllung wird zu Kühlzwecken aus dem weitere Flüssigkeit enthaltenen Aufbewahrungsbehälter entnommen, für die Kühlanwendung herangezogen und nach vollständigem Verdunsten, der in der Umhüllung aufgenommenen Flüssigkeit wieder in den Aufbewahrungsbehälter zurückgelegt, um hier wiederholt beladen und aktiviert zu werden. Dieser Vorgang kann so oft wiederholt werden wie Flüssigkeit im Aufbewahrungsbehälter vorhanden ist.

Als vorteilhaft erweist es sich in diesem Zusammenhang, wenn der Aufbewahrungsbehälter eine antibakterielle oder antimikrobielle Flüssigkeit und/oder eine entsprechende Beschichtung zumindest auf einem Teil der der Umhüllung zugewandten Fläche des Aufbewahrungsbehälters aufweist, um hier eine entsprechende Sterilität des Aufbewahrungsbehälters zum Einen und der Umhüllung zum Anderen sicherstellen zu können. Als weiterhin günstig erweist es sich, wenn der Inhalt des Aufbewahrungsbehälters desinfizierbar und/oder sterilisierbar ist und der Aufbewahrungsbehälter entsprechend ausgeführt ist, um mehrfache Sterilisierungs- beziehungsweise Desinfektionsvorgänge aushalten zu können.

Die Abgabe von Flüssigkeit aus der Umhüllung beziehungsweise dem Kühlsystem erfolgt nur durch Verdunstung, es tritt zu keiner Zeit Flüssigkeit aus. Um eine besonders schonende und effektive Kühlung im Kühlsystem durchführen zu können, wird es als günstig angesehen, wenn ein Sensor zur Erfassung der Außentemperatur und der Umhüllungstemperatur vorgesehen ist. Aus den dabei ermittelten Werten kann dann nach Vergleich mit den bauartbedingt durch das Kühlsystem zur Verfügung stellbaren Parameter hinsichtlich Kühltemperatur und Kühldauer eine optimale Befüllung des Kühlsystems mit Flüssigkeit beziehungsweise die optimale Kühldauer und Kühlintensität festgestellt und eingestellt werden.

Dem Sensor kann dabei eine Temperaturanzeige zugeordnet werden, die die aktuell ermittelten Parameter anzeigt, sodass auf einen Blick schnell der Zustand des Kühlsystems erfasst werden kann. Das erfindungsgemäße Kühlsystem weist bevorzugt einen Sensor auf, der in Form einer Thermofarbenbeschichtung oder als Thermometer ausgebildet ist und in die Umhüllung beziehungsweise das Flächengebilde integriert ist.

Neben einer Erfassung von internen Parametern des Kühlsystems weist dieses günstigerweise auch einen Sensor auf, der neben Temperatur und Feuchtigkeit zusätzliche Klimafaktoren der Umgebung des Kühlsystems erfasst. Hierbei bietet sich insbesondere die Erfassung von Umgebungstemperatur, relativer Luftfeuchte in verschiedenen Abständen von dem Kühlsystem sowie die Erfassung von Luftströmen sowie deren Richtung und Stärke an. Aus den somit ermittelten Werten kann dann ein Klimaprofil abgeleitet werden, das den Verwender des erfindungsgemäßen Kühlsystems Hinweise darauf gibt, wie die Umhüllung beziehungsweise das Kühlsystem optimal eingesetzt werden kann, um eine entsprechend zufriedenstellende Kühlleistung und Kühlwirkung zu erzielen. Aus den ermittelten Parametern kann ein persönlicher Klimaindex für den Träger beziehungsweise Verwender des Kühlsystems errechnet werden, der unter Anderem auch zur Messung des individuellen Stresses herangezogen werden kann, wobei sich dann die Möglichkeit bietet, über punktgenaue beziehungsweise bedarfsgenaue Kühlung eine Minderung der Stressfaktoren zu erreichen und um somit das Wohlbefinden des Verwenders oder Trägers des Kühlsystems sicherstellen zu können.

Ebenfalls von der Erfindung umfasst ist die Verwendung eines Gemisches aus Alkohol und Wasser zur Beladung einer Umhüllung wie vorgenannt, beziehungsweise zur Verwendung in einem Kühlsystem, insbesondere für den Einsatz im Rahmen einer medizinisch notwendigen kontrollierten und überwachbaren Kühlung von Menschen und Tieren beziehungsweise einem entsprechenden mit einem Sensor zur Erfassung der Außentemperatur und der Umhüllungstemperatur ausgerüsteten Kühlsystem wie zuvor beschrieben.

Bevorzugt wird das Gemisch dabei auf die Umhüllung aufgetragen und/oder in die Umhüllung oder ein in der Umhüllung vorgesehenes Flächengebilde eingefüllt oder diese entsprechend mit dem Gemisch beladen. Die Beladung beziehungsweise Befüllung kann entweder über entsprechende Anschlüsse an der Umhüllung erfolgen oder aber dadurch, dass die gesamte Umhüllung beziehungsweise das Flächengebilde in eine entsprechendes Gemisch eingetaucht, eingelegt oder mit diesem besprüht wird, wobei dann eine Aufnahme des Gemisches durch die Umhüllung oder das Flächengebilde erfolgt. Das für die Verwendung zur Verfügung gestellte Gemisch weist bevorzugt 1 bis 70 Vol.%, günstigerweise zwischen 5 und 50 Vol.-%, insbesondere jedoch zwischen 10 und 30 Vol.-% Alkohol und zwischen 30 und 99 Vol.% Wasser auf. Je nach Verwendungszweck können hier die entsprechenden Anteile variiert werden.

Als günstig wird angesehen, wenn der Alkohol ausgewählt ist aus der Gruppe bestehend aus Ethanol, Isopropanol oder Mischungen daraus. Zusätzlich besteht in einer bevorzugten Ausführungsform der Verwendung die Möglichkeit, dem Alkohol-Wasser-Gemisch einen Zusatzstoff beizumischen, wobei es sich insbesondere um ein ätherisches Öl, einen Duftstoff, einen Aromastoff, ein Repellens (jeweils synthetisch oder natürlich) oder ein Vergällungsmittel handelt und der Anteil des entsprechenden Zusatzstoffes im Bereich von 0,1 bis 5 Vol.% liegt. Die Beimischung bleibt dabei nicht auf die Verwendung eines einzigen Zusatzstoffes beschränkt, vielmehr besteht die Möglichkeit, dass die Zusatzstoffe in entsprechenden Mischungen beigegeben werden oder aber, dass das Alkohol-Wasser-Gemisch alle genannten Substanzen als Beimischung aufweist.

Die erfindungsgemäße Verwendung zeichnet sich ferner dadurch aus, dass das Wasser ausgewählt ist aus der Gruppe bestehend aus Leitungswasser, mineralisiertem Wasser, destilliertem Wasser, demineralisiertem Wasser oder Reinstwasser, wodurch eine Verwendung für entsprechend sensible Anwendungen, beispielsweise im medizinischen Bereich ermöglicht wird. Das Gemisch, das sich für die erfindungsgemäße Verwendung eignet, kann zusätzlich oder alternativ zu den vorher bereits genannten Zusatzstoffen eine Beimischung von Bioziden, Fungiziden, Viruziden, Repellentien oder antimikrobiellen beziehungsweise antibiotischen Substanzen aufweisen oder Substanzen beinhalten, die eine Erneuerung, Auffrischung oder Herstellung einer spezifischen Ausrüstung oder zusätzlichen Funktionalisierung der Umhüllung ermöglichen, letztgenannte Substanzen können auch als Alternative zum Alkoholanteil des Alkohol-Wasser-Gemisches verwendet werden und dann im in diesem Zusammenhang beschriebenen Volumenanteil dem Wasser beigemischt werden. Auch möglich ist die Verwendung von Gemischen aus Auffrischungssubstanzen für die Ausrüstung, Alkohol und/oder sonstigen Aktivierungsmitteln oder Beimischungen wie oben angegeben. Der Anteil der entsprechenden Beimischungen oder Beimischung liegt dabei günstigerweise bei einem Anteil von 0,1 bis 5 Vol.%. Durch die oben genannten Substanzen wird die Verwendung des Alkohol-Wasser-Gemisches in Anwendungsbereichen möglich, die besondere Anforderungen an die Hygiene stellen. Darüber hinaus wird durch die entsprechende Verwendung eine weitere Verbesserung der Heilung, beispielsweise bei Verwendung der erfindungsgemäßen Umhüllung als Wundabdeckung möglich. Die entsprechenden Beimischungen erweitern somit das Einsatzspektrum der erfindungsgemäßen Umhüllung beziehungsweise tragen zu einem noch spezifischereren Einsatz bei.

Die Erfindung umfasst zusätzlich auch ein Kleidungsstück, bei dem es sich insbesondere um ein Oberbekleidungsstück handelt, das als Umhüllung, wie zuvor beschrieben, dient. Das Kleidungsstück, Kleidungsaccessoire, Hosenträger, Schutzkleidung, Plane, Decke, Decken- oder Dachkonstruktion, Medizinprodukt, OP-Decke, Pulskühler, Halsband, Kühlband, Kühltasche, Bandage, Fuß- und Gelenkbandage, Orthese, Gesichtsmaske, Augenmaske, Strumpf, Strumpfhose, Kopfbedeckung, Abdeckung, insbesondere Gebäude-, Maschinen- oder Geräteabdeckung, Markise, Zellstoff, Sonnendach, Abdeckplane, beziehungsweise Abdeckschutzplane oder Ausrüstungsgegenstand zum Schutz vor hohen Temperaturen, Tasche, Beutel, Rucksack beziehungsweise anderes Sport- oder Outdoorprodukt , Sporttrikot, Sportbekleidung, Unterwäscheaufbewahrung, Transportschutztierkühlungsprodukt oder dergleichen weist dabei mehrere Bereiche auf, wobei vorgesehen ist, dass wenigstens in einem ersten Bereich ein, wie zuvor beschrieben ausgeführtes Flächengebilde angeordnet ist. Das in dem ersten Bereich eines Kleidungsstücks angeordnete Flächengebilde weist dabei bevorzugt eine superabsorbierende Aktivierung auf und eignet sich besonders zur Kühlung nach Beladung mit Flüssigkeit. Des Weiteren umfasst das erfindungsgemäße Kleidungsstück wenigstens einen zweiten Bereich, in dem ebenfalls ein Flächengebilde angeordnet werden kann jedoch nicht muss, dieses hierbei jedoch eine geringere oder keine Kühlwirkung aufweist, da insbesondere keine superabsorbierende Polymere in dem Flächengebilde oder kein Flächengebilde angeordnet sind. Eine erfindungsgemäße Ausgestaltung des Kleidungsstückes sieht vor, das der erste, mit dem superabsorbierend aktivierten Flächengebilde ausgerüstete Bereich des Kleidungsstückes vom zweiten Bereich, der keine Kühlwirkung aufweist, getrennt werden kann. Daneben besteht auch die Möglichkeit, dass die nicht mit dem superabsorbierend aktivierten Flächengebilde ausgerüsteten Bereiche des Kleidungsstückes im Kleidungsstück selbst angeordnet bleiben und damit Körperbereiche aussparen, in denen keine Kühlung vorgesehen beziehungsweise medizinisch angeraten ist. Die Anordnung eines superabsorbierend aktivierten Flächengebildes in Kleidungsstücken führt zu einer Steigerung der Dicke des Kleidungsstückes, insbesondere nach der Beladung mit Flüssigkeit. Um hier einen Ausgleich und eine gleichmäßige Oberflächenstruktur beziehungsweise gleichmäßige Dicke/Stärke des Kleidungsstücks zu erreichen beziehungsweise zu erhalten, ist in dem zweiten Bereich ebenfalls die Anordnung eines Flächengebildes möglich, das die gleiche Dicke/Stärke aufweist, wie das aktivierbare Flächengebilde, und das dazu beiträgt, dass das Kleidungsstück ein einheitliches Erscheinungsbild zeigt. Eine entsprechende Ausgestaltung ist jedoch nicht zwingend notwendig. Die Ausbildung des Kleidungsstückes mit mehreren Bereichen, die von einander trennbar oder zusammenhängend sind, ermöglicht beispielsweise eine punktuelle oder bereichsweise Kühlung von bestimmten Körperregionen. Dies soll an einem Beispiel ausgeführt werden: Es hat sich zum Beispiel gezeigt, dass es zu einer Leistungssteigerung bei Sportlern beitragen kann, bestimmte Körperbereiche zu kühlen. Diese Kühlung kann über den bereits zuvor beschriebenen Kühleffekt des superabsorbierend aktivierten Flächengebildes erreicht werden. Wäre nun ein entsprechendes Sportbekleidungsstück vollständig mit dem aktivierbaren Flächengebilde ausgestattet, würde eine Komplettkühlung des Sportlerkörpers durchgeführt. Dies erweist sich jedoch als nachteilig in bestimmten Körperregionen, die bei Kühlung schnell zu Entzündungen neigen, wie dies beispielsweise in der Nierenregion der Fall ist. Ist nun eine teilweise Kühlung des Sportlerkörpers vorgesehen, so können, insbesondere im Bereich der Brust oder des oberen Rückens, Flächengebilde, die zur Kühlung dienen und daher entsprechend aktiviert beziehungsweise aktivierbar sind, in dem Kleidungsstück angeordnet werden. Der abtrennbare oder getrennt davon vorgesehene Bereich, beispielsweise im Bereich der Nieren oder des Beckens des Sportlers wäre dann, bei Anordnung der Flächengebilde im oberen Bereich der Sportbekleidung weniger dick und würde somit ein ungleichmäßiges Erscheinungsbild des Kleidungsstücks bewirken. Hier kann nun die Anordnung von nichtaktivierten Flächengebilden in dem zweiten Bereich des Kleidungsstückes erfolgen und somit wieder eine gleichmäßige Oberfläche und ein durchgehendes Erscheinungsbild hergestellt werden. Daneben besteht auch die Möglichkeit, dass der nicht mit der Umhüllung belegte beziehungsweise nicht mit Flächengebilde ausgestattete Teil des Kleidungsstückes von diesem abgetrennt wird, und dieser bei der Aktivierung nicht nass wird oder aber am Kleidungsstück verbleibt und die nicht zu kühlenden Bereiche lediglich bedeckt.

Als vorteilhaft wird angesehen, wenn der erste Bereich des Textilproduktes an den zweite oder weitere Bereiche des Textilproduktes durch ein oder mehrere Verbindungsmittel anschließbar ist. Als Verbindungsmittel kommen hier insbesondere Knöpfe, Druckknöpfe, Klettverschlüsse, Schnallen, Steckverschlüsse oder Reißverschlüsse in Frage, über die die Trennung und Verbindung der Kleidungsstückbereiche realisiert

Als Vorteile der Erfindung in ihren vorgenannten Ausführungsformen sind anzusehen, dass hierdurch eine kontrollierbare, individuelle und temperaturabhängige Kühlung von Gegenständen, Menschen und Tieren zur Verfügung gestellt wird, die zum Einen geräteunabhängig durch physikalische Verdunstungskälte realisiert werden kann und zum Anderen ortsunabhängig einsetzbar und leicht zu transportieren ist. Aufgrund der Ausgestaltung der erfindungsgemäßen Gegenstände und Vorrichtungen erlaubt die Erfindung eine schnelle Einsatzbereitschaft, eine dauerhafte Kühlung über Stunden bis hin zu Tagen sowie eine professionelle Kühlung für eine Vielzahl medizinischer Indikationen. Inbesondere im Medizinbereich ist die Kühlung kombinierbar mit medizinischen und telemedizinischen Anwendungen. Durch die als vorteilhaft angesehenen Ausführungsformen der Erfindung ist das zur Verfügung gestellte Flächengebilde beziehungsweise die damit ausgestattete Umhüllung waschbar und sterilierbar ausgebildet und damit wiederverwertbar. Durch die Unabhängigkeit von Geräten und die einfache Kühlung über die Ausnutzung der physikalischen Verdunstungskälte wird zudem durch die Erfindung der C0²-Ausstoß bei der Kühlung wesentlich reduziert. Die Verwendung eines geräteunabhängigen, ortsunabhängigen und leicht zu transportierenden Systems senkt zusätzlich die Einsatzkosten für die Kühlung und, insbesondere im Zusammenhang mit einer medizinischen Anwendung der Kühlung, damit auch die Gesundheitskosten. Dadurch, dass die ebenfalls von der Erfindung umfasste Umhüllung beziehungsweise die die Umhüllung aufweisenden Kleidungsstücke besonders im unbeladenen Zustand sehr leicht ausgeführt sind, belasten diese den Träger nicht und erhöhen damit dessen Lebensqualität, stellen dabei jedoch auch die bedarfsweise notwendige, teilweise lebensrettende Kühlung sicher.

In einem Beispiel soll der Aufbau einer Zusammensetzung des erfindungsgemäßen Flächengebildes, das sich für den Einsatz in den vorgenannten Gegenständen und Artikeln eignet, beschrieben werden. Die angegebenen Werte verstehen sich jedoch lediglich als Beispiele einer möglichen Ausführungsform des erfindungsgemäßen Flächengebildes, beschränken dabei jedoch nicht den mit der Erfindung angestrebten Schutzumfang. Vorgestellt wird ein Flächengebilde, das ein Polyestervlies umfasst. Das beispielhafte Flächengebilde umfasst ein Polyestervlies, das in einem Schmelzspinnverfahren hergestellte Fasern umfasst. Die Polymerlösung liegt dabei zunächst in flüssiger Form vor und wird aus einer Spinndüse gepresst. Nach dem Austritt des noch flüssigen Polymermaterials aus der Spinndüse erhärtet sich dieses bei Auskühlen an der Luft und bildet dabei, auch in Verbindung mit der zusätzlichen Beaufschlagung durch einen Luftstrom, eine im Prinzip unendliche Faser aus. Die Faser trifft am Ende des Luftstroms auf ein Siebband, an das eine Absaugung angeschlossen ist. Die aus den Düsen austretenden Fasern werden wirr auf dem Siebband angeordnet und bilden dabei das Vlies. Nach Erreichen einer bestimmten Vliesdicke beziehungsweise einer entsprechenden flächenbezogenen Masse von 100 g/m² wird das gebildete Vlies von dem Siebband abgenommen und für die Weiterverarbeitung zur Verfügung gestellt. Es ist hierbei beabsichtigt, die Beaufschlagung des Vlieses mit einem superabsorbierenden Polymer durchzuführen. Das superabsorbierende Polymer liegt im Ausführungsbeispiel als partikelförmiges Pulver vor. Um nun eine Verbindung zwischen superabsorbierenden Polymerpartikeln und Vlies durchzuführen, wird das Vlies zunächst beidseitig mit einer KleberSubstanz belegt. Dies erfolgt durch Aufsprühen des Klebers auf dem Polyestervlies. Das Aufsprühen erfolgt über in einem Düsenbalken angeordneten Düsen, die gleichmäßig beabstandet über das Vlies beziehungsweise dessen Oberfläche geführt werden. Der aufgesprühte Kleber dringt dabei teilweise in das noch lose gestapelte Rohvlies ein und bewirkt auch eine Beschichtung der im Inneren des Vlieses liegenden Fasern. Bei dem verwendeten Kleber handelt es sich bevorzugt um eine wässrige Dispersion von Copolymerisaten auf der Basis von Styrol und Acrylester, wie beispielsweise aus der Papierherstellung unter dem Produktname Acronal® bekannt sind. Es ist hierbei vorgesehen, um eine besonders gute Verbindung zwischen superabsorbierenden Polymerpartikeln und Polyestervlies zu erreichen, 20 g/m² Kleber auf das Vlies aufzubringen. Nach dem Aufbringen des Klebers erfolgt die Belegung des Vlieses mit superabsorbierenden Polymerpartikeln. Hierbei ist vorgesehen, dass 400 g Partikel pro m² Vlies auf diesem aufgebracht beziehungsweise in dieses eingearbeitet werden. Durch die vorherige Behandlung des Vlieses mit Kleber weist dieses eine Haftoberfläche auf, und kann somit mit den Partikeln verbunden werden. Aufgrund der Verwendung von 400 g superabsorbierenden Polymerpartikeln pro m² Vlies liegt der Anteil der superabsorbierenden Aktivierung des Vlieses bei einem flächenbezogenen Gewichtsanteil von 400 % des Ausgangsvliesmaterials. Neben dem Aufsprühen des Klebers auf dem Vliesmaterial besteht auch die Möglichkeit den Kleber in einem sogenannten Foulardierverfahren auf dem Vlies aufzubringen. Hierbei wird das Vlies durch ein sogenanntes Foulardierbad gezogen, das den Kleber beinhaltet. Der Kleber wird anschließend abgepresst, sodass nur eine definierte Menge an Kleber im Vlies verbleibt. Bei den das Vlies bildenden Fasern handelt es sich um Fasern mit einer Garnfeinheit von 2 - 5 dtex. Selbstverständlich besteht auch die Möglichkeit hier geringere oder höhere Garnfeinheiten zu wählen. Die Garnfeinheit ist in Abhängigkeit von der letztendlichen Verwendungseignung des fertigen, aktivierten Vlies auszuwählen. Insgesamt weist das gebildete Flächengebilde somit eine flächenbezogene Masse von 520 g/m² auf. Aufgrund der Verwendung von 400 g superabsorbierendem Polymer, das bis zum tausendfachen seines eigenen Gewichts an Flüssigkeit aufnehmen kann, können hier in dem beispielhaft vorgestellten Flächengebilde bis zu 400.000 g Wasser, das heißt, bis zu 400 Liter Wasser pro m² gespeichert werden.

In einer anderen erfindungsgemäßen Ausgestaltung des Flächengebildes weist das Vlies eine flächenbezogene Masse im Bereich von 50 bis 100 g/m², bevorzugt ca. 50 bis 80 g/m², und einen Anteil an superabsorbierender Aktivierung, insbesondere an Superabsorberpolymeren mit einer flächenbezogenen Masse von 50 bis 400 g/m², bevorzugt ca. 160 bis 240 g/m², insbesondere ca. 200 g/m² +/- 5% beziehungsweise +/- 10%, auf. Gegebenenfalls wird zu einer Verbesserung des Anhaftens der Superabsorberpolymere an dem bevorzugt als Polyestervlies ausgebildeten Vlies ein Klebemittel, zum Beispiel auf Basis einer Polyacrylsäure, eingesetzt, das mit einer flächenbezogenen Masse von 5 bis 20 g/m², ein- oder doppelseitig auf das Vlies aufgetragen, insbesondere aufgesprüht oder foulardiert wird.

Hieraus resultiert ein erfindungsgemäßes Flächengebilde mit einer flächenbezogenen Masse im Bereich von 100 bis 700 g/m².

In diesem Zusammenhang wird insbesondere darauf hingewiesen, dass alle im Bezug auf die Vorrichtung beschriebenen Merkmale und Eigenschaften aber auch Verfahrensweisen sinngemäß auch bezüglich der Formulierung des erfindungsgemäßen Verfahrens übertragbar und im Sinne der Erfindung einsetzbar und als mit offenbart gelten. Gleiches gilt auch in umgekehrter Richtung, das bedeutet, nur im Bezug auf das Verfahren genannte, bauliche also vorrichtungsgemäße Merkmale können auch im Rahmen der Vorrichtungsansprüche berücksichtigt und beansprucht werden und zählen ebenfalls zur Erfindung und zur Offenbarung.

In den Zeichnungen ist die Erfindung schematisch dargestellt. Es zeigt

Fig. 1 eine Schnittdarstellung einer mit dem erfindungsgemäßen Flächengebilde ausgestatteten Umhüllung,

Fig. 2 eine Schnittdarstellung einer mit dem erfindungsgemäßen Flächengebilde ausgestatteten Umhüllung im Beladezustand,

Fig. 3 eine Schnittdarstellung einer mit einer weiteren Ausführungsform des erfindungsgemäßen Flächengebilde ausgestatteten Umhüllung.

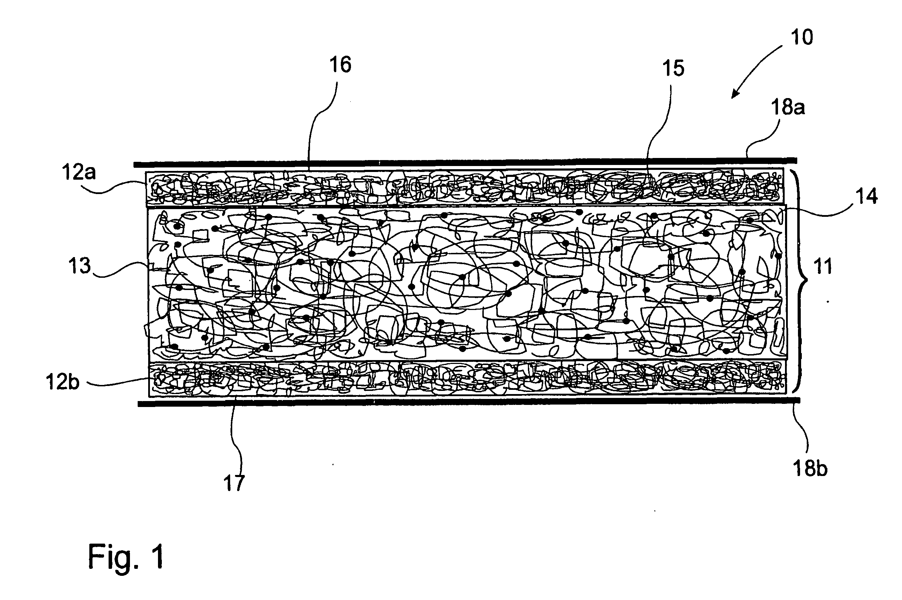

Die in der Fig. 1 dargestellte Umhüllung 10 verfügt in ihrem Inneren über ein Flächengebilde 11, das insgesamt einen dreischichtigen Aufbau aufweist. Die beiden äußeren Schichten 12a, 12b, bestehen dabei aus dem gleichen Material wie die innere Schicht 13, das jedoch eine veränderte Struktur und Dichte aufweist. Im Ausführungsbeispiel der Fig. 1 wird das Flächengebilde 11 durch ein Wirrvlies gebildet, das aus unkontrolliert gestapelten Fasern 14 gebildet ist. Die Fasern 14 wurden im Ausführungsbeispiel in einem Schmelzspinnverfahren gebildet und bestehen aus thermoplastischen Polymeren. Im Vliesmaterial des Flächengebildes 11 angeordnet befinden sich Partikel 15 aus einem superabsorbierenden Polymer, die chemisch mit den Fasern 14 verbunden sind und dauerhaft an diesen anhaften. Zur Verbindung von Partikeln 15 und Fasern 14 erfolgte durch Beaufschlagung des Flächengebildes 11 mit einem Vernetzungsmittel. Dieses bewirkte zunächst eine Vernetzung des superabsorbierenden Polymers beziehungsweise dessen Precursorn und im Zuge dessen eine Verbindung zwischen Fasern 14 und den Partikeln 15. Die Partikel 15 werden von den Fasern 14 durchzogen. Neben der Verwendung von superabsorbierenden Polymeren in Form von Partikeln 15 besteht selbstverständlich auch die Möglichkeit, dass die Fasern 14 mit superabsorbierenden Polymer beschichtet sind beziehungsweise vollständig aus dem superabsorbierenden Polymer bestehen. Neben der vollständigen Bildung der Fasern 14 aus superabsorbierendem Polymer besteht auch die Möglichkeit, dass das superabsorbierende Polymer in die Fasern 14 beziehungsweise in die Ausgangspolymerlösung, aus denen die Fasern 14 gesponnen werden, einzufügen.

Die Bildung des Vlieses, das letztendlich das Flächengebilde 11 bildet, erfolgt durch Auftrag der aus einer Düse ausgestoßenen Fasern 14 auf ein Siebband oder eine sonstige durch Unterdruck beaufschlagbare Fläche (nicht dargestellt) . Zusätzlich kann zur Verbesserung der Vlieseigenschaften beziehungsweise der Eigenschaften des Flächengebildes 11 eine Vernadelung oder sonstige Verfestigung des Flächengebildes 11 durchgeführt werden. Hierbei werden dann, beispielsweise bei der Vernadelung einzelne Fasern von einer ersten Oberfläche 16 wenigstens teilweise in den Vlieskörper beziehungsweise in das Vlies eingeführt und verfestigen dabei dessen Struktur.

Im Ausführungsbeispiel der Fig. 1 erfolgte nur eine Oberflächenbehandlung des Rohvlieses, sodass sich hier an der ersten hier oberen Oberfläche 16 und der zweiten hier unteren Oberfläche 17 eine Vliesschicht gebildet hat, die gegenüber dem übrigen Vlieskörper, das heißt, gegenüber der inneren Schicht 13 eine verfestigte Struktur aufweist. Die Bildung der äußeren Schichten 12a, 12b kann dabei durch thermische, chemische oder mechanische Behandlung des Vlieses beziehungsweise des Rohvlieses unmittelbar nach dessen Bildung oder auch später erfolgen. Bei einer mechanischen Verfestigung wird ein Vernadelung oder Wasserstrahlverfahren eingesetzt, um eine Ausrichtung eines Teils der Fasern 14 der äußeren Schicht 12a, 12b durchzuführen. Über die Eindringtiefe der Nadeln beziehungsweise des Wasserstrahls in das Rohvlies wird die letztendliche Dicke und auch die Dichte der äußeren Schichten 12a, 12b festgelegt.

Durch die Anzahl der in das Vlies eindringenden Nadeln beziehungsweise Wasserstrahlen wird die Dichte und Verfestigung weiter bestimmt. Bei einem chemischen Verfahren werden die äußeren Schichten 12a, 12b des Vlieses mit einer Substanz behandelt, die ein Aufweichen der Fasern 14 beziehungsweise der Fasermäntel (nicht erkennbar) bewirkt und, wobei nach einem Wiedererhärten der Fasern, die Verbindung beziehungsweise das "Verschmelzen" der derart vorbehandelten Fasern durchgeführt wird.

Neben den genannten mechanischen und chemischen Verfahren besteht auch die Möglichkeit zur thermischen Behandlung des Rohvlieses. Dies bietet sich vor allem dann an, wenn der Vlieskörper beziehungsweise das Rohvlies aus thermoplastischen Polymeren gebildet ist, das heißt, wenn die Fasern 14 aus einer thermische verflüssigbaren Polymermischung bestehen. Hierbei kann durch die Beaufschlagung des Rohvlieses mit einem erhitzten Gasstrom oder mit Wasserdampf oder aber durch das Auflegen oder Überrollen des Vlieses mit beheizbaren Walzen, ein Aufschmelzen der Fasern 14 beziehungsweise der Fasermäntel durchgeführt werden. Vor dem Erkalten des dermaßen behandelten Rohvlieses kommt es zu einem Aneinanderlegen beziehungsweise Verkleben der angelösten beziehungsweise aufgeweichten Fasermäntel, die nach dem Abkühlen in dieser Position dauerhaft verbleiben und miteinander verschmolzen sind. Durch die Intensität und Dauer der Beaufschlagung des Rohvlieses kann hier auch die Eindringtiefe beziehungsweise der Anteil der Fasern 14 definiert werden, der dem Schmelzverfahren unterzogen wird und somit die Dicke und Dichte der äußeren Schichten 12a, 12b festgelegt werden, falls diese notwendig sind. Der Übergang zur inneren Schicht 13 kann dabei fließend ausgeführt werden. Neben der Bildung der äußeren Schichten 12a, 12b aus dem Rohvlies in einem einzigen Arbeitsschritt, kann selbstverständlich auch die Anordnung von zusätzlichen Materialbahnen auf der inneren Schicht 13 erfolgen. Diese Materialbahnen können dann auch ohne superabsorbierende Funktionalisierung ausgeführt sein und in ihrem Inneren die innere Schicht 13 des Flächengebildes 11 aufnehmen. Neben der Anordnung zusätzlicher Vliesbahnen als äußere Schichten 12a, 12b besteht auch die Möglichkeit, das Flächengebilde 11 mit Auflagen 18a, 18b die als Membran oder Folie ausgebildet sind, zu belegen beziehungsweise zu umhüllen. Über diese Auflagen 18a, 18b kann eine zusätzliche Funktionalisierung des Flächengebildes 11 beziehungsweise der dieses beinhaltenden oder bildenden Umhüllung 10 durchgeführt werden. So können die Auflagen 18a, 18b beispielsweise wasserdicht, schmutzabweisend oder in sonstiger geeignet erscheinender Art und Weise aktiviert sein. Gleichzeitig besteht auch die Möglichkeit, dass die Auflagen 18a, 18b als Beschichtung ausgebildet sind, die auf der ersten und zweiten Oberfläche 16, 17 oder aber auch nur auf einer der Oberflächen 16, 17 aufgetragen wird. Die Beschichtung kann dann auch eine zusätzliche Aktivierung des Flächengebildes 11 oder der gesamten Umhüllung 10 zur Verfügung stellen. So besteht beispielsweise die Möglichkeit, die Beschichtung aus einem flüssigkeitsdichten, flammfesten, selbstreinigenden, schmutzabweisenden, bioziden, antiviralen oder antimikrobiellen Material zu bilden.

Eine weitere Möglichkeit zur Aktivierung der Umhüllung 10 beziehungsweise des Flächengebildes 11 besteht darin, dass entsprechende Aktivierungsmittel bereits in das Vlies, das heißt, insbesondere in die innere Schicht 13 mit eingefügt werden. Dies kann bei der Bildung des Rohvlieses erfolgen. Um hier beispielsweise eine biozide Aktivierung des Flächengebildes 11 durchzuführen, können einzelne Fasern 14 aus einem Silbermaterial oder mit einer Silberbeschichtung ausgeführt werden. Zusätzlich zu der Aktivierung einzelner Fasern 14 des Flächengebildes 11 besteht natürlich auch die Möglichkeit zusätzliche Partikel, beispielsweise Nanopartikel, in dem Flächengebilde 11 einzufügen, die dann die gewünschten zusätzlichen Eigenschaften des Flächengebildes 11 vermitteln.

Insbesondere bei der flüssigkeitsdichten, selbstreinigenden und/oder schmutzabweisenden Aktivierung handelt es sich um eine Modifikation der ersten oder zweiten Oberfläche 16, 17 des Flächengebildes 11 beziehungsweise der Umhüllung 10. Diese Aktivierung, das heißt, die entsprechende Beschichtung kann dabei durch Aufsprühen oder Bestreichen des Flächengebildes 11 beziehungsweise der Umhüllung 10 mit einem entsprechenden Material erfolgen. Daneben besteht auch die Möglichkeit, dass das gesamte Flächengebilde 11 beziehungsweise die gesamte Umhüllung 10 in eine Lösung eingetaucht wird, die die entsprechenden Funktionalisierungs- beziehungsweise Aktivierungsmittel enthält. Nach dem abschließenden Trocknen ist dann eine vollständige Beschichtung gewährleistet. Das Flächengebilde 11 der Fig. 1 weist zur Bildung der Umhüllung 10 zusätzliche Auflagen 18a, 18b auf, die dieses einhüllen. Diese Auflagen 18a, 18b sind dabei so ausgeführt, dass sie ein Eindringen von Flüssigkeit in das Flächengebilde 11 ermöglichen, jedoch nur einen Austritt von Wasserdampf aus dem Flächengebilde 11 zulassen. Die Auflagen 18a, 18b weisen hierzu entsprechende Poren auf (nicht dargestellt), die einen Durchmesser aufweisen, der kleiner ist als die im Flächengebilde 11 beziehungsweise insbesondere in der inneren Schicht 13 angeordneten Partikel 15. Dies ist vor allem dann von Bedeutung, wenn sich einzelne Partikel 15 oder Partikelteile von den Fasern 14 lösen, durch die Porengröße wird gewährleistet, dass die entsprechenden Partikel 15 nicht aus dem Flächengebilde 11 beziehungsweise der Umhüllung 10 austreten können. Auch wird durch die beschriebene Ausgestaltung der Auflagen 18a, 18b verhindert, dass Feuchtigkeit an den Oberflächen 16, 17, die mit dem mit der Umhüllung 10 bedeckten Gegenstand beziehungsweise Körperteil in Verbindung kommen, auftritt.

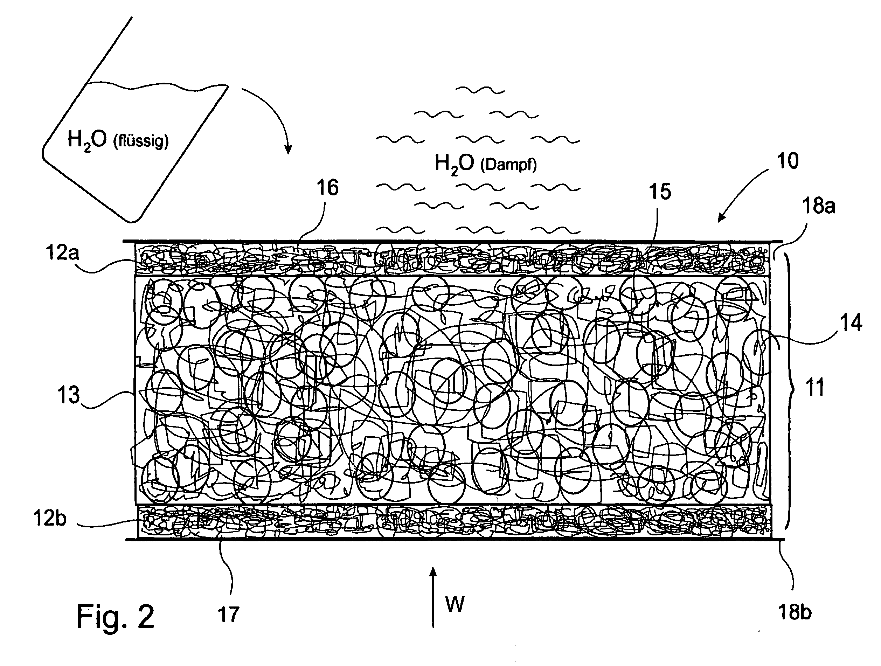

Fig. 2 zeigt das zuvor bereits beschriebene Flächengebilde 11 im beladenen Zustand. Deutlich erkennbar sind die nunmehr in gequollenem Zustand dargestellten Partikel 15 aus einem super-absorbierenden Polymer. Diese sind physikalisch, mechanisch oder chemisch mit den Fasern 14 verbunden und haften dauerhaft an diesen an. Aus Fig. 2 wird auch die relativ homogene Verteilung der Partikel 15 im Flächengebilde 11 erkennbar, die dazu führt, dass kaum Berührungspunkte zwischen den einzelnen Partikel 15 vorhanden sind, diese somit ihre maximale Quellfähigkeit und damit maximale Flüssigkeitsaufnahme erreichen können, ohne durch benachbarte Partikel 15 hierbei beeinträchtigt zu werden. In der Fig. 2 wird ein Aufgießen von Flüssigkeit auf das Flächengebilde 11 dargestellt. Neben dieser Möglichkeit des Aufgießens von Flüssigkeit besteht selbstverständlich auch die Möglichkeit, dass Flüssigkeit auf das Flächengebilde 11 aufgesprüht wird oder aber, dass das gesamte Flächengebilde 11 in die Flüssigkeit eingetaucht wird, sodass dann eine entsprechende Flüssigkeitsaufnahme durch die Partikel 15 stattfinden kann. Als Flüssigkeit wird im Ausführungsbeispiel der Fig. 2 einfaches Wasser verwendet, denkbar ist hier aber auch die Verwendung von Alkohol-Wassergemischen oder von Gemischen aus Alkohol, Wasser und/oder weiteren Zusatzstoffen, die dann gleichzeitig eine entsprechende Aktivierung oder Funktionalisierung des Flächengebildes 11 bewirken. Im Ausführungsbeispiel der Fig. 2 erfolgt eine Beaufschlagung von Flüssigkeit von der ersten Oberfläche 16 des Flächengebildes her. Die aufgebrachte Flüssigkeit durchdringt dabei die als Decklage der Umhüllung 10 ausgeführte Auflage 18a, die die äußere Schicht 12a des Vlieses umgibt und wird in der inneren Schicht 13 des Vlieskörpers von den dort angeordneten und homogen verteilten Partikeln 15 aufgenommen. Von der zweiten Oberseite 17 des Flächengebildes her erfolgt dann eine Wärmebeaufschlagung des Flächengebildes 11, beispielsweise dadurch, dass die Umhüllung 11 als Abdeckung für ein wärmeabgebendes Gerät verwendet wird oder auf einem Körper aufliegt. Diese Wärmebeaufschlagung, im Ausführungsbeispiel dargestellt mit dem Pfeil W, führt zu einem Verdunsten beziehungsweise Verdampfen der in den Partikeln 15 reversibel gebundenen Flüssigkeit. Dieser Verdampfungsprozess setzt voraus, dass dem abgedeckten Gegenstand beziehungsweise Körper oder Körperteil Wärme entzogen wird, wodurch dann eine entsprechende Kühlleistung oder Kühlwirkung eintritt. Die aus den Partikeln 15 verdampfte Flüssigkeit tritt in Form von Wasserdampf an der oberen Oberfläche 16 des Flächengebildes 11 aus diesem aus. Die als Decklage der Umhüllung ausgebildete Auflage 18a weist hierzu eine entsprechende Funktionalisierung auf, die den gleichmäßigen Austritt von Wasserdampf ermöglicht. Im Zuge des Austritts von Flüssigkeit aus den Partikeln 15 unterliegen diese einem Schrumpfungsprozess und erreichen nach vollständiger Abgabe der Flüssigkeit den in Fig. 1 dargestellten ungequollenen Zustand. Nach oder auch bereits während des Verdunstungsprozesses kann eine erneute Beladung des Flächengebildes 11 mit weiterer Flüssigkeit erfolgen, die dann ein Wiederaufquellen der Partikel 15 bewirkt. Das erfindungsgemäße Flächengebilde 11 beziehungsweise die damit ausgestattete Umhüllung 10 ermöglicht so ein wiederholtes Be- und Entladen mit Flüssigkeit und einem dadurch erzielbaren langanhaltenden Kühleffekt. Dieser Kühleffekt beziehungsweise die vorausgehende Flüssigkeitsbeaufschlagung wird dadurch verbessert, dass zum Einen eine homogene Verteilung der Partikel 15 im Flächengebilde 11 vorgesehen ist, und zum Anderen die Partikelgrößen in einem relativ engen Durchmesserbereich liegen, die Partikel 15 somit identische Eigenschaften hinsichtlich der Aufnahme- und Abgabegeschwindigkeit der Flüssigkeit aufweisen. Die dauerhafte Verbindung der Partikel 15 mit den Fasern 14 des Vlieses verhindern ein Ablösen der Partikel 15 und deren Ansammlung beispielsweise im Bereich der unteren Oberfläche 18 der Umhüllung.

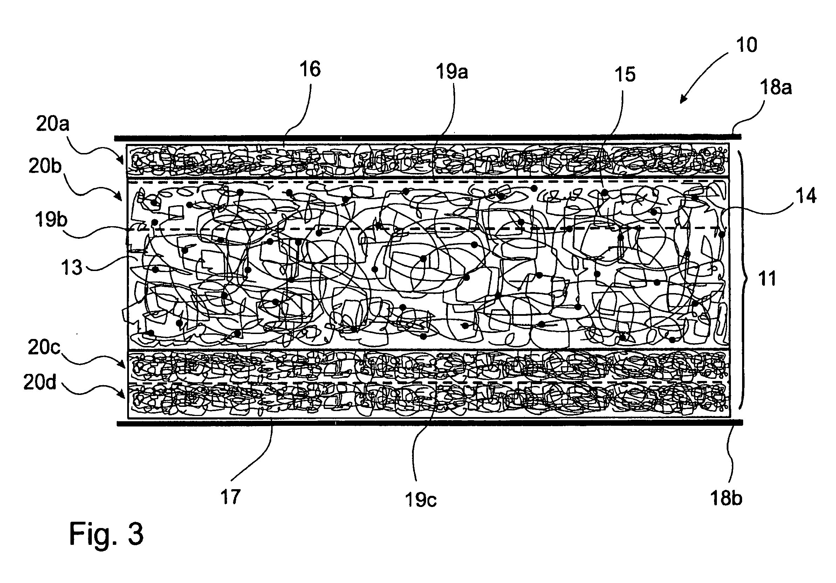

Fig. 3 zeigt den Einsatz von Membranen 19a, 19b, 19c in einer weiteren Ausführungsform des erfindungsgemäßen Flächengebildes 11. Dieses weist insgesamt vier Lagen 20a, 20b, 20c, 2Od auf, die somit eine mehrschichtige Ausführung des Flächengebildes realisieren. Zusätzlich zu den Lagen des Flächengebildes 11 sind Decklagen der Umhüllung 10 in Form von Auflagen, 18a, 18b vorgesehen, die die äußeren Begrenzungsflächen der Umhüllung 10 darstellen und zwischen denen das Flächengebilde 11 aufgenommen ist. Das Flächengebilde 11 weist im Ausführungsbeispiel der Fig. 3 eine innere Schicht 13 auf, die durch ein superabsorbierend aktiviertes Vlies gebildet ist. Hier liegen die superabsorbierenden Polymere ebenfalls in Form von Partikeln 15 vor, die mit den Fasern 14 verbunden und damit dauerhaft in der inneren Schicht 13 fixiert sind. Die innere Schicht 13 bildet somit eine innere Lage 20b des Flächengebildes. Eingeschlossen wird die innere Schicht durch weitere, nicht aktivierte Lagen 20a, 20c, 2Od, die im Ausführungsbeispiel ebenfalls aus einem Vliesmaterial gebildet sind, das keine superabsorbierende Aktivierung aufweist, jedoch den Durchtritt von Flüssigkeit zulässt.

Die innere Schicht 13 ist gegenüber der ersten, oberen Lage 20a des Flächengebildes durch eine erste Membran 19a abgegrenzt. Diese Membran ist jedoch nach Art einer Klimabeziehungsweise Funktionsmembran ausgebildet und erlaubt den Durchtritt von Wasserdampf, der somit aus der inneren Schicht 13 austreten und in die obere Lage 20a eindringen kann, um aus dieser über die Auflage 18a an die Umwelt abgegeben zu werden.

In der inneren Schicht 13 ist eine weitere Membran 19b angeordnet, die dazu dient, eine verbesserte Verteilung der in das Flächengebilde eingebrachten Flüssigkeit zu gewährleisten.

Die innere Schicht 13 des Flächengebildes 11 wird durch zwei weitere Lagen 20c, 20d begrenzt, die wiederum durch eine zwischengelagerte Membran 19c getrennt sind. Diese Membran 19c ist dahingehend realisiert, dass ein Austritt von in das Flächengebilde 11 eingebrachter Flüssigkeit zur unteren Oberfläche 17 des Flächengebildes 11 hin durch diese Membran 19c verhindert wird. Die Verwendung von zwei Lagen 20c, 2Od bewirkt eine zusätzliche Stabilisierung des Flächengebildes 11 und der diesen aufnehmenden Umhüllung 10. Die Umhüllung 10 weist insgesamt zwei als Decklagen anzusehenden Auslagen 18a, 18b auf, zwischen denen das Flächengebilde 11 angeordnet ist. Eine entsprechende Anordnung kann beispielsweise durch Einschieben oder Einlegen des Flächengebildes 11 zwischen die beiden Auflagen 18a, 18b erfolgen. Auch können die Auflagen 18a, 18b insgesamt eine taschenartige Aufnahme für das Flächengebilde 11 bilden, in die dieses bedarfsweise eingesteckt werden kann. Die Auflagen 18a, 18b selbst können auch eine entsprechende Funktionalisierung aufweisen, um somit der Umhüllung weitere Eigenschaften zu verleihen, die dann eine Einsetzbarkeit in verschiedensten Anwendungsgebieten ermöglichen. So können beispielsweise die Auflagen 18a, 18b aber selbstverständlich auch die Lagen 20a, 20c, 2Od und gegebenenfalls auch die innere Schicht 13 eine flüssigkeitsdichte, flammfeste, funkenflugresistente, gegenüber Metall- oder sonstigen Glutspritzern resistente, schmutzabweisende, biozide, antivirale, UV-blockende, antistatische, repellente, kosmetisch wirksame, medizinisch wirksame, hydrophilisierende, antimikrobielle oder elektromagnetische Strahlung abschirmende oder absorbierende Ausstattung oder Ausrüstung beziehungsweise Beschichtung aufweisen. Hierdurch ergibt sich ein sehr weites Einsatzgebiet der erfindungsgemäßen Umhüllung 10.

Während in Fig. 3 die Ausstattung mit insgesamt drei zusätzlichen Membranen 19a, 19b, 19c dargestellt ist, besteht selbstverständlich auch die Möglichkeit, dass hier lediglich zwei die innere Schicht 13 einfassende Membrane vorgesehen sind, daneben können selbstverständlich auch weitere Membrane in dem Flächengebilde 11 angeordnet werden, die dann eine weitergehende Kompartimentierung des Aufbaus des Flächengebildes 11 bewirken und diverse weitere Schutz- oder Verteilungsfunktionen übernehmen beziehungsweise den Eintritt oder Austritt von Flüssigkeit weiter verbessern. Die Membrane können nachträglich im Flächengebilde angeordnet werden, denkbar ist jedoch auch, dass eine entsprechende Membran, beispielsweise als Träger für die Fasern 14, die letztendlich das Vlies bilden, verwendet wird und ein- oder beidseitig mit entsprechenden Fasern 14 im Vliesbildungsprozess belegt wird. Auch denkbar ist daneben eine Teilung des Vlieses nach dessen Bildung, sodass die beiden Vlieshälften dann eine dazwischen angeordnete Membran einfassen. Es besteht daneben auch die Möglichkeit der Bildung des Flächengebildes 11 in einer Sandwichbauweise, das heißt die einzelnen Schichten oder Lagen 20a, 20b, 20c, 2Od beziehungsweise die dazwischen angeordneten Membranen 19a, 19b, 19c werden in einem Schichtaufbauprozess zusammengefügt, das heißt es wird beispielsweise eine erste untere Lage 2Od zur Verfügung gestellt, die dann mit einer Membran 19c belegt wird, wonach dann eine weitere Lage 20c auf der Membran 19c aufgebracht wird. Danach kann dann, beispielsweise eine innere superabsorbierend aktivierte Schicht 13 auf der zweiten Lage 20c angeordnet werden, die wiederum mit einer Membran 19a belegt wird, die die innere Schicht 13 gegenüber einer weiteren Lage 20a abgrenzt, die im Herstellungsprozess dann auf der oberen Membran 19a aufgebracht, aufgetragen, aufgelegt oder aufgeklebt wird.

Über die entsprechende Schichtung können die Eigenschaften, wie beispielsweise Flüssigkeitsaufnahme- und Flüssigkeitsabgabegeschwindigkeit sowie weitere mechanische Parameter des Flächengebildes 11 definiert werden.

Durch die Auswahl von geeigneten Membranen kann eine weitere Funktionalisierung beziehungsweise Aktivierung des Flächengebildes 11 beziehungsweise der Umhüllung 10 erreicht werden. Die Auswahl geeigneter Membrane beeinflusst beispielsweise die Durchlässigkeit für Flüssigkeit beziehungsweise verdampfte Flüssigkeit. Um einen Abschluss gegenüber einem zu kühlenden Körper zu erreichen, kann beispielsweise eine impermeable Membran verwendet werden, währen der Austritt von Wasserdampf aus der Umhüllung 10 beziehungsweise dem Flächengebilde 11 durch eine teilweise undurchlässige, semipermeable oder selektivpermeable beziehungsweise eine in einer Richtung durchlässige, unidirektionale Membran gebildet werden kann. Zusätzlich kann über die Auswahl einer geeigneten Membran auch die mechanische Belastbarkeit der Umhüllung 10 beziehungsweise des Flächengebildes 11 beeinflusst werden, da Membrane die Eigenschaft aufweisen unter einer Belastung nur Zugkräfte aufzunehmen und an ihre Ränder weiterzugeben.

Durch die Ausführung der Membran mit definierter Stärke, Dicke oder Funktionalität kann eine weitere Aktvierung oder Funktionalisierung des Flächengebildes 11 beziehungsweise der Umhüllung 10 erreicht werden, da hier beispielsweise über entsprechend aufgebrachte Membrane die Abdichtung des Flächengebildes 10 erreicht werden kann. Auch unter hygienischen Gesichtspunkten erweist sich die Verwendung von Membranen als vorteilhaft, da diese eine besonders leichte Reinigung des Flächengebildes beziehungsweise der Umhüllung zulassen, und gleichzeitig das innere des Flächengebildes 11 gegenüber aggressiven Reinigungssubstanzen abschirmen.

In den Fig. 1 bis 3 wird die Einbringung von superabsorbierenden Polymeren in dem Flächengebilde 11 lediglich in Form von Partikeln 15 dargestellt. Neben dieser Möglichkeit zur Beaufschlagung besteht selbstverständlich auch die Möglichkeit, dass die Fasern 14 aus superabsorbierendem Polymer gebildet sind, beziehungsweise die superabsorbierenden Polymere in die Fasern eingelagert werden, sodass dann bei Flüssigkeitskontakt ein Quellen der gesamten Faser stattfindet. Im Zuge des Quellvorganges erhöht sich selbstverständlich die Dicke oder Stärke des Flächengebildes, wobei diese Form beziehungsweise Dimensionsänderung aufgrund der homogenen Verteilung der superabsorbieren-Polymerpartikeln 15 im Flächengebilde 11, in engen Grenzen verläuft .

Die jetzt mit der Anmeldung und später eingereichten Ansprüche sind Versuche zur Formulierung ohne Präjudiz für die Erzielung weitergehenden Schutzes.

Sollte sich hier bei näherer Prüfung, insbesondere auch des einschlägigen Standes der Technik, ergeben, dass das eine oder andere Merkmal für das Ziel der Erfindung zwar günstig, nicht aber entscheidend wichtig ist, so wird selbstverständlich schon jetzt eine Formulierung angestrebt, die ein solches Merkmal, insbesondere im Hauptanspruch, nicht mehr aufweist.

Es ist weiter zu beachten, dass die in den verschiedenen Ausführungsformen beschriebenen und in den Figuren gezeigten Ausgestaltungen und Varianten der Erfindung beliebig untereinander kombinierbar sind. Dabei sind einzelne oder mehrere Merkmale beliebig gegeneinander austauschbar. Diese Merkmalskombinationen sind ebenso mit offenbart.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Merkmale, die bislang nur in der Beschreibung offenbart wurden, können im Laufe des Verfahrens als von erfindungswesentlicher Bedeutung, zum Beispiel zur Abgrenzung vom Stand der Technik beansprucht werden.

Merkmale, die nur in der Beschreibung offenbart wurden, oder auch Einzelmerkmale aus Ansprüchen, die eine Mehrzahl von Merkmalen umfassen, können jederzeit zur Abgrenzung vom Stande der Technik in den ersten Anspruch übernommen werden, und zwar auch dann, wenn solche Merkmale im Zusammenhang mit anderen Merkmalen erwähnt wurden beziehungsweise im Zusammenhang mit anderen Merkmalen besonders günstige Ergebnisse erreichen.

## Patentansprüche

1. Mehrfach wiederverwendbares, mit Flüssigkeit beladbares und dadurch aktivierbares, bei Entladung kühlendes Flächengebilde beinhaltend eine superabsorbierende Aktivierung aufweisendes Vlies, wobei das Flächengebilde (11) in trockenem, unbeladenen Zustand eine flächenbezogene Masse zwischen 100 und 1500 g/m², bevorzugt von 100 bis 500 g/m², aufweist und das Vlies eine flächenbezogene Masse zwischen 50 und 120 g/m², insbesondere zwischen 60 und 100 g/m², bevorzugt zwischen 50 und 80 g/m² und/oder das Vlies in dem Flächengebilde (11) eine Dicke/Stärke von 1 mm bis 3 cm, bevorzugt von 1 mm bis 10 mm, insbesondere von 3 mm bis 9 mm, bevorzugt von 4 mm bis 7 mm, aufweist wobei das Flächengebilde waschbar und desinfizierbar ausgebildet ist.

2. Flächengebilde nach Anspruch 1, **dadurch gekennzeichnet, dass** das Vlies die Dicke/Stärke des Vlieses im Wesentlichen durchdringende, insbesondere durch eine mechanische Nachbearbeitung des Vlieses, insbesondere durch Vernadeln, entstandene Verfestigungsfasern aufweist und/oder das Flächengebilde **(11)** eine oder mehrere Vliesschichten umfasst und/oder eine mechanische, chemische oder thermische Vernetzung und/oder Vernadelung der einzelnen Fasern **(14)** beziehungsweise Filamente des Vlieses untereinander und/oder das Vlies als Nano- oder Mikrofaservlies und/oder aus Fasern **(14)** oder Filamenten ausgebildet ist.

3. Flächengebilde nach einem oder mehreren der vorhergehenden Ansprüche, **dadurch gekennzeichnet, dass** die superabsorbierende Aktivierung dauerhaft, abriebresistent, mechanisch stabil mit dem Flächengebilde (**11**), dem Vlies und/oder den Fasern **(14)** oder Filamenten verbunden ist, beziehungsweise die Fasern oder Filamente ganz oder teilweise aus Superabsorber Polymer (SAP) bestehen und/oder das Vlies und/oder die Fasern **(14)** oder Filamente eine Beaufschlagung mit wenigstens einem superabsorbierenden Aktivierungsmittel aufweist/en, wobei der Anteil des beaufschlagten superabsorbierenden Aktivierungsmittels am Vlies und/oder an den Fasern **(14)** oder Filamenten zwischen 0 und 800 Massenprozent bezogen auf die flächenbezogene Masse des Vlieses beträgt und/oder zur Verbindung des Vlieses und/oder der Fasern **(14)** oder Filamenten mit dem superabsorbierenden Aktivierungsmittel die Beaufschlagung mit einem Klebemittel, insbesondere auf Basis von Polyacrylat, vorgesehen ist, wobei der Anteil des beaufschlagten Klebemittels am Vlies zwischen 0 und 85 Massenprozent bezogen auf die flächenbezogene Masse des Vlieses beträgt und/oder das Vlies und/oder die Oberfläche **(16, 17)** der Fasern **(14)** oder Filamente eine Beschichtung mit dem superabsorbierenden Aktivierungsmittel und/oder dem Klebemittel aufweist/en

4. Flächengebilde nach einem oder mehreren der vorhergehenden Ansprüche, **dadurch gekennzeichnet, dass** die Fasern **(14)** oder Filamente eine Garnfeinheit von 0,1 bis 20 dtex [Gramm/10000 m] aufweisen und/oder die Fasern **(14)** oder Filamente aus natürlichen Polymeren, insbesondere auf Basis von Baumwolle, Bambus, Hanf, Cellulose, mineralischen Fasern **(14)** oder aus künstlichen Polymeren, insbesondere aus thermoplastischen Polymeren, bevorzugt Polyethylen, Polyamid, Polyester oder Polypropylen, oder Polyurethan, Polyvinylchlorid, Kunstseide-Acrylfasern oder aus biotechnologisch erzeugten Polymeren und/oder Mischungen und/oder Abfallfasern der vorgenannten Polymere gefertigt sind und/oder das Vlies aus einer Mischung verschiedener Fasern **(14),** Filamente oder Fasergruppen gebildet ist, wobei die Mischung 25-100 Polymerfasern, 0-20 Abfallfasern und/oder 0-10 niedrigschmelzende Polymerfasern umfasst und/oder die Fasern, Filamente oder Fasergruppen Faser- oder Filamentanteile mit unterschiedlichen Garnfeinheiten von 0,1 bis 20 dtex aufweisen und/oder die Fasern **(14)** oder Filamente aus Bikomponentenfasern die insbesondere aus einer Mischung von zwei Polymeren unterschiedlicher Schmelztemperaturen, insbesondere ausgewählt aus der Gruppe bestehend aus Polyethylen, Polyamid, Polyester, Polyurethan und Polypropylen, gebildet sind und/oder die Fasern **(14)** oder Filamente durch Trockenspinnen, Nassspinnen, Schmelzspinnen, Matrixspinnen oder Elektrospinnen gebildet sind

5. Flächengebilde nach einem oder mehreren der vorhergehenden Ansprüche, **dadurch gekennzeichnet, dass** eine superabsorbierende Aktivierung des Flächengebildes **(11)** und/oder des Vlieses vor, während oder nach dem Spinnvorgang erfolgt und/oder die superabsorbierende Aktivierung durch wenigstens ein auf oder in die Fasern **(14)** oder Filamente und/oder in und/oder auf das Vlies auf- oder eingebrachtes superabsorbierendes Polymer erfolgt und/oder dass die Fasern **(14)** oder Filamente ganz oder teilweise aus wenigstens einem superabsorbierenden Polymer gebildet sind und/oder das superabsorbierende Polymer in Form von Partikeln **(15)** vorliegt, wobei die Partikel **(15)** Durchmesser zwischen 45 und 875 µm, insbesondere zwischen 326 und 674 µm, bevorzugt zwischen 400 und 500 µm aufweisen oder die Partikel bevorzugt einen Durchmesser zwischen 50 und 150 µm aufweisen und/oder Mischungen von Partikeln **(15)** mit verschiedenen Durchmessern vorgesehen sind und/oder die Partikel **(15)** kubisch, stäbchenförmig, polyedrisch, kugelig, abgerundet, winkelig, nadelartig, flocken- oder faserförmig o. dgl., insbesondere als Pulver, Kügelchen, Flocken oder Fasern **(14)** oder als Molekül-/Partikelketten ausgeformt sind und/oder das superabsorbierende Polymer ein chemisch vernetztes Copolymer aus Acrylsäure und einem ihrer Salze, insbesondere Natriumacrylat, ist und/oder die Verbindung von superabsorbierendem Polymer und Vlies und/oder Fasern **(14)** oder Filamenten vor der chemischen Vernetzung des Copolymers erfolgt und/oder das superabsorbierende Polymer und/oder die Partikel **(15)** die Fasern **(14)** oder Filamente umlagern, um- bzw. einschließen und/oder beschichten und/oder das Vlies, insbesondere das Nano- oder Mikrofaservlies, als Wirr-, Spinn- und/oder Stapelfaservlies ausgebildet ist und/oder die Partikel **(15)** durch sich kreuzende und/oder im Wesentlichen parallel ausgerichtete Fasern **(14)** oder Filamente im Vlies und/oder im Flächengebilde **(11)** gehalten, insbesondere eingesponnen, sind und/oder die Strukturierung oder Beschichtung der Oberfläche der Partikel **(15)** und/oder der Fasern **(14)** oder Filamente nach Art einer eine Adhäsionswirkung entfaltenden Oberflächenstrukturierung oder -beschichtung, insbesondere Nanostrukturierung oder -beschichtung, ausgebildet ist

6. Flächengebilde nach einem oder mehreren der vorhergehenden Ansprüche, **dadurch gekennzeichnet, dass** wenigstens eine Oberfläche **(16, 17)** des Flächengebildes **(11)** aus von dem Flächengebilde **(11)** abweichenden oder aus dem gleichen Material gebildet ist und/oder Eigenschaften aufweist die von den übrigen Eigenschaften des Flächengebildes **(11)** abweichen und/oder die Oberfläche **(16, 17)** durch mechanische, thermische und/oder chemische Nachbehandlung, insbesondere Vernadelung des Vlieses gebildet ist und/oder die Oberfläche **(16, 17)** durch ein nicht superabsorbierend aktiviertes Vlies, eine Membran, eine Folie, einen Stoff, eine Stoffbahn, eine Beschichtung oder eine Laminierung gebildet ist, insbesondere wobei die Oberfläche **(16, 17)** fest oder lösbar mit dem übrigen Material des Flächengebildes **(11)** verbunden ist und/oder die Oberfläche **(16, 17)** durch An- oder Verschmelzen der Fasern **(14)** oder Filamente, insbesondere in Verbindung mit der Vernadelung, gebildet ist

7. Flächengebilde nach einem oder mehreren der vorhergehenden Ansprüche, **dadurch gekennzeichnet, dass** die Oberfläche **(16, 17)** wenigstens bereichsweise permeabel ausgebildet ist und Poren für den Durchtritt von Flüssigkeiten und/oder Gasen, insbesondere von Wasser, Alkohol und/oder Wasser-/Alkoholdampf oder Gemischen daraus, aufweist, insbesondere wobei die Porengröße kleiner ausgebildet ist als der Minimaldurchmesser der Partikel **(15)** und/oder eine Kompartimentierung des Flächengebildes **(11)** vorgesehen ist, wobei die Kompartimentierung durch Einsteppen, Abnähen, Abkleben oder Schweißen der Oberflächen **(16, 17)** des Flächengebildes **(11)** erfolgt und/oder wenigstens eine Oberfläche **(16, 17)** eine zusätzliche Aktivierung, insbesondere eine flüssigkeitsdichte, flammfeste, feuerfeste, funkenflugresistente, gegenüber Metall- oder Glutspritzern resistente, flüssiges Metall abweisende, selbstreinigende, schmutzabweisende, biozide, antivirale, UV-blockende, antistatische, repellente, kosmetisch wirksame, medizinisch wirksame, hydrophilisierende, antimikrobielle, antibakterielle, antibiotische oder elektromagnetische Strahlung absorbierende beziehungsweise abschirmende Beschichtung beziehungsweise Ausrüstung aufweist.

8. Flächengebilde nach einem oder mehreren der vorhergehenden Ansprüche, **dadurch gekennzeichnet, dass** das Flächengebilde **(11)** für eine mehrfache Benutzung vorgesehen ist, wobei das Flächengebilde **(11)** waschbar, insbesondere maschinell waschbar, chemisch reinigbar, desinfizierbar und/oder sterilisierbar ausgebildet ist.

9. Verfahren zur Herstellung eines Flächengebildes gemäß einem oder mehreren der vorhergehenden Ansprüche, umfassend die Schritte - Zurverfügungstellen von Fasern **(14)** oder Filamenten, - Auf- oder Einbringen von superabsorbierendem Polymer in, an oder auf die Fasern **(14)** oder Filamente vor, während oder nach dem Zurverfügungstellen, - Verbinden von Fasern **(14)** oder Filamenten und superabsorbierendem Polymer und/oder Vernetzen des superabsorbierenden Polymers, und - Bildung eines Flächengebildes **(11)** oder Vlieses aus Fasern **(14)** oder Filamenten.

10. Verfahren nach Anspruch 9, **dadurch gekennzeichnet, dass** das Verbinden und/oder das Vernetzen mechanisch und/oder chemisch, insbesondere durch ein Klebe- oder Vernetzungsmittel erfolgt und/oder die Fasern **(14)** oder Filamente durch Trockenspinnen, Nassspinnen, Schmelzspinnen, Matrixspinnen oder Elektrospinnen gebildet sind und eine Verbindung von Fasern **(14)** oder Filamenten und/oder einem Ausgangsmaterial für die Fasern **(14)** oder Filamente und superabsorbierendem Polymer vor, während oder nach dem Spinnvorgang erfolgt und/oder das die Fasern **(14)** oder Filamente teilweise oder vollständig aus dem superabsorbierenden Polymer gebildet sind und/oder das superabsorbierende Polymer nach der Bildung des Flächengebildes **(11)** oder Vlieses auf dieses aufgetragen wird und vorher, während oder anschließend eine chemische Vernetzung und/oder mechanische Verbindung des superabsorbierenden Polymers mit dem Flächengebilde **(11)** oder dem Vlies und/oder eine Vernetzung des superabsorbierenden Polymers erfolgt.

11. Umhüllung mit zwei oder mehr Lagen, umfassend wenigstens eine aus einem textilen Flächengebilde **(11)** gemäß einem oder mehreren der vorhergehenden Ansprüche 1 bis 8 gebildeten Lage.

12. Umhüllung nach Anspruch 11, **dadurch gekennzeichnet, dass** zumindest eine Lage flächig an das Flächengebilde anschließt oder sich das Flächengebilde zwischen zwei Lagen befindet und/oder die Lage aus einem Stoffgewebe oder einem Textil gebildet ist, und/oder die Lage als Membran oder Folie, jeweils natürlichen oder künstlichen Ursprungs, ausgebildet ist, oder die Lage aus einer Kombination oder in einer Sandwichbauweise aus einem oder mehreren Stoffgeweben und/oder einer oder mehreren Membranen oder Folien, jeweils natürlichen oder künstlichen Ursprungs, besteht, und/oder die Lage aus einem Mikrofaserstoffgewebe besteht, und/oder die Lage aus einem Metallfäden und/oder Funktionsfasern, beispielsweise Kokos-, Carbon-, oder mineralischen Fasern aufweisenden Stoffgewebe oder Textil, jeweils natürlichen oder künstlichen Ursprungs, besteht und/oder die Lage eine elektromagnetische Strahlung abschirmende oder absorbierende Ausstattung aufweist und/oder das Flächengebilde **(11)** eine oder mehrere Membranen **(19a, 19b, 19c)** aufweist und/oder die Umhüllung **(10)** als Textilmaterial, Funktionstextil, kühlendes Funktionselement oder als eines der nachfolgend genannten Gegenstände ausgebildet ist und/oder welches insbesondere an einem Bekleidungsstück oder mit einem anderen insbesondere nachfolgend genannten Gegenstand verbindbar, an diesem anordenbar oder in diesen einsteckbar ausgebildet ist: Bekleidungsstück, Kleidungsaccessoire, Schutzkleidung, Futter (lösbar oder fest) für Bekleidungsstücke (wie insbesondere Jacke, Anzüge, Hosen, Overalls, Schürzen), Plane, Decke, Decken- bzw. Dachkonstruktion, Medizinprodukt, OP-Decke, Pulskühler, Halsband, Kühlband, Kühltasche, Bandage, Hosenträger, Fuß- und Gelenkbandage, Orthese, Gesichtsmaske, Augenmaske, Strumpf, Strumpfhose, Kopfbedeckung, Helm, Schuh oder Stiefel, Abdeckung, insbesondere Fahrzeug-, Schiffs-, Flugzeug-, Gebäude-, Maschinen-, Bodenflächen-, Oberflächen- oder Geräteabdeckung, Markise, Zeltstoff, Sonnendach, Abdeckplane bzw. Abdeckschutzplane oder Ausrüstungsgegenstand zum Schutz vor hohen Temperaturen, Tasche, Beutel, Blutbeutel, Flaschen- oder Ampullenkühlung, Medikamentenschutztasche, Aufbewahrungsgefäß zum Schutz von biologischen oder humanen Komponenten und/oder Stoffen und/oder pharmazeutischen Wirkstoffen, Produkten und/oder Substanzen gegenüber hohen Temperaturen, Rucksack bzw. anderes Sport- oder Outdoorprodukt, Sporttrikot, Sportbekleidung, Unterwäsche, Aufbewahrung, Transportschutz, Tierkühlungsprodukt oder dergleichen und/oder die Verbindung über ein oder mehrere Verbindungsmittel, insbesondere wenigstens einen Knopf, Druckknopf, Klettverschluss, eine Schnalle, einen Steckverschluss, einen Reißverschluss, ein mehrfach verwendbares Haftmittel, einen haftvermittelnden nanostrukturenaufweisenden Bereich oder eine Clipsverbindung herstellbar ist.

13. Umhüllung nach einem oder beiden der Ansprüche 11 und 12 **dadurch gekennzeichnet, dass** die Umhüllung **(10)** waschbar, waschmaschinenwaschbar, chemisch reinigbar, sterilisierbar und/oder desinfizierbar ist, die Umhüllung **(10)** nach Beladung mit Flüssigkeit, insbesondere Wasser oder einem Wassergemisch einen Kühl- oder Klimaeffekt aufweist und/oder die Umhüllung **(10)** nach oder durch Beladung mit Flüssigkeit, insbesondere Wasser oder einem Gemisch mit Wasser gekühlt wird.

14. Umhüllung nach einem oder mehreren der Ansprüche 11 bis 13, **dadurch gekennzeichnet, dass** die Umhüllung **(10)** mit einer Beladung mit einem Gemisch aus Flüssigkeit und einer die flüssigkeitsdichte, flammfeste, feuer feste, selbstreinigende, schmutzabweisende, biozide, antivirale, UV-blockende, antistatische, funkenflugresistente, gegenüber Metall- oder Glutspritzern resistente, flüssiges Metall abweisende, repellente, hydrophilisierende, kosmetisch wirksame, medizinisch wirksame, antimikrobielle, antibakterielle, antibiotische und/oder elektromagnetische Strahlung absorbierende oder abschirmenden Beschichtung beziehungsweise Ausrüstung erneuernden, auffrischenden oder erstellenden Substanz erfolgt und/oder die Beladung mit einem Gemisch aus Alkohol und Wasser als Flüssigkeit erfolgt, insbesondere wobei das Gemisch zwischen 1 und 70 Vol.-%, bevorzugt zwischen 5 und 50 Vol.%, insbesondere zwischen 10 und 30 Vol.% Alkohol und zwischen 30 und 99 Vol.-% Wasser enthält und wobei bevorzugt der Alkohol ausgewählt ist aus der Gruppe bestehend aus Ethanol, Isopropanol oder Mischungen daraus und/oder das die Flüssigkeit beziehungsweise das Alkohol-Wasser-Gemisch eine Beimischung wenigstens eines Zusatzstoffes, von wenigstens einem ätherischen Öl, eines Duftstoffes, eines Aromastoffes, eines Repellens oder eines Vergällungsmittels oder von Mischungen daraus aufweist, wobei der Anteil des Zusatzstoffes im Bereich von 0,1 bis 5 Vol.% liegt.

15. Umhüllung nach einem oder mehreren der Ansprüche 11 bis 14, **dadurch gekennzeichnet, dass** und/oder zusätzlich oder alternativ zum Alkohol- oder Wasseranteil des Alkohol-Wasser-Gemisches die Beimischung einer Substanz zur Erneuerung, Auffrischung oder Erstellung der flüssigkeitsdichten, flammfesten, feuerfesten, funkenflugresistenten, gegen Metall- oder Glutspritzer resistenten, flüssiges Metall abweisenden, selbstreinigenden, schmutzabweisenden, bioziden, antiviralen, UVblockenden, antistatischen, repellenten, kosmetisch wirksamen, medizinisch wirksamen, hydrophilisierenden, antimikrobiellen, antibakteriellen, antibiotischen oder elektromagnetische Strahlung absorbierenden oder abweisenden Beschichtung beziehungsweise Ausrüstung vorgesehen ist und/oder das Wasser ausgewählt ist aus der Gruppe bestehend aus Leitungswasser, mineralisiertem Wasser, destilliertem Wasser, demineralisiertem Wasser oder Reinstwasser, wobei bevorzugt das Gemisch aus Alkohol und Wasser und/oder wenigstens einem Zusatzstoff als Konzentrat vorgehalten wird und vor der Beladung der Umhüllung durch Zugabe von Wasser auf eine Endkonzentration verdünnt wird und/oder das Gemisch eine Beimischung von bioziden, fungiziden, viruziden, antimikrobiellen, kosmetischen oder medizinischen und/oder antibiotischen Substanzen, insbesondere einer Silberlösung, aufweist, wobei die Beimischung bei einem Anteil von 0,1 bis 5 Vol.% liegt.

16. Umhüllung nach einem oder mehreren der Ansprüche 11 bis 15, **dadurch gekennzeichnet, dass** die Umhüllung **(10)** zur oder nach der Vorkühlung in fest installierten oder tragbaren Kühlbehältern aufbewahrt oder transportiert wird und/oder in Kombination mit Kaltluft oder in Kühlkammern verwendet wird und/oder wenigstens ein Teil der Umhüllung (10) und/oder wenigstens eine Lage aus feuer-, funkenflug- oder flammresistentem, flüssiges Metall abweisendem und/oder gegenüber Metall- oder Glutspritzern resistentem, flüssigkeitsdichtem, selbstreinigendem, schmutzabweisendem, biozidem, antimikrobiellem, antiviralem, antibakteriellem, antibiotischem, UV-blockendem, repellentem, kosmetisch und/oder medizinisch wirksamem, hydrophilisierendem, elektromagnetische Strahlung absorbierendem oder abschirmendem und/oder gummiartigem Material gebildet ist oder eine feuer-, funkenflug- oder flammresistente, flüssiges Metall abweisende und/oder gegenüber Metall- oder Glutspritzern resistente, flüssigkeitsdichte, selbstreinigende, schmutzabweisende, biozide, antimikrobielle, antivirale, antibakterielle, antibiotische, UV-blockend, repellente, kosmetisch oder medizinisch wirksame, hydrophilisierende, elektromagnetische Strahlung absorbierende oder abschirmende und/oder gummiartige Beschichtung, Ausrüstung oder Aktivierung aufweist

17. Umhüllung nach einem oder mehreren der Ansprüche 11 bis 16, **dadurch gekennzeichnet, dass** in einer oder mehreren der Lagen Kanäle zur Belüftung oder zum Verteilen der Flüssigkeit vorgesehen sind, wobei bevorzugt die Kanäle manuell verschließbar und/oder automatisch schließend ausgebildet sind und/oder zur Aufnahme und/oder Abgabe von Flüssigkeit Mikropumpen vorgesehen sind und/oder dass die Aufnahme und/oder Abgabe von Flüssigkeit osmotisch erfolgt und/oder an oder in der Umhüllung (10) ein Flüssigkeitsreservoir, insbesondere eine Wanne, ein Kanister, eine Flasche o. dgl. zur Aufnahme oder Abgabe von Flüssigkeit vorgesehen ist und dem Flüssigkeitsreservoir ein die Umhüllung **(10)** und/oder das Flächengebilde **(11)** durchziehendes Schlauchsystem für die Flüssigkeit zugeordnet ist

18. Umhüllung nach einem oder mehreren der Ansprüche 11 bis 17, **dadurch gekennzeichnet, dass** wenigstens ein Flächengebilde **(11)** an oder in der Umhüllung (10) fest, insbesondere durch Verkleben, Verschweißen oder Vernähen oder lösbar, insbesondere durch ein Verbindungsmittel verbindbar, angeordnet ist und/oder die Umhüllung **(10)** wenigstens bereichsweise permeabel ausgebildet ist und Poren für den Durchtritt von Flüssigkeit und/oder Gas, insbesondere von Wasser, Alkohol- und/oder Wasser-/Alkoholdampf oder Gemischen daraus aufweist, wobei die Porengröße kleiner ausgebildet ist, als der Minimaldurchmesser der Partikel **(15)** und/oder eine Kompartimentierung der Umhüllung **(10)** vorgesehen ist, wobei die Kompartimentierung durch Einsteppen, Abnähen, Abkleben oder Schweißen der Oberflächen **(16,17)** beziehungsweise Lagen der Umhüllung **(10)** erfolgt und/oder wenigstens eine Lage eine zusätzliche Aktivierung, insbesondere eine flüssigkeitsdichte, flammfeste, feuerfeste, funkenflugresistente, gegenüber Metall- oder Glutspritzern resistente, flüssiges Metall abweisende, selbstreinigende, schmutzabweisende, biozide, antivirale, UV-blockende, antistatische, repellente, kosmetisch wirksame, medizinisch wirksame, hydrophilisierende, antimikrobielle, antibakterielle, antibiotische und/oder elektromagnetische Strahlung absorbierende oder abschirmende Beschichtung, Ausrüstung beziehungsweise Aktivierung aufweist.

19. Kühlsystem, insbesondere für den Einsatz im Rahmen einer kontrollierten und überwachbaren Kühlung von Menschen, Tieren und/oder Gegenständen umfassend eine Umhüllung (10) gemäß einem oder mehreren der vorhergehenden Ansprüche 11 bis 18.

20. Kühlsystem nach Anspruch 19, **dadurch gekennzeichnet, dass** das Kühlsystem einen Aufbewahrungsbehälter umfasst, welcher die mit Flüssigkeit beladene Umhüllung aufnimmt und/oder der Aufbewahrungsbehälter als insbesondere evakuierbare, verschweißbare Kunststoffverpackung oder als wiederverschließbarer Behälter, insbesondere aus Kunststoff oder Metall ausgebildet ist und/oder der Aufbewahrungsbehälter auch weitere Flüssigkeit für das wiederholte Beladen und Aktivieren der Umhüllung aufnimmt, bereitstellt oder weitergibt und/oder der Aufbewahrungsbehälter eine antimikrobielle Beschichtung zumindest eines Teiles seiner der Umhüllung zugewandten Flächen aufweist und/oder der Inhalt des Aufbewahrungsbehälters desinfizierbar und/oder sterilisierbar ist beziehungsweise eine sterile Umhüllung darin, insbesondere mit beladenem Flächengebilde, vorgehalten ist und/oder ein Sensor zur Erfassung der Außentemperatur und/oder der Umhüllungstemperatur vorgesehen ist, insbesondere wobei dem Sensor eine Temperaturanzeige zugeordnet ist und/oder der Sensor in Form einer Thermofarbenbeschichtung oder eines Thermometers ausgebildet ist und/oder wenigstens ein Sensor zur Erfassung von Klimafaktoren in der Umgebung des Kühlsystems, insbesondere zur Erfassung von Umgebungstemperaturen, relativer Luftfeuchte und/oder Luftströmungen vorgesehen ist.

21. Verwendung eines Gemisches aus Alkohol und Wasser und/oder eines Gemisches aus Wasser, Alkohol und/oder einer die Ausrüstung der Umhüllung und/oder des Flächengebildes erneuernden, auffrischenden und/oder erstellenden Substanz zur Beladung einer Umhüllung nach einem oder mehreren der Ansprüche 11 bis 18 und/oder in einem Kühlsystem nach einem oder beiden der Ansprüche 19 und 20.

22. Verwendung nach Anspruch 21, **dadurch gekennzeichnet, dass** das Gemisch auf die Umhüllung **(10)** aufgebracht oder aufgesprüht und/oder in die Umhüllung **(10)** und/oder ein in der Umhüllung **(10)** vorgesehenes Flächengebilde **(11)** eingefüllt oder -geladen wird und/oder die Umhüllung **(10)** und/oder ein in der Umhüllung **(10)** vorgesehenes Flächengebilde **(11)** in das Gemisch eingetaucht wird, wobei bevorzugt das Gemisch zwischen 1 und 70 Vol.-%, bevorzugt zwischen 5 und 50 Vol.-%, insbesondere zwischen 10 und 30 Vol.% Alkohol und/oder der die Ausrüstung erneuernden, auffrischenden oder erstellenden Substanz und zwischen 30 und 99 Vol.% Wasser enthält und wobei insbesondere der Alkohol ausgewählt ist aus der Gruppe bestehend aus Ethanol, Isopropanol oder Mischungen daraus und/oder das Alkohol- und/oder -Substanz-Wasser-Gemisch eine Beimischung wenigstens eines Zusatzstoffes, insbesondere wenigstens eines ätherischen Öles, eines Duftstoffes, eines Aromastoffes eines Repellens oder eines Vergällungsmittels oder Mischungen daraus aufweist, wobei der Anteil des wenigstens einen Zusatzstoffes im Bereich von 0,1 bis 5 Vol.-% liegt und wobei das Wasser ausgewählt ist aus der Gruppe bestehend aus Leitungswasser, mineralisiertem Wasser, destilliertem Wasser, demineralisiertem Wasser oder Reinstwasser und/oder das Gemisch eine Beimischung von Bioziden, Fungiziden, Viruziden, Repellentien, antimikrobiellen und/oder antibiotischen Substanzen aufweist, wobei die Beimischung bei einem Anteil von 0,1 bis 5 Vol.-% liegt.

23. Kühlartikel, insbesondere Textilprodukt, bevorzugt flächiges Textilprodukt, vorzugsweise Kleidungsstück, insbesondere Oberbekleidungsstück, Kleidungsaccessoire, Hosenträger, Schutzkleidung, Plane, Decke, Decken- oder Dachkonstruktionen, Medizinprodukt, OP-Decke, Pulskühler, Halsband, Kühlband, Kühltasche, Bandage, Fuß- und Gelenkbandage, Orthese, Gesichtsmaske, Augenmaske, Strumpf, Strumpfhose, Kopfbedeckung, Helm, Schuh oder Stiefel, Abdeckung, insbesondere Gebäude-, Fahrzeug-, Flugzeug-, Schiffs-, Maschinen-, Bodenflächen- oder Oberflächen-, oder Geräteabdeckung, Markise, Zeltstoff, Sonnendach, Abdeckplane bzw. Abdeckschutzplane oder Ausrüstungsgegenstand zum Schutz vor hohen Temperaturen, Tasche, Beutel, Blutbeutel, Flaschen- oder Ampullenkühler, Medikamentenschutztasche, Aufbewahrungsgefäß zum Schutz von biologischen oder humanen Komponenten und/oder Stoffen sowie pharmazeutischen Wirkstoffen, Produkten, und/oder Substanzen gegenüber hohen Temperaturen, Rucksack bzw. anderes Sport- oder Outdoorprodukt, Sporttrikot, Sportbekleidung, Unterwäsche, Aufbewahrung, Transportschutz, Tierkühlungsprodukt oder dergleichen, aus dem eine Umhüllung **(10)** nach einem oder mehreren der vorhergehenden Ansprüche 11 bis 18 gebildet ist, **dadurch gekennzeichnet, dass** der Kühlartikel wenigstens einen Bereich mit einem Flächengebilde **(11)** nach einem oder mehreren der vorhergehenden Ansprüche 1 bis 10 aufweist und/oder **dadurch gekennzeichnet, dass** der Kühlartikel in wenigstens einem zweiten Bereich, insbesondere in einem abtrennbaren Kühlartikelteil angeordnet, wenigstens ein Flächengebilde **(11)** ohne oder mit geringerer Kühlwirkung insbesondere ohne superabsorbierendes Polymer oder mit einem verringerten Anteil an superabsorbierendem Polymer aufweist und wobei bevorzugt ein Kühlartikelbereich durch ein oder mehrere Verbindungsmittel, insbesondere wenigstens einen Knopf, Druckknopf, Klettverschluss, eine Schnalle, einen Steckverschluss, einen Reißverschluss, ein mehrfach verwendetes Haftmittel, einen haftungsvermittelnden Nanostrukturen aufweisenden Bereich oder einen, Clipsverschluss an dem ersten Bereich abtrennbar ausgebildet ist, anschließbar ist.

## Claims

1. Reusable textile fabric able to be charged with liquid and thereby activated, having a cooling effect on discharge, containing a fleece with super-absorbing activation, the dry and uncharged textile fabric (11) having a mass per unit area of between 100 and 1500 g/m², preferably between 100 and 500 g/m², and the fleece having a mass per unit area of between 50 and 120 g/m², in particular between 60 and 100 g/m², preferably between 50 and 80 g/m² and/or the fleece in the textile fabric (11) having a thickness of between 1 mm and 3 cm, preferably between 1 mm and 10 mm, in particular between 3 mm and 9 mm, preferably between 4 mm and 7 mm, the textile fabric being designed to be washable and disinfectable.

2. Textile fabric according to claim 1, **characterised in that** the fleece comprises reinforcing fibres penetrating through the thickness of the fleece, in particular produced by mechanical reworking of the fleece, especially by needling, and/or the textile fabric **(11)** comprises one or more layers of fleece and/or is formed by mechanical, chemical or thermal cross-linking and/or needling of the individual fibres **(14)** or filaments of the fleece to each other and/or the fleece is formed from nanofibre or microfibre fleece and/or from fibres **(14)** or filaments.

3. Textile fabric according to one or more of the previous claims, **characterised in that** the super-absorbent activation is permanently attached to the textile fabric **(11),** the fleece and/or the fibres **(14)** or filaments in a wear-resistant and mechanically stable manner, or the fibres or filaments consist entirely or partially of super-absorber polymer (SAP) and/or the fleece and/or the fibres **(14)** or filaments is/are treated with at least one super-absorbing activating agent, the percentage of the treating super-absorbing activating agent on the fleece and/or on the fibres **(14)** or filaments being between 0 and 800 weight percent related to the mass per unit area of the fleece and/or the treatment being provided with an adhesive to bond the fleece and/or the fibres **(14)** or filaments with the super-absorbing activating agent, in particular a polyacrylate-based adhesive, the percentage of the treating adhesive on the fleece being between 0 and 85 weight % relative to the mass per unit area of the fleece and/or the fleece and/or the surface **(16, 17)** of the fibres **(14)** or filaments is/are coated with the super-absorbent activating agent and/or the adhesive.

4. Textile fabric according to one or more of the previous claims, **characterised in that** the fibres **(14)** or filaments have a yarn count of 0.1 to 20 dtex [grams/10000 m] and/or the fibres **(14)** or filaments are made from natural polymers, especially based on cotton, bamboo, hemp, cellulose, mineral fibres **(14)** or from artificial polymers, in particular thermoplastic polymers, preferably polyethylene, polyamide, polyester or polypropylene, or polyurethane, polyvinylchloride, artificial silk-acrylic fibres or from biotechnically produced polymers and/or mixtures and/or waste fibres of the above-mentioned polymers and/or the fleece is formed from a mixture of different fibres **(14),** filaments or fibre groups, the mixture comprising 25-100 polymer fibres, 0-20 waste fibres and/or 0-10 low-melting polymer fibres and/or the fibres, filaments or fibre groups comprising proportions of fibres or filaments with different yarn counts of between 0.1 and 20 dtex and/or the fibres **(14)** or filaments are formed from bicomponent fibres formed from a mixture of two polymers with different melting temperatures, in particular selected from the group comprising polyethylene, polyamide, polyester, polyurethane and polypropylene, and/or the fibres **(14)** or filaments being formed by dry spinning, wet spinning, melt spinning, matrix spinning or electro-spinning.

5. Textile fabric according to one or more of the previous claims, **characterised in that** a super-absorbent activation of the textile fabric **(11)** and/or the fleece takes place before, during or after the spinning process and/or the super-absorbent activation takes place by means of at least one super-absorbent polymer applied onto or into the fibres **(14)** or filaments and/or into and/or onto the fleece and/or the fibres **(14)** or filaments being formed wholly or partially from at least one super-absorbent polymer and/or the super-absorbent polymer takes the form of particles **(15),** the said particles **(15)** having a diameter of between 45 and 875 µm, in particular between 326 and 674 µm, preferably between 400 and 500 µm or the particles preferably having a diameter of between 50 and 150 µm and/or mixtures of particles **(15)** of different diameters being provided and/or the particles **(15)** being cubic, rodlike, polyhedral, spherical, rounded, angular, needle-like, flake or fibre-like in shape, or similar, especially in the form of powder, spheres, flakes or fibres **(14)** or as molecular/particle chains and/or the super-absorbent polymer is a chemically linked copolymer of acrylic acid or one of its salts, in particular sodium acrylate, and/or the combination of the super-absorbent polymer and the fleece and/or fibres **(14)** or filaments taking place before chemical cross-linking of the copolymer and/or the super-absorbent polymer and/or the particles **(15)** surround, enclose or include and/or coat the fibres **(14)** or filaments and/or the fleece, in particular the nanofibre or microfibre fleece, is designed as a random-fibre, non-woven and/or spun bonded fleece and/or the particles **(15)** being held in the fleece and/or textile fabric **(11)** by criss-crossing and/or substantially parallel fibres **(14)** or filaments, in particular spun in, and/or the structuring or coating of the surface of the particles **(15)** and/or the fibres **(14)** or filaments is formed by means of a surface structuring or coating, in particular nano-structuring or nano-coating with adhesive properties.

6. Textile fabric according to one or more of the previous claims, **characterised in that** at least one surface **(16, 17)** of the textile fabric **(11)** is formed from a different material than the textile fabric **(11)** or from the same material and/or has properties that differ from the normal properties of the textile fabric **(11)** and/or the surface **(16, 17)** is formed by mechanical, thermal and/or chemical reworking, especially needling, of the fleece and/or the surface **(16, 17)** is formed by a membrane, foil, textile fabric or textile fabric layer, a coating or lamination or fleece that has not been super-absorbently activated, in particular the surface **(16, 17)** being permanently or removably attached to the remaining material of the textile fabric **(11)** and/or the surface **(16, 17)** being formed by fusing on or melting the fibres **(14)** or filaments, in particular in combination with needling.

7. Textile fabric according to one or more of the previous claims, **characterised in that** the surface **(16, 17)** is permeable at least in some areas and comprises pores for the passage of liquids and/or gases, in particular water, alcohol and/or water/alcohol vapour or mixtures thereof, in particular the pore size being smaller than the minimum diameter of the particles **(15)** and/or the textile fabric **(11)** being compartmentalised by inserting lining, stitching down, sticking down or welding the surfaces **(16, 17)** of the textile fabric **(11)** and/or at least one surface **(16, 17)** having an additional activation, in particular a liquid-tight, flame resistant, fireproof, flying spark resistant, metal or molten spray resistant, liquid metal repellent, self-cleaning, dirt-repellent, biocidal, antiviral, UV-blocking, antistatic, repellent, cosmetically active, medicinally active, hydrophilic, antimicrobial, antibacterial, antibiotic or electromagnetic radiation absorbing all shielding coating of finishing.

8. Textile fabric according to one or more of the previous claims, **characterised in that** the textile fabric **(11)** is intended for multiple use, the textile fabric **(11)** being washable, especially machine washable, chemically cleanable, disinfectable and/or sterilisable.

9. Process for manufacturing a textile fabric according to one or more of the previous claims, comprising the steps - provision of fibres **(14)** or filaments, - application or injection of super-absorbent polymer in, at or on the fibres **(14)** or filaments before, during or after their provision, - bonding of fibres **(14)** or filaments and super-absorbent polymer and/or cross-linking of the super-absorbent polymers, and - formation of a textile fabric **(11)** or fleece from fibres **(14)** or filaments.

10. Process according to claim 9, **characterised in that** the combination and/or cross-linking takes place mechanically and/or chemically, in particular by means of an adhesive or cross-linking agent and/or the fibres **(14)** or filaments are formed by dry spinning, wet spinning, melt spinning, matrix spinning or electrospinning and the combination of fibres **(14)** or filaments and/or a source material for the fibres **(14)** or filaments and super-absorbent polymer takes place before, during or after the spinning process and/or the fibres **(14)** or filaments are formed wholly or partially from the super-absorbent polymer and/or the super-absorbent polymer is applied to the textile fabric **(11)** or fleece after it has been formed and chemical cross-linking and/or mechanical combination of the super-absorbent polymer with the textile fabric **(11)** or fleece and/or cross-linking the super-absorbent polymer takes place beforehand, during or afterwards.

11. Covering with two or more layers, comprising at least one layer formed from a textile fabric **(11)** according to one or more of the previous claims 1 to 8.

12. Covering according to claim 11, **characterised in that** at least one layer connects two-dimensionally to the textile fabric or the textile fabric is positioned between two layers and/or the layer is formed by a textile fabric or textile, and/or the layer is designed as a membrane or foil, of natural or synthetic origin respectively, or the layer consists of a combination or a sandwich construction comprising one or more fabrics and/or one or more membranes or foils, of natural or synthetic origin respectively, and/or the layer consists of a microfibre fabric, and/or the layer consists of a fabric or textile comprising metallic threads and/or functional fibres, for example coconut, carbon or mineral fibres, of natural or synthetic origin respectively, and/or the layer is equipped to screen or absorb electromagnetic radiation and/or the textile fabric **(11)** comprises one or more membranes **(19a, 19b, 19c)** and/or the covering **(10)** is designed as textile material, functional textile, cooling functional element or as one of the items listed below and/or which can be attached to, arranged on or inserted into an item of clothing or with another item, in particular those mentioned below: item of clothing, clothing accessory, protective clothing, lining (loose or fixed) for items of clothing (such as, in particular, jacket, suits, trousers, overalls, aprons), tarpaulin, covering, ceiling or roof construction, medical device, operating theatre ceiling, pulse cooler, neckband, cooling belt, cool bag, bandage, braces, foot and joint bandage, orthosis, face mask, eye mask, sock, tights, headgear, helmet, shoe or boot, cover, especially vehicle, ship, aeroplane, building, machine, floor, surface or equipment cover, awning, canvas, sunroof, tarpaulin cover or protective covering sheet or piece of equipment to protect against high temperatures, bag, pouch, blood bag, bottle or ampoule cooling, protective bag for medicines, storage vessel to protect against biological or human components and/or materials and/or to protect pharmaceutical products and/or substances against high temperatures, rucksack or other sports or outdoor item, sports jersey, sports clothing, underwear, storage item, transport protection, animal cooling product or similar and/or joining via one or more joining means, in particular at least a button, press-stud" Velcro fastener, buckle, clasp, zip fastener, a reusable bonding agent, an adhesive nanostructured area or a clip.

13. Covering according to one or both of claims 11 and 12, **characterised in that** the covering **(10)** is washable, machine washable, chemically cleanable, sterilisable and/or disinfectable and, after charging with liquid, in particular water or a water mixture, the covering **(10)** provides a cooling or climatising effect and/or the covering **(10)** is chilled after or by charging with liquid, in particular water or a water mixture.

14. Covering according to one or more of claims 11 to 13, **characterised in that** the covering (10) charged with a mixture of liquid and a substance to restore, refresh or create the liquid-tight, flame resistant, fireproof, flying spark resistant, metal or molten spray resistant, liquid metal repellent, self-cleaning, dirt-repellent, biocidal, antiviral, UV-blocking, antistatic, repellent, cosmetically active, medicinally active, hydrophilic, antimicrobial, antibacterial, antibiotic or electromagnetic radiation absorbing or shielding coating or finishing and/or the covering is charged with a mixture of alcohol and water as a liquid, in particular the mixture containing between 1 and 70 vol.-%, preferably between 5 and 50 vol.-%, in particular between 10 and 30 vol.-% alcohol and between 30 and 99 vol.-% water and the alcohol preferably being selected from the group comprising ethanol, isopropanol or mixtures thereof and/or the liquid or the alcohol-water mixture containing an addition of at least one additive, in particular at least an essential oil, a fragrance, an aromatic, a repellent or a denaturant or mixtures thereof, the proportion of the additive being in the range between 0.1 and 5 vol.-%.

15. Covering according to one or more of claims 11 to 14, **characterised in that**, additionally or alternatively to the alcohol or water proportion of the alcohol-water mixture, the admixture of a substance to renew, refresh or create the liquid-tight, flame-resistant, fireproof, flying spark resistant, metal or molten spray resistant, liquid metal repellent, self-cleaning, dirt-repellent, biocidal, antiviral, UV-blocking, antistatic, repellent, cosmetically active, medicinally active, hydrophilic, antimicrobial, antibacterial, antibiotic or electromagnetic radiation absorbing or shielding coating or finishing is provided and/or the water is selected from the group comprising tap water, mineral water, distilled water, demineralised water or ultrapure water, the mixture of alcohol and water and/or at least one additive preferably being provided as a concentrate and being diluted by the addition of water to a final concentration before charging of the covering and/or the mixture comprising an addition of biocidal, fungicidal, virucidal, antimicrobial, cosmetic or medicinal and/or antibiotic substances, in particular a silver solution, the admixture being in a proportion of 0.1 to 5 vol.-%.

16. Covering according to one or more of claims 11 to 15, **characterised in that** the covering **(10)** is kept or transported in fixed or portable cooling containers for or after cooling and/or used in combination with cold air or in refrigeration chambers and/or at least one part of the covering **(10)** and/or at least one layer is formed from fire-resistant, flying spark resistant or flame resistant, liquid metal repellent and/or metal or molten spray resistant, liquid-tight self-cleaning, dirt-repellent, biocidal, antimicrobial, antiviral, antibacterial, antibiotic, UV-blocking, repellent, cosmetically or medicinally active, hydrophilic, electromagnetic radiation absorbing or shielding and/or rubber-like material or comprises a fire-resistant, flying spark resistant or flame resistant, liquid metal repellent and/or metal or molten spray resistant, liquid-tight, self-cleaning, dirt-resistant, biocidal, antimicrobial, antiviral, antibacterial, antibiotic, UV-blocking, repellent, cosmetically or medicinally active, hydrophilic, electromagnetic radiation absorbing or shielding and/or rubber-like coating, finishing or activation

17. Covering according to one or more of claims 11 to 16, **characterised in that** channels are provided in one or more layers for ventilation or to distribute the liquid, the channels preferably being designed so that they can be closed by hand and/or close automatically and/or micro-pumps are provided to incorporate and/or discharge liquid and/or the incorporation and/or discharge of liquid takes place osmotically and/or a liquid reservoir, in particular a trough, canister, a bottle or similar is provided on or in the covering **(10)** to incorporate or discharge liquid and a tube system running through the covering **(10)** and/or the textile fabric **(11)** is provided for the liquid and associated with the liquid reservoir.

18. Covering according to one or more of claims 11 to 17, **characterised in that** at least one textile fabric **(11)** is arranged on or in the covering **(10),** either firmly attached, in particular by glueing, welding or sewing or removable, in particular by a connecting means and/or the covering **(10)** is designed so that it is permeable at least in some areas and comprises pores to allow the passage of liquids and/or gas, in particular water, alcohol vapour and/or water/alcohol vapour or mixtures thereof, the pore size being designed to be smaller than the minimum diameter of the particles **(15)** and/or compartmentalisation of the covering **(10)** being provided, the compartmentalisation being achieved by inserting a lining, stitching down, sticking down all welding the surfaces **(16,17)** or layers of the covering **(10)** and/or at least one layer comprising an additional activation, in particular a liquid-tight, flame-resistant, fireproof, flying spark resistant, metal or molten spray repellent, liquid metal repellent, self-cleaning, dirt-resistant, biocidal, antiviral, UV-blocking, antistatic, repellent, cosmetically active or medicinally active, hydrophilic, antimicrobial, antibacterial, antibiotic and/or electromagnetic radiation absorbing or screening finishing or activation.

19. Cooling system, in particular for use in connection with the controlled and monitored cooling of people, animals and/or objects comprising a covering **(10)** according to one or more of the previous claims 11 to 18.

20. Cooling system according to claim 19, **characterised in that** the cooling system comprises a storage container, which houses the covering charged with liquid and/or the storage container is designed in particular as an evacuable, weldable plastic packaging or as a resealable container, particularly made of plastic or metal and/or **in that** the storage container also carries, provides or reproduces additional liquid for the repeated charging and activation of the covering and/or the storage container comprises an antimicrobial coating at least of part of its surface facing the covering and/or the contents of the storage container is disinfectable and/or sterilisable or contains a covering, in particular with charged textile fabric, and/or a sensor is provided to record the outside temperature and/or the covering temperature, in particular the sensor being associated with a temperature display and/or the sensor being designed as a temperature-reactive colour coating or a thermometer and/or at least one sensor being provided to record climatic factors in the surroundings of the cooling system, in particular to record ambient temperatures, relative atmospheric humidity and/or air streams.

21. Use of a mixture of alcohol and water and/or or a mixture of water, alcohol and/or a substance for repairing, refreshing or and/or preparing the finishing of the covering and/or the textile fabric to charge a covering according to one or more of claims 11 to 18 and/or in a cooling system according to one or both of claims 19 and 20.

22. Use according to claim 21, **characterised in that** the mixture is applied or sprayed onto the covering **(10)** and/or into the covering **(10)** and/or a textile fabric **(11)** is filled into or charged into the covering **(10)** and/or a fabric **(11)** provided in the covering **(10)** is immersed in the mixture, the mixture preferably containing between 1 and 70 vol.-%, preferably between 5 and 50 vol.-%, in particular between 10 and 30 vol.-% alcohol and/or the substance restoring, refreshing or creating the finishing, and between 30 and 99 vol.-% water, and the alcohol in particular being selected from the group comprising ethanol, isopropanol or mixtures thereof and/or alcohol and/or substance-water mixture having an addition of at least one additive, in particular at least an essential oil, a fragrance, an aromatic, a repellent or a denaturant or mixtures thereof, the proportion of the minimum of one additive being in the range between 0.1 and 5 vol.-% and the water being selected from the group comprising tap water, mineral water, distilled water, demineralised water or ultrapure water and/or the mixture having an addition of biocidal, fungicidal, virucidal, repellent, antimicrobial and/or antibiotic substances, the admixture being in a proportion of between 0.1 and 5 vol.-%.

23. Cooling item, in particular a textile product, preferably an extensive textile product, preferably a clothing item, in particular an item of top clothing, clothing accessory, braces, protective clothing, tarpaulin, covering, ceiling or roof construction, medical device, operating theatre ceiling, pulse cooler, neckband, cooling belt, cool bag, bandage, foot and joint bandage, orthosis, face mask, eye mask, sock, tights, headgear, helmet, shoe or boot, cover, especially vehicle, ship, aeroplane, machine, floor, surface or equipment cover, awning, canvas, sunroof, tarpaulin cover or protective covering sheet or piece of equipment to protect against high temperatures, bag, pouch, blood bag, bottle or ampoule cooler, protective bag for medicines, storage vessel to protect against biological or human components and/or materials and/or pharmaceuticals, products and/or substances to protect against high temperatures, rucksack or other sports or outdoor item, sports jersey, sports clothing, underwear, storage item, transport protection, animal cooling item or similar, from which a covering **(10)** according to one or more of the previous claims 11 to 18 is formed, **characterised in that** the cooling item comprises at least one area with a textile fabric **(11)** according to one or more of the previous claims 1 to 10 and/or **characterised in that** the cooling article is arranged in at least a second area, in particular in a removable part of the cooling item , at least one textile fabric **(11)** with or without a slight cooling effect, in particular without super-absorbent polymer or with a small proportion of super-absorbent polymer and an area of the cooling item preferably being attachable to the first area by means of one or more attachment means, in particular at least a button, press-stud, Velcro fastener, buckle, clasp, zip fastener, a reusable bonding means, an area with adhesive nanostructure or a clip, so that it is detachable.

## Revendications

1. Structure plate réutilisable, pouvant être chargée avec du liquide et activable par ce biais, refroidissante lors du déchargement, composée d'un non-tissé présentant une activation superabsorbante, structure plate **(11)** présentant à l'état sec et non chargé une masse surfacique comprise entre 100 et 1 500 g/m², de préférence entre 100 et 500 g/m², et dont le non-tissé présente une masse surfacique comprise entre 50 et 120 g/m², en particulier entre 60 et 100 g/m², de préférence entre 50 et 80 g/m², et/ou dont le non-tissé dans la structure plate **(11)** présente une épaisseur comprise entre 1 mm à 3 cm, de préférence entre 1 mm à 10 mm, en particulier entre 3 mm à 9 mm, idéalement entre 4 mm à 7 mm, et pouvant être lavée et désinfectée.

2. Structure plate selon la revendication 1, **caractérisée en ce que** le non-tissé présente des fibres de solidification pénétrant de façon substantielle dans l'épaisseur du non-tissé, notamment par un traitement ultérieur mécanique du non-tissé, en particulier par aiguilletage, et/ou **en ce que** la structure plate **(11)** englobe une ou plusieurs couches de non-tissé et/ou **en ce qu'**une réticulation mécanique, chimique ou thermique et/ou un aiguilletage des différentes fibres **(14)** ou des filaments du non-tissé est réalisé, et/ou **en ce que** le non-tissé est formé comme un non-tissé de nanofibres ou de microfibres et/ou à partir de fibres **(14)** ou de filaments.

3. Structure plate selon une ou plusieurs des revendications précédentes, **caractérisée en ce que** l'activation superabsorbante durable, résistante à l'usure et mécaniquement stable est liée à la structure plate (**11**), au non-tissé et/ou aux fibres **(14)** ou aux filaments, ou bien **en ce que** les fibres ou les filaments se composent en tout ou partie de polymères superabsorbants, et/ou **en ce qu'**au moins un produit d'activation superabsorbant est/a été appliqué sur le non-tissé et/ou les fibres **(14)** ou les filaments, la part en non-tissé et/ou en fibres **(14)** ou filaments du produit d'activation superabsorbant étant comprise entre 0 et 800 pourcentage en masse, en fonction de la masse surfacique du non-tissé, et/ou **en ce que**, pour lier le non-tissé et/ou les fibres **(14)** ou les filaments avec le produit d'activation superabsorbant, une application avec de la colle est prévue, à base notamment de polyacrylate, dont la part en non-tissé est comprise entre 0 et 85 pourcentage en masse en fonction de la masse surfacique du non-tissé, et/ou **en ce que** le non-tissé et/ou la surface **(16, 17)** des fibres **(14)** ou des filaments présente un revêtement à base de produit d'activation superabsorbant et/ou de colle.

4. Structure plate selon une ou plusieurs des revendications précédentes, **caractérisée en ce que** les fibres **(14)** ou les filaments présentent un titre de filé compris entre 0,1 et 20 dtex [gramme/10 000 m], et/ou **en ce que** les fibres **(14)** ou les filaments sont fabriqués à partir de polymères naturels, en particulier à base de coton, bambou, chanvre, cellulose, fibres minérales **(14),** ou à partir de polymères synthétiques, en particulier à partir de polymères thermoplastiques, de préférence du polyéthylène, du polyamide, du polyester ou du polypropylène, ou du polyuréthane, du polychlorure de vinyle, des fibres acryliques en soie artificielle, ou à partir de polymères et/ou de mélanges produits selon des procédés biotechnologiques, et/ou à partir de fibres de déchets issues des polymères précités, et/ou en ce que le non-tissé est formé à partir d'un mélange de différents fibres **(14),** filaments ou groupes de fibres, englobant 25 à 100 fibres de polymères, 0 à 20 fibres de déchets et/ou 0 à 10 fibres de polymères à bas point de fusion, et/ou **en ce que** les fibres, filaments ou groupes de fibres présentent des parts en fibres ou en filaments avec différents titres de filés compris entre 0,1 et 20 dtex, et/ou **en ce que** les fibres **(14)** ou les filaments à base de fibres à deux composants sont notamment formés à partir d'un mélange de deux polymères de différentes températures de fusion, choisis en particulier dans le groupe composé de polyéthylène, polyamide, polyester, polyuréthane et polypropylène, et/ou **en ce que** les fibres **(14)** ou les filaments sont formés par filage à sec, filage au mouillé, filage par fusion, filage d'une matrice ou filage électrique.

5. Structure plate selon une ou plusieurs des revendications précédentes, **caractérisée en ce qu'**une activation superabsorbante de la structure plate **(11)** et/ou du non-tissé est réalisée avant, pendant ou après le procédé de filage, et/ou **en ce que** l'activation superabsorbante est réalisée par au moins un polymère superabsorbant appliqué sur ou dans les fibres **(14)** ou les filaments et/ou dans et/ou sur le non-tissé, et/ou **en ce que** les fibres **(14)** ou les filaments sont formés en tout ou partie d'au moins un polymère superabsorbant, et/ou **en ce que** le polymère superabsorbant se présente sous la forme de particules **(15)** dont le diamètre est compris entre 45 et 875 µm, en particulier entre 326 et 674 µm, de préférence entre 400 et 500 µm, ou de particules présentant de préférence un diamètre entre 50 et 150 µm, et/ou de mélanges de particules **(15)** avec différents diamètres, et/ou de particules **(15)** sous forme de cube, de barre, de polyèdre, de cône, arrondie, angulaire, d'aiguille, de flocon ou de fibre **(14)** ou comme un collier de molécules/particules, et/ou **en ce que** le polymère superabsorbant est un copolymère chimiquement réticulé à base d'acide ou d'un de ses sels, notamment l'acrylate de sodium, et/ou **en ce que** la liaison du polymère superabsorbant et du non-tissé et/ou des fibres **(14)** ou des filaments survient avant la réticulation chimique du copolymère, et/ou **en ce que** le polymère superabsorbant et/ou les particules **(15)** se pressent autour, entourent et/ou recouvrent les fibres **(14)** ou les filaments, et/en **en ce que** le non-tissé, en particulier le non-tissé de nanofibres ou de microfibres est formé comme un non-tissé de fibres enchevêtrées, de fibres textiles et/ou de fibres discontinues, et/ou **en ce que** les particules **(15)** contiennent des fibres **(14)** ou des filaments se croisant et/ou essentiellement parallèles et liés dans le non-tissé et/ou dans la structure plate **(11),** et/ou **en ce que** la structuration ou le revêtement de la surface des particules **(15)** et/ou des fibres **(14)** ou des filaments est formée selon une structuration ou un revêtement de surface exerçant un effet adhésif, notamment une nanostructuration ou un nanorevêtement.

6. Structure plate selon une ou plusieurs des revendications précédentes, **caractérisée en ce qu'**au moins une surface **(16, 17)** de la structure plate **(11)** est formée à partir d'un matériau différent de la structure plate **(11)** ou à partir du même matériau, et/ou présentant des caractéristiques différentes des caractéristiques restantes de la structure plate **(11),** et/ou **en ce que** la surface **(16, 17)** est formée par un traitement ultérieur mécanique, thermique et/ou chimique, en particulier par l'aiguilletage du non-tissé, et/ou **en ce que** la surface **(16, 17)** est formée par un non-tissé activé non superabsorbant, une membrane, une feuille, une substance, un lé, un revêtement ou un traitement plastifiant, tout en étant liée solidement ou de façon soluble au matériel restant de la structure plate **(11),** et/ou **en ce que** la surface **(16,** 17) est formée par fusion des fibres **(14)** ou des filaments, notamment en combinaison avec l'aiguilletage.

7. Structure plate selon une ou plusieurs des revendications précédentes, **caractérisée en ce que** la surface **(16, 17)** est formée au moins partiellement de façon perméable et est composée de pores pour le passage de liquides et/ou de gaz, notamment d'eau, d'alcool et/ou de vapeur d'eau/d'alcool ou de leurs mélanges, la taille des pores étant inférieure au diamètre minimal des particules **(15),** et/ou **en ce qu'**une compartimentation de la structure plate **(11)** est prévue et réalisée en doublant, cousant, collant ou soudant les surfaces **(16, 17)** de la structure plate **(11),** et/ou **en ce qu'**au moins une surface **(16, 17)** présente une activation supplémentaire, notamment un revêtement ou un matériel étanche, ignifuge, réfractaire, résistant aux étincelles, résistant aux projections de métal ou incandescentes, étanche au métal liquide, autonettoyant, résistant à la saleté, biocide, antiviral, absorbant les UV, antistatique, répulsif, efficace sur le plan cosmétique et médical, hydrophilisé, antimicrobien, antibactérien, antibiotique ou absorbant ou protégeant contre le rayonnement électromagnétique.

8. Structure plate selon une ou plusieurs des revendications précédentes, **caractérisée en ce que** la structure plate **(11)** est prévue pour une utilisation multiple et est par conséquent formée à partir de matériaux lavables, notamment en machine, chimiquement nettoyables, pouvant être désinfectés et/ou stérilisés.

9. Procédé de fabrication d'une structure plate conformément à une ou plusieurs des revendications précédentes, comprenant les étapes qui consistent à mettre à disposition les fibres **(14)** ou les filaments, à apposer le polymère superabsorbant dans ou sur les fibres **(14)** ou les filaments, avant, pendant ou après la mise à disposition, à raccorder les fibres **(14)** ou les filaments et le polymère superabsorbant et/ou à réticuler le polymère superabsorbant, et à former une structure plate **(11)** ou un non-tissé à base de fibres **(14)** ou de filaments.

10. Procédure selon la revendication 9, **caractérisée en ce que** la liaison et/ou la réticulation est réalisée mécaniquement et/ou chimiquement, en particulier par un agent de collage ou de réticulation, et/ou **en ce que** les fibres **(14)** ou les filaments sont formés par filage à sec, filage au mouillé, filage par fusion, filage d'une matrice ou filage électrique, et **en ce qu'**une liaison des fibres **(14)** ou des filaments et/ou d'une matière première des fibres **(14)** ou des filaments et du polymère superabsorbant est réalisée avant, pendant ou après le procédé de filage, et/ou **en ce que** les fibres **(14)** ou les filaments sont formés en tout ou partie à partir du polymère superabsorbant, et/ou **en ce que** le polymère superabsorbant est appliqué sur ce dernier après la formation du tissu textile **(11)** ou du non-tissé, avant, pendant ou après une réticulation chimique, et/ou **en ce qu'**une liaison mécanique du polymère superabsorbant avec la structure plate **(11)** ou le non-tissé et/ou une réticulation du polymère superabsorbant est réalisée.

11. Enveloppe avec deux ou plusieurs couches, comprenant au moins une couche formée d'une structure plate **(11)** textile selon une ou plusieurs des revendications 1 à 8 précédentes.

12. Enveloppe selon la revendication 11, **caractérisée en ce qu'**au moins une couche est raccordée en surface à la structure plate ou **en ce que** la structure plate se trouve entre deux couches, et/ou **en ce que** la couche est formée d'un tissu ou d'un textile, et/ou **en ce que** la couche, sous forme de membrane ou de feuille, est formée à partir de matériaux naturels ou artificiels, ou **en ce que** la couche est formée à partir d'une combinaison ou d'une structure en sandwich composée d'un ou de plusieurs tissus et/ou d'une ou de plusieurs membranes ou feuilles, d'origine naturelle ou artificielle, et/ou **en ce que** la couche se compose d'un tissu de microfibres, et/ou **en ce que** la couche se compose de fils de métal et/ou de fibres fonctionnelles, par exemple un tissu ou un textile à base de fibres de coco, de carbone ou minérales, d'origine naturelle ou artificielle, et/ou **en ce que** la couche est composée d'un matériau absorbant ou protégeant contre le rayonnement électromagnétique, et/ou **en ce que** la structure plate **(11)** est composée d'une ou de plusieurs membranes **(19a, 19b, 19c)** et/ou **en ce que** l'enveloppe **(10)** est formée comme un textile, un textile fonctionnel, un élément fonctionnel de refroidissement ou comme l'un des objets mentionnés ci-après, et/ou pouvant être relié notamment à un vêtement ou à un autre objet mentionné ci-après, agencé ou fixé sur ce dernier : vêtement, accessoire pour vêtement, vêtement de protection, doublure (fixe ou amovible) pour vêtements (comme notamment des vestes, costumes, pantalons, combinaisons, tabliers), bâche, couverture, construction de bâches ou de toitures, produit médical, blouse de chirurgien, refroidisseur de pouls, collier, bande réfrigérante, sac réfrigérant, bandage, bretelle, bandage pour pied et articulation, orthèse, masque facial, masque oculaire, chaussette, collant, couvre-chef, casque, chaussure ou botte, revêtement, notamment pour voiture, bateau, avion, bâtiment, machine, plancher, surface ou appareil, store, toile de tente, marquise, bâche de recouvrement ou bâche de recouvrement de protection ou pièce d'équipement pour protéger contre les températures élevées, sac, sachet, poche de sang, refroidissement des sacs ou des ampoules, poche de protection pour médicaments, récipient de conservation pour protéger les composants biologiques ou humaines et/ou les tissus et/ou les substances pharmaceutiques, les produits et/ou les substances contre des températures élevées, sac à dos ou autre produit sportif ou pour l'extérieur, maillot de sport, vêtement de sport, sous-vêtement, conservation, protection pendant le transport, produit pour refroidir les animaux ou produit similaire, et/ou la combinaison par un ou plusieurs moyens de combinaison, en particulier au moins un bouton, un bouton pression, une bande velcro, une boucle, une fermeture à crochets, une fermeture à glissière, un adhésif réutilisable, un moyen composé d'une nanostructure produisant l'adhérence ou un assemblage clipsé.

13. Enveloppe selon une ou deux des revendications 11 et 12, **caractérisée en ce que** l'enveloppe (10) est lavable, lavable en machine, chimiquement nettoyable, peut être stérilisée et/ou peut être désinfectée, a un effet refroidissant ou climatique après le chargement avec du liquide, notamment de l'eau ou un mélange à base d'eau, et/ou **en ce que** l'enveloppe **(10)** est refroidie après ou par le biais du chargement avec du liquide, notamment de l'eau ou un mélange à base d'eau.

14. Enveloppe selon une ou plusieurs des revendications 11 à 13, **caractérisée en ce que** l'enveloppe **(10)** est réalisée en procédant à un chargement avec un mélange à base de liquide ou à un chargement qui produit une substance renouvelant, rafraîchissant ou produisant un revêtement ou un matériel étanche, ignifuge, réfractaire, autonettoyant, résistant à la saleté, biocide, antiviral, absorbant les UV, antistatique, résistant aux étincelles, résistant aux projections de métal ou incandescentes, étanche au métal liquide, répulsif, hydrophilisé, antimicrobien, antibactérien, antibiotique ou absorbant ou protégeant contre le rayonnement électromagnétique, et/ou **en ce que** le chargement est réalisé avec un mélange à base d'alcool et d'eau servant de liquide, qui contient notamment entre 1 et 70 % du volume, de préférence entre 5 et 50 % du volume, en particulier entre 10 et 30 % d'alcool et entre 30 et 99 % d'eau et dont l'alcool est choisi dans le groupe composé d'éthanol, d'isopropanol ou de leurs mélanges, et/ou **en ce que** le liquide ou le mélange alcool-eau est composé d'un mélange d'un additif au moins, à savoir d'au moins une huile essentielle, un parfum, un arôme, un répulsif ou un agent aversif ou des mélanges de ces derniers, dont le pourcentage se situe entre 0,1 et 5 % en volume.

15. Enveloppe selon une ou plusieurs des revendications 11 à 14, **caractérisée en ce que**, en plus de ou alternativement à la part en alcool ou eau du mélange alcool-eau, le mélange d'une substance visant à renouveler, rafraîchir ou produire le revêtement ou le matériel étanche, ignifuge, réfractaire, résistant aux étincelles, résistant aux projections de métal ou incandescentes, étanche au métal liquide, autonettoyant, résistant à la saleté, biocide, antiviral, absorbant les UV, antistatique, répulsif, efficace sur le plan cosmétique et médical, hydrophilisé, antimicrobien, antibactérien, antibiotique ou électromagnétique, et/ou **en ce que** l'eau est choisie dans le groupe composé d'eau du robinet, d'eau minérale, d'eau distillée, d'eau déminéralisée ou d'eau très pure, le mélange à base d'alcool et d'eau et/ou d'au moins un additif sous forme concentrée étant mis à disposition et dilué avant le chargement de l'enveloppe avec de l'eau ajoutée jusqu'à atteindre une concentration finale, et/ou **en ce que** le mélange présente une addition de substances biocides, fongicides, virucides, antimicrobiennes, cosmétiques ou médicales et/ou antibiotiques, notamment une solution d'argent, la part de cette addition étant comprise entre 0,1 et 5 % en volume.

16. Enveloppe selon une ou plusieurs des revendications 11 à 15, **caractérisée en ce que** l'enveloppe **(10)** est conservée ou transportée, pendant ou après le refroidissement préliminaire, dans des récipients de refroidissement mobiles ou portables, et/ou est utilisée en combinaison avec de l'air froid ou dans des chambres froides, et/ou **en ce qu'**au moins une partie de l'enveloppe **(10)** et/ou au moins une couche est formée à partir d'un matériel étanche, résistant aux étincelles ou aux flammes, étanche au métal liquide et/ou résistant aux projections de métal ou incandescentes, étanche aux liquides, autonettoyant, résistant à la saleté, biocide, antimicrobien, antiviral, antibactérien, antibiotique, absorbant les UV, répulsif, efficace sur le plan cosmétique et médical, hydrophilisé, absorbant ou protégeant contre le rayonnement électromagnétique et/ou caoutchouteux, ou bien présente un revêtement, un matériel ou une activation étanche, résistant aux étincelles ou aux flammes, étanche au métal liquide et/ou résistant aux projections de métal ou incandescentes, étanche aux liquides, autonettoyant, résistant à la saleté, biocide, antimicrobien, antiviral, antibiotique, absorbant les UV, répulsif, efficace sur le plan cosmétique et médical, hydrophilisé, absorbant ou protégeant contre le rayonnement électromagnétique et/ou caoutchouteux.

17. Enveloppe selon une ou plusieurs des revendications 11 à 16, **caractérisée en ce que** des canaux pour l'aération ou la répartition du liquide sont prévus dans une ou plusieurs des couches, les canaux devant de préférence pouvoir être fermés manuellement et/ou automatiquement, et/ou **en ce que** des micropompes sont prévues pour la réception et/ou la distribution de liquide, et/ou **en ce que** la réception et/ou la distribution de liquide est réalisée osmotiquement et/ou **en ce qu'**un réservoir de fluide, notamment une cuve, un bidon, une bouteille ou autre récipient similaire est prévu pour la réception ou la distribution du liquide sur ou dans l'enveloppe **(10),** et **en ce qu'**un système de tuyaux pour le liquide traversant l'enveloppe **(10)** et/ou la structure plate **(11)** est agencé sur le réservoir de fluide.

18. Enveloppe selon une ou plusieurs des revendications 11 à 17, **caractérisée en ce qu'**au moins une structure plate **(11)** est agencée sur ou dans l'enveloppe **(10),** notamment par collage, soudage ou couture ou de façon soluble, ou par le biais d'un élément de raccordement, et/ou **en ce que** l'enveloppe **(10)** est formée au moins partiellement de façon perméable et présente des pores pour le passage de liquides et/ou de gaz, notamment d'eau, d'alcool et/ou de vapeur d'eau/d'alcool ou de leurs mélanges, la taille des pores étant inférieure au diamètre minimal des particules **(15),** et/ou **en ce qu'**une compartimentation de l'enveloppe **(10)** est prévue et réalisée en doublant, cousant, collant ou soudant les surfaces **(16, 17)** ou les couches de l'enveloppe **(10),** et/ou **en ce qu'**au moins une couche présente une activation supplémentaire, notamment un revêtement ou un matériel étanche, ignifuge, réfractaire, résistant aux étincelles, résistant aux projections de métal ou incandescentes, étanche au métal liquide, autonettoyant, résistant à la saleté, biocide, antiviral, absorbant les UV, antistatique, répulsif, efficace sur le plan cosmétique et médical, hydrophilisé, antimicrobien, antibactérien, antibiotique et/ou absorbant ou protégeant contre le rayonnement électromagnétique.

19. Système de refroidissement, notamment pour l'utilisation dans le cadre d'un refroidissement contrôlé et surveillé de personnes, d'animaux et/ou d'objets, composé d'une enveloppe **(10)** conformément à une ou plusieurs des revendications 11 à 18 précédentes.

20. Système de refroidissement selon la revendication 19, **caractérisé en ce que** le système de refroidissement comprend un récipient de conservation qui absorbe l'enveloppe chargée de liquide, et/ou **en ce que** le récipient de conservation est formé comme notamment un emballage en plastique évacuable et soudable ou comme un récipient pouvant être refermé, à base notamment de plastique ou de métal, et/ou **en ce que** le récipient de conservation absorbe, prépare ou transmet également d'autres liquides pour le chargement et l'activation répété de l'enveloppe, et/ou **en ce que** le récipient de conservation est doté d'un revêtement antimicrobien sur au moins une partie de ses surfaces appliquées à l'enveloppe, et/ou **en ce que** le contenu du récipient de conservation peut être désinfecté et/ou stérilisé ou est doté d'une enveloppe stérile composée notamment d'une structure plate chargée, et/ou **en ce qu'**un capteur est prévu pour saisir la température extérieure et/ou la température de l'enveloppe, plus particulièrement en disposant un indicateur de température sur le capteur, et/ou **en ce que** le capteur présente la forme d'un revêtement de couleurs thermiques ou d'un thermomètre, et/ou **en ce qu'**au moins un capteur est prévu pour saisir les facteurs climatiques autour du système refroidissant, en particulier pour saisir les températures environnantes de l'humidité relative de l'air et/ou des courants d'air.

21. Utilisation d'un mélange à base d'alcool et d'eau et/ou d'un mélange à base d'eau, d'alcool et/ou d'une substance renouvelant, rafraîchissant et/ou produisant le matériel de l'enveloppe et/ou de la structure plate, en vue de charger une enveloppe selon une ou plusieurs des revendications 11 à 18 et/ou dans un système refroidissant selon une des revendications 19 et 20 ou les deux.

22. Utilisation selon la revendication 21, **caractérisée en ce que** le mélange est apposé ou vaporisé sur l'enveloppe **(10)** et/ou dans l'enveloppe **(10)** et/ou **en ce qu'**une structure plate (11) prévue dans l'enveloppe **(10)** est remplie ou chargée, et/ou **en ce que** l'enveloppe **(10)** et/ou une structure plate **(11)** prévuedans l'enveloppe **(10)** est plongé dans le mélange, qui contient entre 1 et 70 % du volume, de préférence entre 5 et 50 % du volume, en particulier entre 10 et 30 % d'alcool et entre 30 et 99 % de volume d'eau et dont l'alcool est choisi dans le groupe composé d'éthanol, d'isopropanol ou de leurs mélanges, et/ou **en ce que** l'alcool ou le mélange est composé d'un mélange d'un additif au moins, à savoir d'au moins une huile essentielle, un parfum, un arôme, un répulsif ou un agent aversif ou des mélanges de ces derniers, dont le pourcentage en additif se situe entre 0,1 et 5 % en volume, et dont l'eau est choisie dans le groupe composé d'eau du robinet, d'eau minérale, d'eau distillée, d'eau déminéralisée ou d'eau très pure, et/ou **en ce que** le mélange présente une addition de substances biocides, fongicides, virucides, répulsives, antimicrobiennes et/ou antibiotiques, la part de cette addition étant comprise entre 0,1 et 5 % en volume.

23. Article de refroidissement, en particulier un produit textile, en priorité un produit textile de grande étendue, de préférence des vêtements (en particulier vêtement de dessus, accessoire pour vêtements, bretelles, vêtement de protection, bâche, couverture, construction de bâches ou de toitures, produit médical, blouse de chirurgien, refroidisseur de pouls, collier, bande réfrigérante, sac réfrigérant, bandage, bandage pour pied et articulation, orthèse, masque facial, masque oculaire, chaussette, collant, couvre-chef, casque, chaussure ou botte, revêtement, notamment pour voiture, bateau, avion, bâtiment, machine, plancher, surface ou appareil, store, toile de tente, marquise, bâche de recouvrement ou bâche de recouvrement de protection ou pièce d'équipement pour protéger contre les températures élevées, sac, sachet, poche de sang, refroidissement des sacs ou des ampoules, poche de protection pour médicaments, récipient de conservation pour protéger les composants biologiques ou humaines et/ou les tissus ainsi que les substances pharmaceutiques, les produits et/ou les substances contre des températures élevées, sac à dos ou autre produit sportif ou pour l'extérieur, maillot de sport, vêtement de sport, sous-vêtement, conservation, protection pendant le transport, produit pour refroidir les animaux ou produit similaire), à partir duquel une enveloppe **(10)** est formée selon une ou plusieurs des revendications précédentes 11 à 18, **caractérisée en ce que** l'article de refroidissement présente au moins une zone composée d'une structure plate **(11)** selon une ou plusieurs des revendications précédentes 1 à 10, et/ou **caractérisée en ce que** l'article de refroidissement présente, dans au moins une seconde zone, notamment dans une partie détachable de l'article de refroidissement, au moins une structure plate **(11)** sans ou avec un faible effet de refroidissement, notamment sans polymère superabsorbant ou avec un pourcentage réduit en polymère superabsorbant, et de préférence une zone de l'article de refroidissement pouvant être détachée de la première zone et pouvant être raccordée par un ou plusieurs moyens de combinaison, notamment au moins un bouton, un bouton pression, une bande velcro, une boucle, une fermeture à crochets, une fermeture à glissière, un adhésif réutilisable, un moyen présentant une nanostructure produisant l'adhérence ou un assemblage clipsé.
